# EUROPEAN PATENT SPECIFICATION

(11) **EP 3 261 638 B1**
(45) Date of publication and mention of the grant of the patent: **08.05.2019**
(21) Application number: 16756548.0
(22) Date of filing: 29.02.2016
(51) Int. Cl.: A61K 31/404, A61K 31/506, C07D 401/14, C07D 403/12, C07D 405/14, C07D 403/14, C07D 417/14, C07D 487/14, A61P 29/00, A61P 35/00, A61P 37/00

(54) **PYRIMIDINE DERIVATIVES AS KINASE INHIBITORS AND THEIR THERAPEUTICAL APPLICATIONS**
PYRIMIDINDERIVATE ALS KINASEINHIBITOREN UND DEREN THERAPEUTISCHE ANWENDUNGEN
DÉRIVÉS DE PYRIMIDINE UTILISÉS EN TANT QU'INHIBITEURS DE KINASE, ET LEURS APPLICATIONS THÉRAPEUTIQUES

(30) Priority: 27.02.2015 WO PCT/US2015/018085
(43) Date of publication of application: 03.01.2018
(73) Proprietor: NantBioScience, Inc., Culver City, CA 90232 (US)
(72) Inventor: TAO, Chunlin, Newport Coast, California 92657 (US); WANG, Qinwei, Alhambra, California 91801 (US); NALLAN, Laxman, Rancho Mission Viejo, California 92694 (US); HO, David, Monterey Park, California 91754 (US); POLAT, Tulay, Tustin, California 92782 (US); ARP, Forrest, Irvine, California 92618 (US); WEINGARTEN, Paul, Anaheim, California 92807 (US)
(74) Representative: Grünecker Patent- und Rechtsanwälte PartG mbB
(86) International application number: PCT/US2016/020095
(87) International publication number: WO 2016/138527

(56) References cited:
- WO-A1-2014/183300
- US-A1- 2007 004 764
- US-A1- 2010 305 084
- US-A1- 2011 053 923

## Description

### FIELD OF THE INVENTION

The present invention relates generally to the use of compounds to treat a variety of disorders, diseases and pathologic conditions and more specifically to the use of substituted pyrimidine derivatives to modulate protein kinases and for treating protein kinase-mediated diseases.

### BACKGROUND OF THE INVENTION

Protein kinases constitute large families of structurally related proteinaceous enzymes that are responsible for the control of numerous signal transduction pathways in the eukaryotic cell. Protein kinases, containing a similar 250-300 amino acid catalytic domain, catalyze the phosphorylation of target protein substrates. As such protein kinases are among the most promising small molecule drug targets.

The kinases may be categorized into families by the substrates in the phosphorylate (e.g., protein-tyrosine, protein-serine/threonine, lipids, etc.). Tyrosine phosphorylation is a central event in the regulation of a variety of biological processes such as cell proliferation, migration, differentiation and survival. Several families of receptor and non-receptor tyrosine kinases control these events by catalyzing the transfer of phosphate from ATP to a tyrosine residue of specific cell protein targets. Sequence motifs have been identified that generally correspond to each of these kinase families [Hanks et al., FASEB J., (1995), 9, 576-596; Knighton et al., Science, (1991), 253, 407-414; Garcia-Bustos et al., EMBO J., (1994),13:2352-2361). Examples of kinases in the protein kinases include (without limitation): abl, Akt, bcr-abl, Blk, Brk, Btk, c-kit, c-Met, c-src, c-fms, CDK1, CDK2, CDK3, CDK4, CDK5, CDK6, CDK7, CDK8, CDK9, CDK10, cRaf1, CSF1R, CSK, EGFR, ErbB2, ErbB3, ErbB4, Erk, Fak, fes, FGFR1, FGFR2, FGFR3, FGFR4, FGFR5, Fgr, flt-1, Fps, Frk, Fyn, Hck, IGF-1R, INS-R, Jak, KDR, Lck, Lyn, MEK, p38, PDGFR, PIK, PKC, PYK2, ros, Tie, Tie-2, TRK, Yes, and Zap70.

Studies indicated that protein kinases play a central role in the regulation and maintenance of a wide variety of cellular processes. For example, kinase activity acts as molecular switches regulating cell proliferation, activation, and/or differentiation. Uncontrolled or excessive kinase activity, whether from mutant kinases or wild type kinases has been observed in many disease states including benign and malignant proliferation disorders, as well as diseases resulting from inappropriate activation of the immune system (autoimmune disorders), allograft rejection, and graft vs host disease.

It is reported that many diseases are associated with abnormal cellular responses triggered by protein kinase-mediated events. These diseases include autoimmune diseases, inflammatory diseases, bone diseases, metabolic diseases, neurological and neurodegenerative diseases, cancer, cardiovascular diseases, allergies and asthma, Alzheimer's disease and hormone-related diseases. In addition, endothelial cell specific receptor PTKs, such as VEGF-2 and Tie-2, mediate the angiogenic process and are involved in supporting the progression of cancers and other diseases involving uncontrolled vascularization. Accordingly, there has been a substantial effort in medicinal chemistry to find protein kinase inhibitors that are effective as therapeutic agents.

Many cancers are characterized by disruptions in cellular signaling pathways that lead to uncontrolled growth and proliferation of cancerous cells. Receptor tyrosine kinases (RTKs) play a crucial role in these signaling pathways, transmitting extracellular molecular signals into cytoplasm and/or nucleus of a cell. RTKs are transmembrane proteins that generally include an extracellular ligand-binding domain, a membrane-spanning domain and a catalytic cytoplasmic tyrosine kinase domain. The binding of ligand to the extracellular potion is believed to promote dimerization, resulting in trans-phosphorylation and activation of the intracellular tyrosine kinase domain (Schlessinger et al. Neuron 1992;9:383-391).

Considering the lack of currently available treatment options for the majority of the conditions associated with protein kinases, there is still a great need for new therapeutic agents that inhibit protein kinases. In particular, there is a need for highly active kinase inhibitors that are also non-toxic, and specific to certain protein kinases.

### SUMMARY OF THE INVENTION

The invention is defined by the following embodiments 1-12:
1. A compound of formula 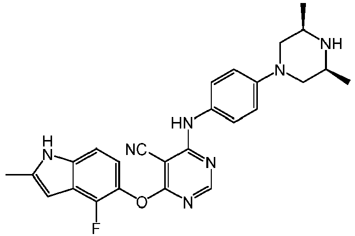 or a pharmaceutically acceptable salt thereof.
2. A compound of formula 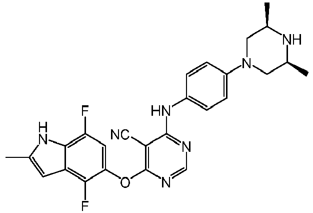 or a pharmaceutically acceptable salt thereof.
3. A compound of formula 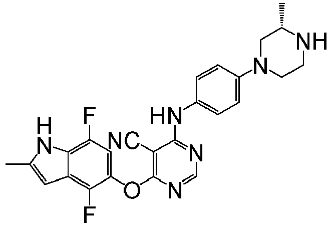 or a pharmaceutically acceptable salt thereof.
4. A compound of formula 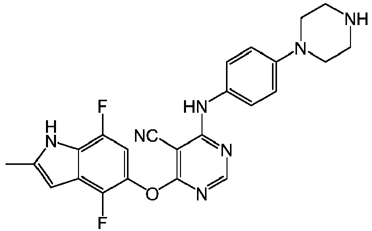 or a pharmaceutically acceptable salt thereof.
5. A compound of formula 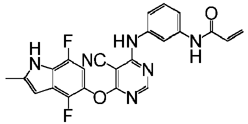 or a pharmaceutically acceptable salt thereof.
6. A compound of formula 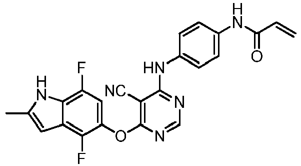 or a pharmaceutically acceptable salt thereof.
7. The compound or pharmaceutically acceptable salt of any one of items 1-6 for use in treating an animal suffering from a cellular proliferative disorder wherein the use comprises:
   a. Preparing, or causing to be prepared, the compound or pharmaceutically acceptable salt of any one of items 1-6;
   b. optionally, formulating, or causing to be formulated, the compound or pharmaceutically acceptable salt of step a with a pharmaceutically acceptable carrier; and
   c. that the compound or pharmaceutically acceptable salt of step a or the formulation of step b is to be administered, or caused to be administered to the animal suffering from the proliferative disorder.
8. The compound or pharmaceutically acceptable salt for use according to item 7, wherein the animal is a mammal.
9. The compound or pharmaceutically acceptable salt for use according to item 8, wherein the animal is a human.
10. The compound or pharmaceutically acceptable salt for use according to any one items 7-9, wherein the cellular proliferative disorder is a cancer, a precancerous state, a benign tumor, autoimmune disorder, transplant rejection, graft versus host disease, response to an infection, response to an environmental insult or a genetic disorder.
11. The compound or pharmaceutically acceptable salt for use according to any one of items 7-9, wherein the cellular proliferative disorder is a cancer.
12. The compound or pharmaceutically acceptable salt for use according to item 11, wherein the cancer is a skin cancer, breast cancer, uterine cancer, ovarian cancer, testicular cancer, prostate cancer, nasopharyngeal cancer, lung cancer, esophageal cancer, gastric cancer, hepatic cancer, biliary cancer, pancreatic cancer, lymphoma, lung cancer, renal cancer, bladder cancer, esophageal cancers, myeloid leukemia, lymphocytic leukemia, myleoproliferative disorder, myelodysplastic syndrome, lymphoma, neuroendocrine cancer, sarcoma or brain tumor.

Further disclosed herein is a compound of the formula 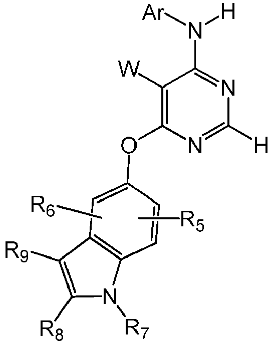 or a pharmaceutically acceptable salt thereof, wherein:
W is selected from: F, Cl, Br, I, CN, C₁-C₄ alkyl, C₁-C₆ alkoxy, C₂-C₆ alkenyl, CF₃, CF₂H, CFH₂, C₂-C₆ alkynyl, CON(R1)R2.

R1and R2 represent hydrogen, alkyl, cycloalkyl, alkenyl, alkynyl, alkylthio, aryl, arylalkyl.

Ar represents heteroaryl or aryl, each of which is substituted with from 0 to 4 substituents independently chosen from:
(1) halogen, hydroxy, amino, amide, cyano, -COOH, -SO₂NH₂, oxo, nitro and alkoxycarbonyl; and
(2) NR1
(3) groups of the formula (Ia):

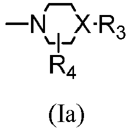

   wherein:
   R₄ represents hydrogen, C₁-C₄ alkyl, oxo;
   X is CH, when R₃ is hydrogen; or X-R₃ is O; or X is N, R₃ represents groups of hydrogen, C₁-C₆ alkyl, C₂-C₆ alkenyl, C₂-C₆ alkynyl, C₃-C₁₀ aryl or heteroaryl, (C₃-C₇cycloalkyl)C₁-C₄ alkyl, C₁- C₆ haloalkyl, C₁-C₆ alkoxy, C₁- C₆ alkylthio, C₂-C₆ alkanoyl, C₁- C₆ alkoxycarbonyl, C₂- C₆ alkanoyloxy, mono- and di-(C₃-C₈ cycloalkyl)aminoCo-C₄alkyl, (4- to 7- membered heterocycle)C₀-C₄alkyl, C₁-C₆ alkylsulfonyl, mono- and di-(C₁-C₆ alkyl) sulfonamido, and mono- and di-(C₁-C₆ alkyl)aminocarbonyl, each of which is substituted with from 0 to 4 substituents independently chosen from halogen, hydroxy, cyano, amino, -COOH and oxo.

Substituents on indole are as the following:R₅ and R₆ are independently selected from: Hydrogen, F, Cl, Br, CN, C₁-C₄ alkyl, C₁-C₆ alkoxy.

R₇,R₈ and R₉ are independently selected from Hydrogene, C₁-C₄ alkyl, C₂-C₆ alkenyl, C₂-C₆ alkynyl, C₃-C₁₀ aryl or heteroaryl, C₁-C₆ alkoxy, C₁- C₆ alkylthio, C₂-C₆ alkanoyl, C₁- C₆ alkoxycarbonyl, C₂- C₆ alkanoyloxy.

A pharmaceutical composition comprising at least one compound of claim I or its pharmaceutically acceptable salts, hydrates, solvates, crystal forms salts and individual diastereomers thereof, and a pharmaceutically acceptable carrier.

Accordingly, it is an objective of the present invention to provide an antitumor agent comprising substituted pyrimidine derivatives as described in formula (I), pharmaceutically-acceptable formulations thereof, methods for making novel compounds and compositions for using the compounds. The compounds and compositions comprising the compounds in formula (I) have utility in treatment of a variety of diseases.

The combination therapy described herein may be provided by the preparation of the substituted pyrimidine derivatives of formula (I) and the other therapeutic agent as separate pharmaceutical formulations followed by the administration thereof to a patient simultaneously, semi-simultaneously, separately or over regular intervals.

The present invention provides certain chemical compounds such as kinase inhibitors for use in methods of treatment of various diseases, disorders, and pathologies, for example, cancer, and vascular disorders, such as myocardial infarction (MI), stroke, or ischemia. The compounds described in this invention may block the enzymatic activity of some or many of the members of the FGFR kinase family, in addition to blocking
the activity of other receptor and non-receptor kinases.

### BRIEF DISCRIPTION OF THE FIGURES

Figure 1 depicts a dose response curve for Compound 19.
Figure 2 depicts Compound 19's anti-tumor acivity.
Figure 3 depicts Compound 19's toxicity in rats based on weight loss.

### DETAILED DESCRIPTION OF THE INVENTION

The present disclosure is related to compounds having general Formula (I) 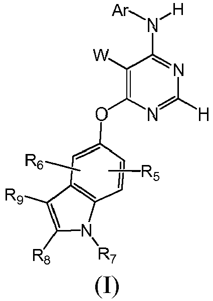 or a pharmaceutically acceptable salt thereof, wherein:
W is selected from: F, Cl, Br, I, CN, C₁-C₄ alkyl, C₁-C₆ alkoxy, C2-C6 alkenyl, CF3, CF2H, CFH2, C2-C6 alkynyl, CON(R1)R2.

R1and R2 represent hydrogen, alkyl, cycloalkyl, alkenyl, alkynyl, alkylthio, aryl, arylalkyl.

Ar represents heteroaryl or aryl, each of which is substituted with from 0 to 4 substituents independently chosen from:
(1) halogen, hydroxy, amino, amide, cyano, -COOH, -SO₂NH₂, oxo, nitro and alkoxycarbonyl; and
(2) NR1
(3) groups of the formula (Ia):

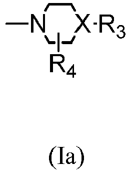

A compound of the formula,
or a pharmaceutically acceptable salt thereof, wherein: 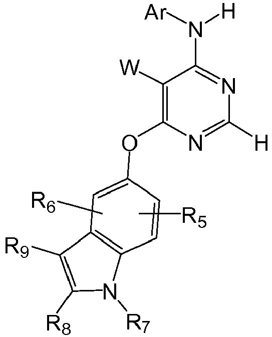 W is selected from: F, Cl, Br, I, CN, C₁-C₄ alkyl, C₁-C₆ alkoxy, C₂-C₆ alkenyl, CF₃, CF₂H, CFH₂, C₂-C₆ alkynyl, CON(R1)R2.
1. R1and R2 represent hydrogen, alkyl, cycloalkyl, alkenyl, alkynyl, alkylthio, aryl, arylalkyl.
2. Ar represents heteroaryl or aryl, each of which is substituted with from 0 to 4 substituents independently chosen from:
   (1) halogen, hydroxy, amino, amide, cyano, -COOH, -SO₂NH₂, oxo, nitro and alkoxycarbonyl; and
   (2) NR1
   (3) groups of the formula (Ia): 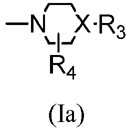 wherein:
      R₄ represents hydrogen, C₁-C₄ alkyl, oxo;
      X is CH, when R₃ is hydrogen; or X-R₃ is O; or X is N, R₃ represents groups of hydrogen, C₁-C₆ alkyl, C₂-C₆ alkenyl, C₂-C₆ alkynyl, C₃-C₁₀ aryl or heteroaryl, (C₃-C₇cycloalkyl)C₁-C₄ alkyl, C₁- C₆ haloalkyl, C₁-C₆ alkoxy, C₁- C₆ alkylthio, C₂-C₆ alkanoyl, C₁- C₆ alkoxycarbonyl, C₂- C₆ alkanoyloxy, mono- and di-(C₃-C₈ cycloalkyl)aminoCo-C₄alkyl, (4- to 7- membered heterocycle)C₀-C₄alkyl, C₁-C₆ alkylsulfonyl, mono- and di-(C₁-C₆ alkyl) sulfonamido, and mono- and di-(C₁-C₆ alkyl)aminocarbonyl, each of which is substituted with from 0 to 4 substituents independently chosen from halogen, hydroxy, cyano, amino, -COOH and oxo.
3. Substituents on indole are as the following:
   R₅ and R₆ are independently selected from: Hydrogen, F, Cl, Br, CN, C₁-C₄ alkyl, C₁-C₆ alkoxy.
4. R₇,R₈ and R₉ are independently selected from Hydrogene, C₁-C₄ alkyl, C₂-C₆ alkenyl, C₂-C₆ alkynyl, C₃-C₁₀ aryl or heteroaryl, C₁-C₆ alkoxy, C₁- C₆ alkylthio, C₂-C₆ alkanoyl, C₁- C₆ alkoxycarbonyl, C₂- C₆ alkanoyloxy.
5. A pharmaceutical composition comprising at least one compound of claim I or its pharmaceutically acceptable salts, hydrates, solvates, crystal forms salts and individual diastereomers thereof, and a pharmaceutically acceptable carrier.
   wherein:
   R₄ represents hydrogen, C₁-C₄ alkyl, oxo;
   X is CH, when R₃ is hydrogen; or X-R₃ is O; or X is N, R₃ represents groups of hydrogen, C₁-C₆ alkyl, C₂-C₆ alkenyl, C₂-C₆ alkynyl, C₃-C₁₀ aryl or heteroaryl, (C₃-C₇cycloalkyl)C₁-C₄ alkyl, C₁- C₆ haloalkyl, C₁-C₆ alkoxy, C₁- C₆ alkylthio, C₂-C₆ alkanoyl, C₁- C₆ alkoxycarbonyl, C₂- C₆ alkanoyloxy, mono- and di-(C₃-C₈ cycloalkyl)aminoCo-C₄alkyl, (4- to 7- membered heterocycle)C₀-C₄alkyl, C₁-C₆ alkylsulfonyl, mono- and di-(C₁-C₆ alkyl) sulfonamido, and mono- and di-(C₁-C₆ alkyl)aminocarbonyl, each of which is substituted with from 0 to 4 substituents independently chosen from halogen, hydroxy, cyano, amino, -COOH and oxo.
   R₅ and R₆ are independently selected from: hydrogen, F, Cl, Br, CN, C₁-C₄ alkyl, C₁-C₆ alkoxy.
   R₇,R₈ and R₉ are independently selected from Hydrogene, C₁-C₄ alkyl, C₂-C₆ alkenyl, C₂-C₆ alkynyl, C₃-C₁₀ aryl or heteroaryl, C₁-C₆ alkoxy, C₁- C₆ alkylthio, C₂-C₆ alkanoyl, C₁-C₆ alkoxycarbonyl, C₂- C₆ alkanoyloxy.

The term "halo" or "halogen" refers to fluorine, chlorine, bromine or iodine.

The term "alkyl" herein alone or as part of another group refers to a monovalent alkane (hydrocarbon) derived radical containing from 1 to 12 carbon atoms unless otherwise defined. Alkyl groups may be substituted at any available point of attachment. An alkyl group substituted with another alkyl group is also referred to as a "branched alkyl group". Exemplary alkyl groups include methyl, ethyl, propyl, isopropyl, n-butyl, t-butyl, isobutyl, pentyl, hexyl, isohexyl, heptyl, dimethylpentyl, octyl, 2,2,4-trimethylpentyl, nonyl, decyl, undecyl, dodecyl, and the like. Exemplary substituents include but are not limited to one or more of the following groups: alkyl, aryl, halo (such as F, Cl, Br, I), haloalkyl (such as CCl₃ or CF₃), alkoxy, alkylthio, hydroxy, carboxy (-COOH), alkyloxycarbonyl (-C(O)R), alkylcarbonyloxy (- OCOR), amino (-NH₂), carbamoyl (-NHCOOR- or -OCONHR-), urea (-NHCONHR-) or thiol (-SH). In some preferred embodiments of the present disclosure, alkyl groups are substituted with, for example, amino, heterocycloalkyl, such as morpholine, piperazine, piperidine, azetidine, hydroxyl, methoxy, or heteroaryl groups such as pyrrolidine,

The term 'cycloalkyl" herein alone or as part of another group refers to fully saturated and partially unsaturated hydrocarbon rings of 3 to 9, preferably 3 to 7 carbon atoms. The examples include cyclopropyl, cyclobutyl, cyclopentyl and cyclohexyl, and like. Further, a cycloalkyl may be substituted. A substituted cycloalkyl refers to such rings having one, two, or three substituents, selected from the group consisting of halo, alkyl, substituted alkyl, alkenyl, alkynyl, nitro, cyano, oxo (=O), hydroxy, alkoxy, thioalkyl, -CO₂H, -C(=O)H, CO₂-alkyl, - C(=O)alkyl, keto, =N-OH, =N-O-alkyl, aryl, heteroaryl, heterocyclo, -NR'R", - C(=O)NR'R", -CO₂NRR", -C(=O)NR'R", -NRCO₂R", - NR'C(=O)R", -SO₂NR'R", and - NR'SO₂R", wherein each of R' and R" are independently selected from hydrogen, alkyl, substituted alkyl, and cycloalkyl, or R' and R" together form a heterocyclo or heteroaryl ring.

The term 'alkenyl" herein alone or as part of another group refers to a hydrocarbon radical straight, branched or cyclic containing from 2 to 12 carbon atoms and at least one carbon to carbon double bond. Examples of such groups include the vinyl, allyl, 1-propenyl, isopropenyl, 2-methyl-1-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1-heptenyl, and like. Alkenyl groups may also be substituted at any available point of attachment. Exemplary substituents for alkenyl groups include those listed above for alkyl groups, and especially include C3 to C7 cycloalkyl groups such as cyclopropyl, cyclopentyl and cyclohexyl, which may be further substituted with, for example, amino, oxo, hydroxyl, etc.

The term "alkynyl" refers to straight or branched chain alkyne groups, which have one or more unsaturated carbon-carbon bonds, at least one of which is a triple bond. Alkynyl groups include C2-C8 alkynyl, C₂-C₆ alkynyl and C₂-C₄ alkynyl groups, which have from 2 to 8, 2 to 6 or 2 to 4 carbon atoms, respectively. Illustrative of the alkynyl group include ethenyl, propenyl, isopropenyl, butenyl, isobutenyl, pentenyl, and hexenyl. Alkynyl groups may also be substituted at any available point of attachment. Exemplary substituents for alkynyl groups include those listed above for alkyl groups such as amino, alkylamino, etc. The numbers in the subscript after the symbol "C" define the number of carbon atoms a particular group can contain.

The term "alkoxy" alone or as part of another group denotes an alkyl group as described above bonded through an oxygen linkage (-O-). Preferred alkoxy groups have from 1 to 8 carbon atoms. Examples of such groups include the methoxy, ethoxy, n-propoxy, isopropoxy, n-butoxy, isobutoxy, sec-butoxy, tert-butoxy, n-pentyloxy, isopentyloxy, n-hexyloxy, cyclohexyloxy, n-heptyloxy, n-octyloxy and 2-ethylhexyloxy.

The term "alkylthio" refers to an alkyl group as described above attached via a sulfur bridge. Preferred alkoxy and alkylthio groups are those in which an alkyl group is attached via the heteroatom bridge. Preferred alkylthio groups have from 1 to 8 carbon atoms. Examples of such groups include the methylthio, ethylthio, n-propythiol, n-butylthiol, and like.

The term "oxo," as used herein, refers to a keto (C=O) group. An oxo group that is a substituent of a nonaromatic carbon atom results in a conversion of-CH₂- to -C(=O)-.

The term "alkoxycarbonyl" herein alone or as part of another group denotes an alkoxy group bonded through a carbonyl group. An alkoxycarbonyl radical is represented by the formula: -C(O)OR, where the R group is a straight or branched C₁-C₆ alkyl group, cycloalkyl, aryl, or heteroaryl.

The term "arylalkyl" herein alone or as part of another group denotes an aromatic ring bonded through an alkyl group (such as benzyl) as described above.

The term "aryl" herein alone or as part of another group refers to monocyclic or bicyclic aromatic rings, e.g. phenyl, substituted phenyl and the like, as well as groups which are fused, e.g., napthyl, phenanthrenyl and the like. An aryl group thus contains at least one ring having at least 6 atoms, with up to five such rings being present, containing up to 20 atoms therein, with alternating (resonating) double bonds between adjacent carbon atoms or suitable heteroatoms. Aryl groups may optionally be substituted with one or more groups including, but not limited to halogen such as I, Br, F, or Cl; alkyl, such as methyl, ethyl, propyl, alkoxy, such as methoxy or ethoxy, hydroxy, carboxy, carbamoyl, alkyloxycarbonyl, nitro, alkenyloxy, trifluoromethyl, amino, cycloalkyl, aryl, heteroaryl, cyano, alkyl S(O)m (m=0, 1, 2), or thiol.

The term "aromatic" refers to a cyclically conjugated molecular entity with a stability, due to delocalization, significantly greater than that of a hypothetical localized structure, such as the Kekule structure.

The term "amino" herein alone or as part of another group refers to -NH₂. An "amino" may optionally be substituted with one or two substituents, which may be the same or different, such as alkyl, aryl, arylalkyl, alkenyl, alkynyl, heteroaryl, heteroarylalkyl, cycloheteroalkyl, cycloheteroalkylalkyl, cycloalkyl, cycloalkylalkyl, haloalkyl, hydroxyalkyl, alkoxyalkyl, thioalkyl, carbonyl or carboxyl. These substituents may be further substituted with a carboxylic acid, any of the alkyl or aryl substituents set out herein. In some embodiments, the amino groups are substituted with carboxyl or carbonyl to form N-acyl or N-carbamoyl derivatives.

The term "alkylsulfonyl" refers to groups of the formula (SO₂)-alkyl, in which the sulfur atom is the point of attachment. Preferably, alkylsulfonyl groups include C₁- C₆ alkylsulfonyl groups, which have from 1 to 6 carbon atoms. Methylsulfonyl is one representative alkylsulfonyl group.

The term "heteroatom" refers to any atom other than carbon, for example, N, O, or S.

The term "heteroaryl" herein alone or as part of another group refers to substituted and unsubstituted aromatic 5 or 6 membered monocyclic groups, 9 or 10 membered bicyclic groups, and 11 to 14 membered tricyclic groups which have at least one heteroatom (O, S or N) in at least one of the rings. Each ring of the heteroaryl group containing a heteroatom can contain one or two oxygen or sulfur atoms and/or from one to four nitrogen atoms provided that the total number of heteroatoms in each ring is four or less and each ring has at least one carbon atom.

The term "heterocycle" or "heterocycloalkyl" herein alone or as part of another group refers to a cycloalkyl group (nonaromatic) in which one of the carbon atoms in the ring is replaced by a heteroatom selected from O, S or N. The "heterocycle" has from 1 to 3 fused, pendant or spiro rings, at least one of which is a heterocyclic ring (i.e. , one or more ring atoms is a heteroatom, with the remaining ring atoms being carbon). The heterocyclic ring may be optionally substituted which means that the heterocyclic ring may be substituted at one or more substitutable ring positions by one or more groups independently selected from alkyl (preferably lower alkyl), heterocycloalkyl, heteroaryl, alkoxy (preferably lower alkoxy), nitro, monoalkylamino (preferably a lower alkylamino), dialkylamino (preferably a alkylamino), cyano, halo, haloalkyl (preferably trifluoromethyl), alkanoyl, aminocarbonyl, monoalkylaminocarbonyl, dialkylaminocarbonyl, alkyl amido (preferably lower alkyl amido), alkoxyalkyl (preferably a lower alkoxy; lower alkyl), alkoxycarbonyl (preferably a lower alkoxycarbonyl), alkylcarbonyloxy (preferably a lower alkylcarbonyloxy) and aryl (preferably phenyl), said aryl being optionally substituted by halo, lower alkyl and lower alkoxy groups. A heterocyclic group may generally be linked via any ring or substituent atom, provided that a stable compound results. N-linked heterocyclic groups are linked via a component nitrogen atom.

Typically, a heterocyclic ring comprises 1-4 heteroatoms; within certain embodiments each heterocyclic ring has 1 or 2 heteroatoms per ring. Each heterocyclic ring generally contains from 3 to 8 ring members (rings having from to 7 ring members are recited in certain embodiments), and heterocycles comprising fused, pendant or spiro rings typically contain from 9 to 14 ring members which consists of carbon atoms and contains one, two, or three heteroatoms selected from nitrogen, oxygen and/or sulfur.

Examples of "heterocycle" or "heterocycloalkyl groups include piperazine, piperidine, morpholine, thiomorpholine, pyrrolidine, imidazolidine and thiazolide.

The term "substituent," as used herein, refers to a molecular moiety that is covalently bonded to an atom within a molecule of interest. For example, a "ring substituent" may be a moiety such as a halogen, alkyl group, haloalkyl group or other group discussed herein that is covalently bonded to an atom (preferably a carbon or nitrogen atom) that is a ring member.

The term "optionally substituted " as it refers that the aryl or heterocyclyl or other group may be substituted at one or more substitutable positions by one or more groups independently selected from alkyl (preferably lower alkyl), alkoxy (preferably lower alkoxy), nitro, monoalkylamino (preferably with one to six carbons), dialkylamino (preferably with one to six carbons), cyano, halo, haloalkyl (preferably trifluoromethyl), alkanoyl, aminocarbonyl, monoalkylaminocarbonyl, dialkylaminocarbonyl, alkyl amido (preferably lower alkyl amido), alkoxyalkyl (preferably a lower alkoxy and lower alkyl), alkoxycarbonyl (preferably a lower alkoxycarbonyl), alkylcarbonyloxy (preferably a lower alkylcarbonyloxy) and aryl (preferably phenyl), said aryl being optionally substituted by halo, lower alkyl and lower alkoxy groups. Optional substitution is also indicated by the phrase "substituted with from 0 to X substituents," where X is the maximum number of possible substituents. Certain optionally substituted groups are substituted with from 0 to 2, 3 or 4 independently selected substituents.

A dash ("-") that is not between two letters or symbols is used to indicate a point of t attachment for a substituent. For example, -CONH₂ is attached through the carbon atom.

A dashed cycle that locates inside of a heterocyle ring is used to indicate a conjugated system. The bonds between two atoms may be single bond or double bond. The term "kinase" refers to any enzyme that catalyzes the addition of phosphate groups to a protein residue; for example, serine and threonine kinases catalyze the addition of phosphate groups to serine and threonine residues.

The term "therapeutically effective amount" refers to the amount of the compound or pharmaceutical composition that will elicit the biological or medical response of a tissue, system, animal or human that is being sought by the researcher, veterinarian, medical doctor or other clinician, e.g., restoration or maintenance of vasculostasis or prevention of the compromise or loss or vasculostasis; reduction of tumor burden; reduction of morbidity and/or mortality.

The term "pharmaceutically acceptable" refers to the fact that the carrier, diluent or excipient is compatible with the other ingredients of the formulation and not deleterious to the recipient thereof.

The terms "administration of a compound" or "administering a compound" refer to the act of providing a compound of the invention or pharmaceutical composition to the subject in need of treatment.

The term "protected" refers that the group is in modified form to preclude undesired side reactions at the protected site. Suitable protecting groups for the compounds of the present invention will be recognized from the present application taking into account the level of skill in the art, and with reference to standard textbooks, such as Greene, T. W. et al., Protective Groups in Organic Synthesis, John Wiley & Sons, New York (1999).

The term "pharmaceutically acceptable salt" of a compound recited herein is an acid or base salt that is suitable for use in contact with the tissues of human beings or animals without excessive toxicity or carcinogenicity, and preferably without irritation, allergic response, or other problem or complication. Such salts include mineral and organic acid salts of basic residues such as amines, as well as alkali or organic salts of acidic residues such as carboxylic acids. Specific pharmaceutical salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2- hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC- (CH₂)n-COOH where n is 0-4, and the like. Similarly, pharmaceutically acceptable cations include, but are not limited to sodium, potassium, calcium, aluminum, lithium and ammonium. Those of ordinary skill in the art will recognize further pharmaceutically acceptable salts for the compounds provided herein. In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two; generally, the use of nonaqueous media, such as ether, ethyl acetate, ethanol, isopropanol or acetonitrile, is preferred. It will be apparent that each compound of Formula I may, but need not, be formulated as a hydrate, solvate or non- covalent complex. In addition, the various crystal forms and polymorphs are within the the present disclosure. Also disclosed herein are prodrugs of the compounds of Formula I.

Preferred W groups of formula (I) are: F, Cl, Br, CN, CF₃, CF₂H, CFH₂, CH₃, OCH₃, NH₂ and the list below:

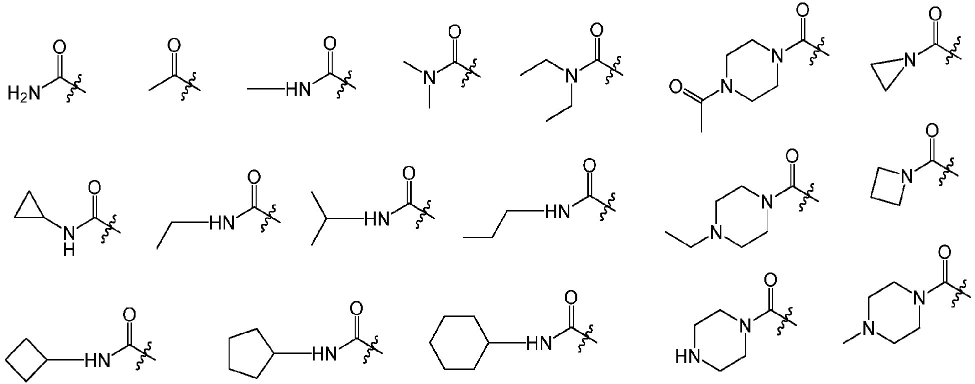

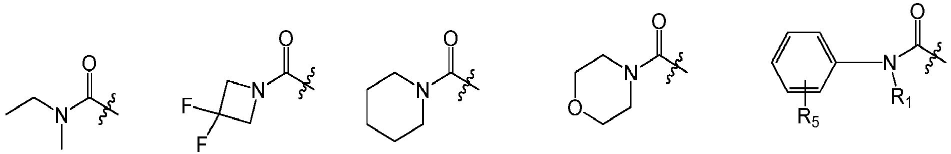

Preferred substituted indole groups of formula (I) are listed below:

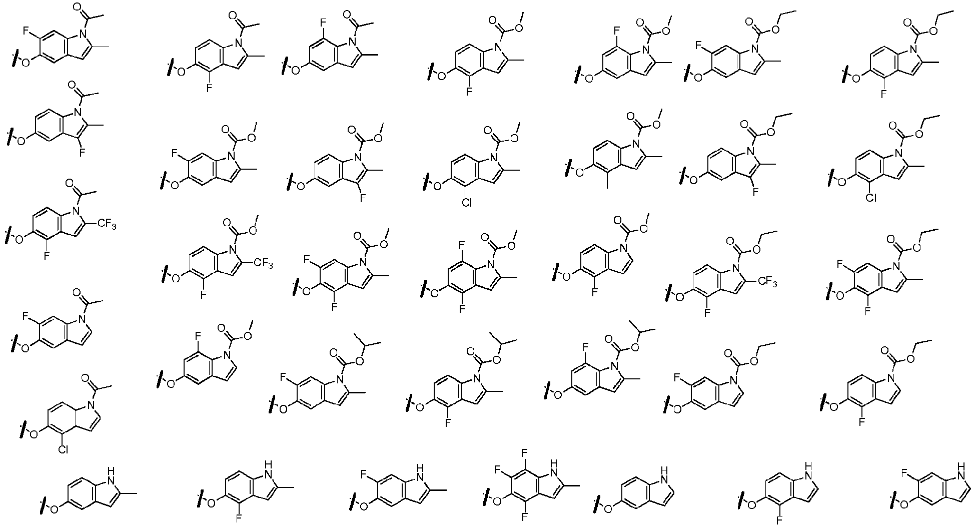

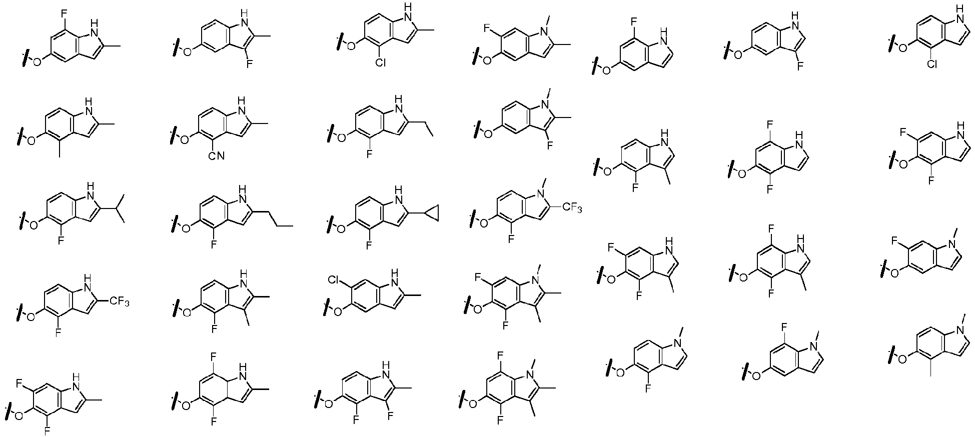

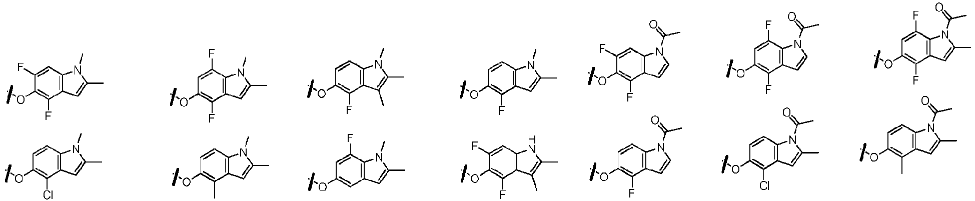

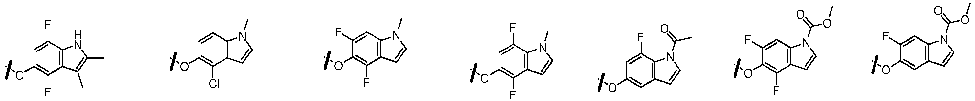

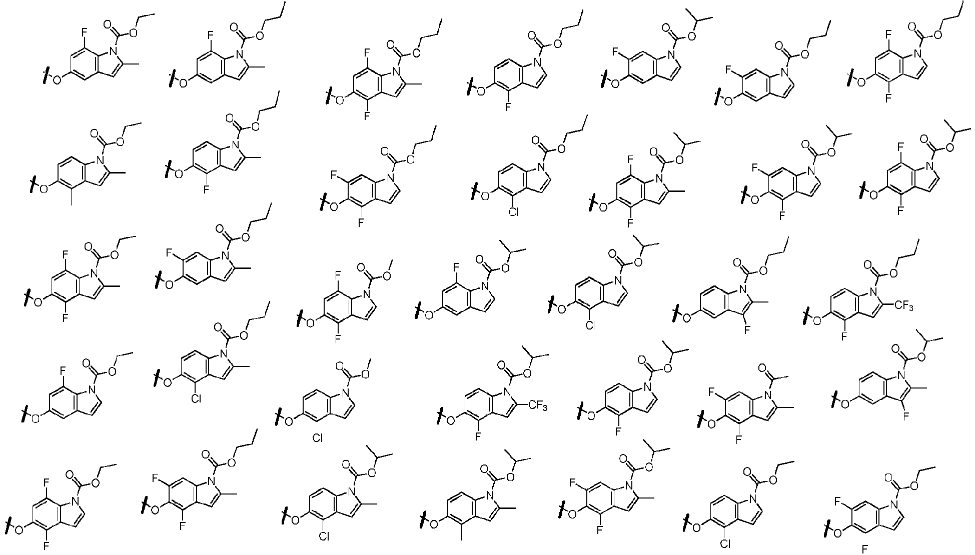

Preferred Ar groups of formula (I) are as below:

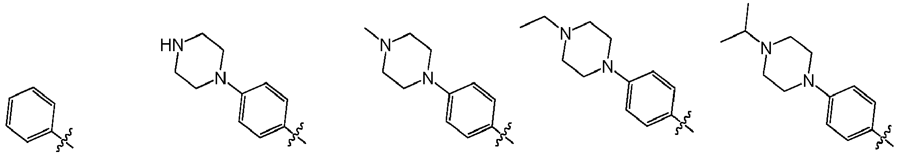

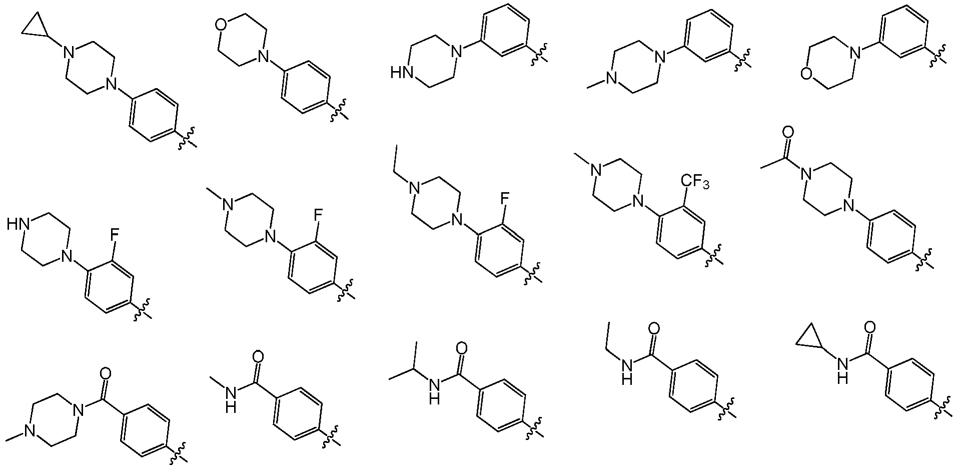

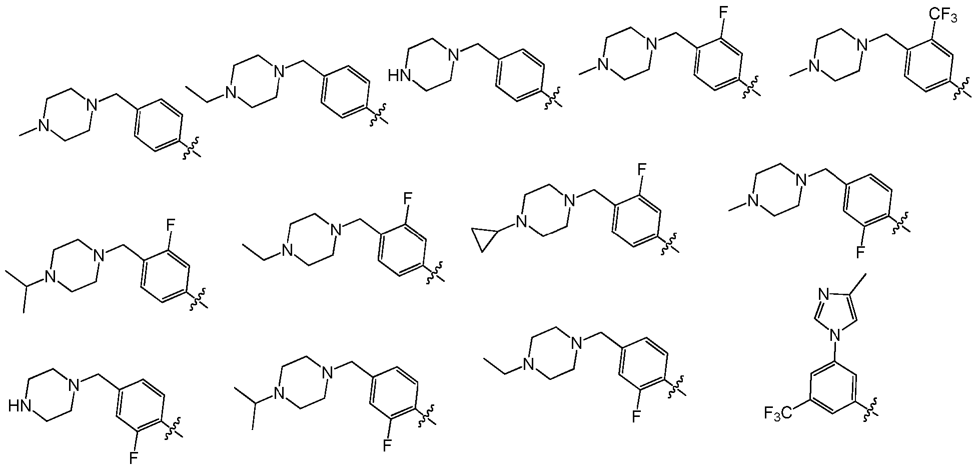

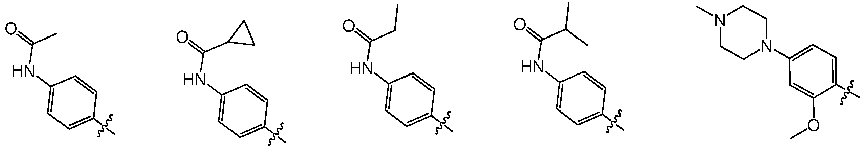

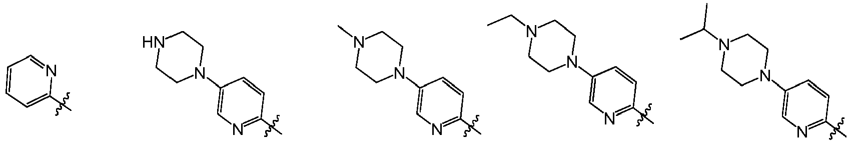

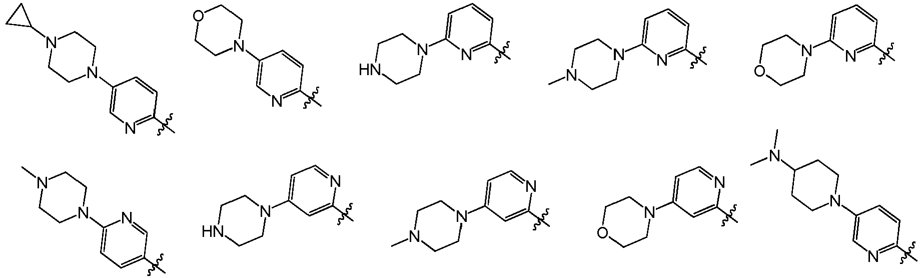

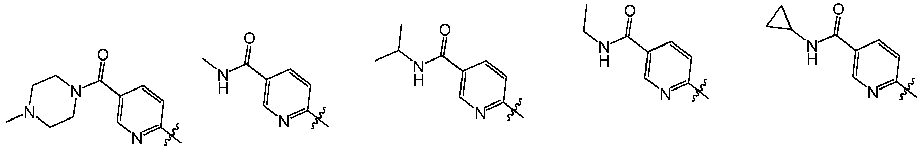

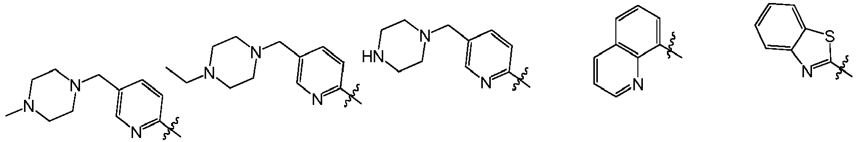

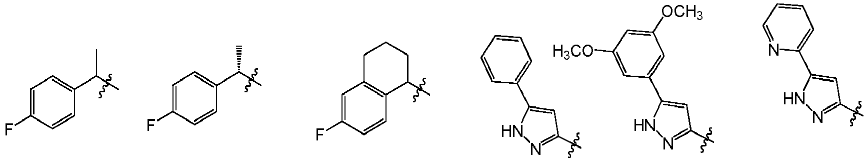

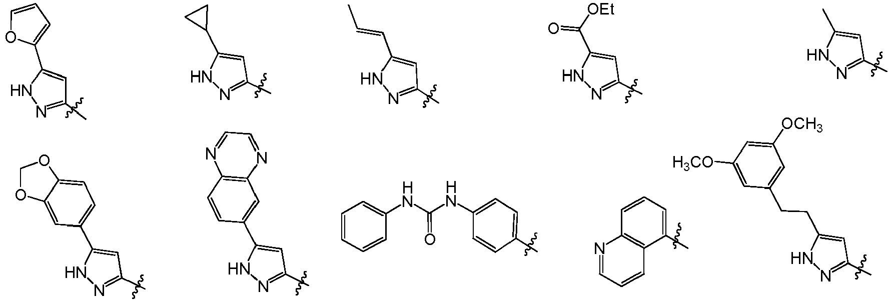

Examples of specific compounds of the present disclosure are those compounds defined in the following:

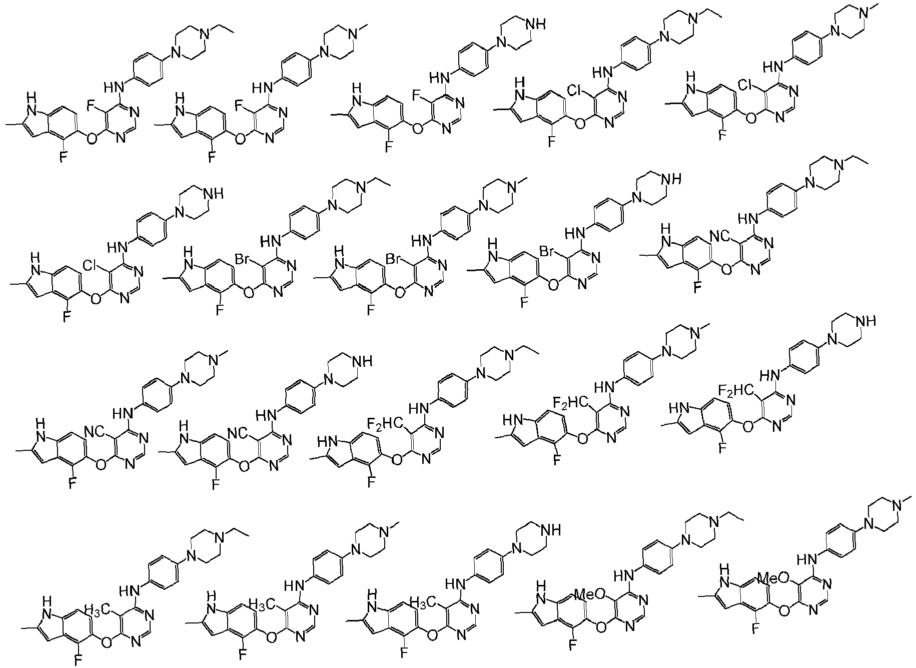

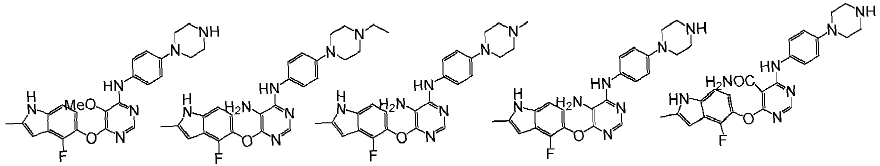

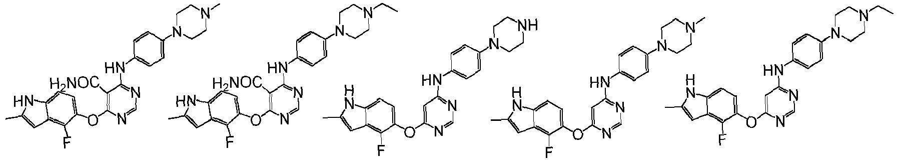

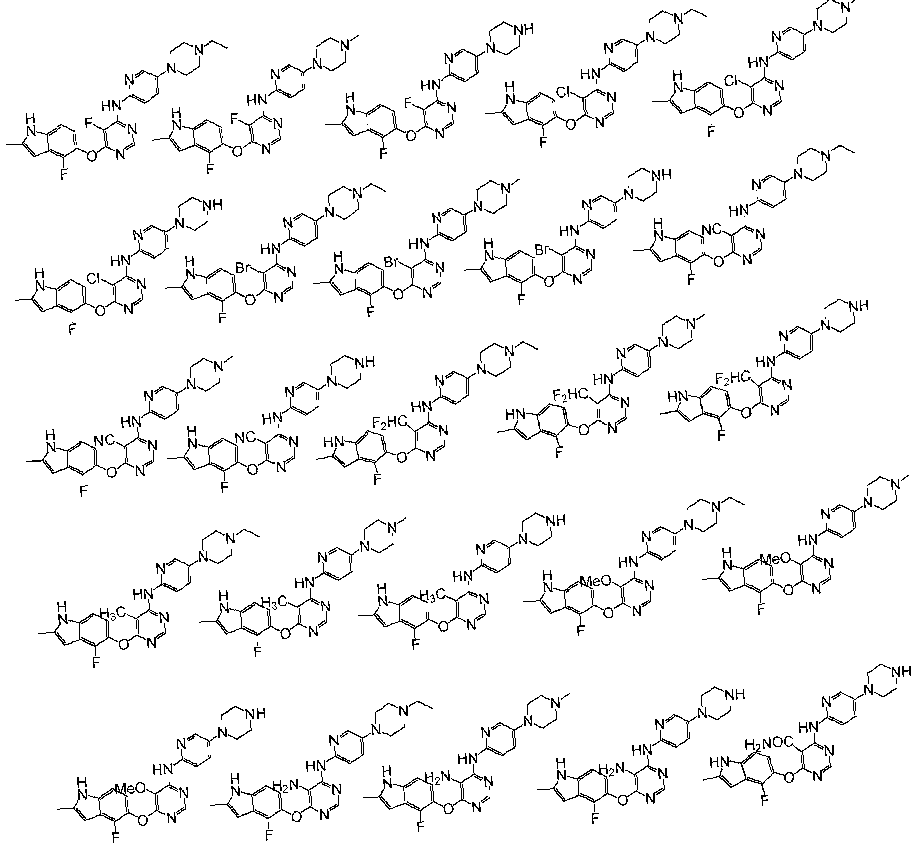

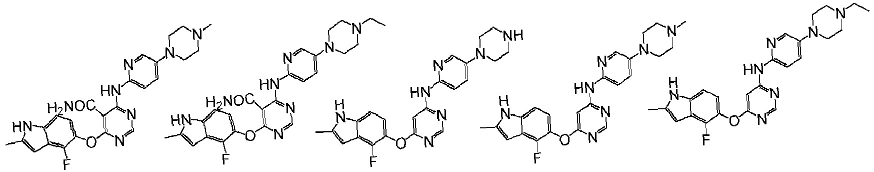

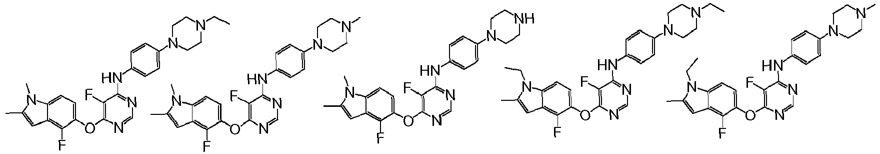

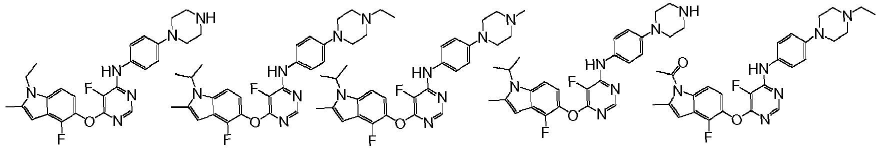

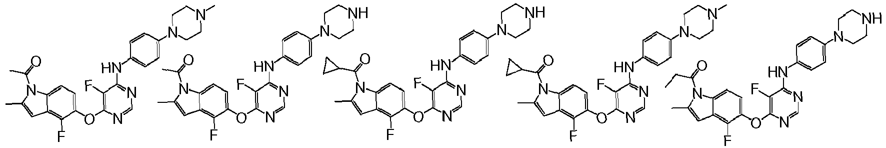

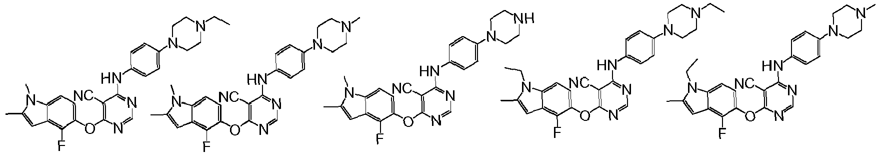

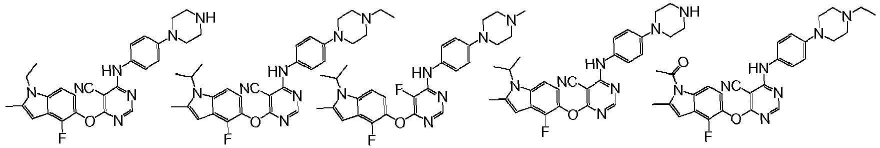

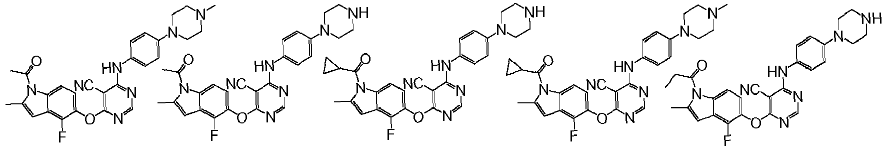

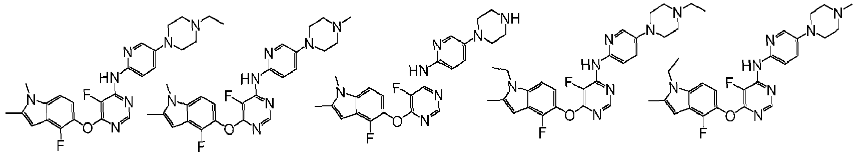

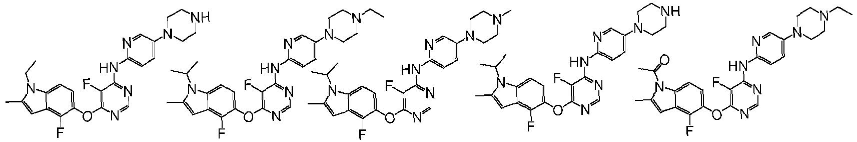

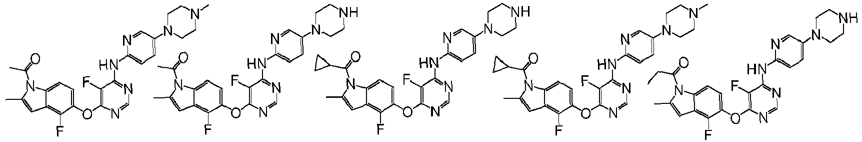

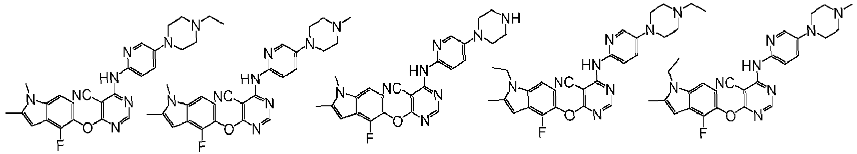

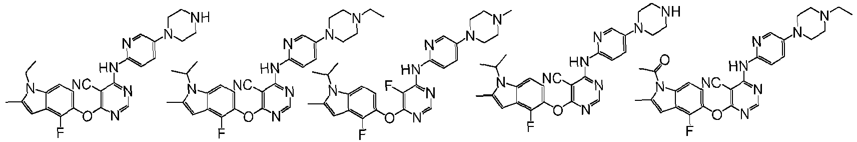

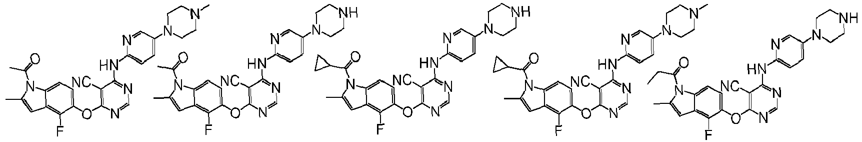

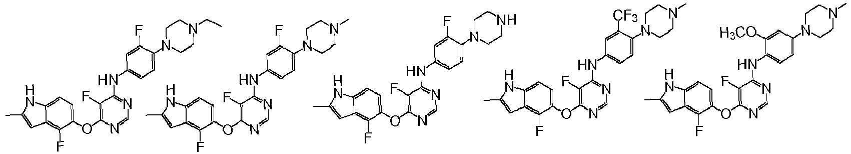

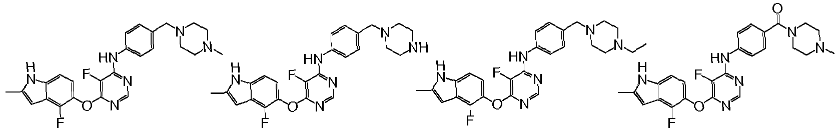

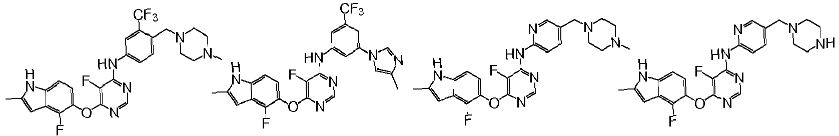

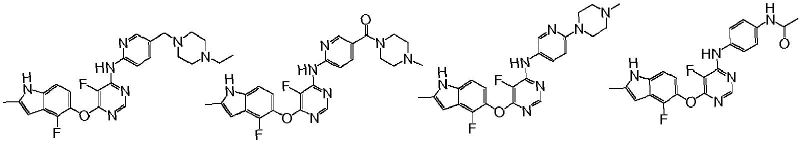

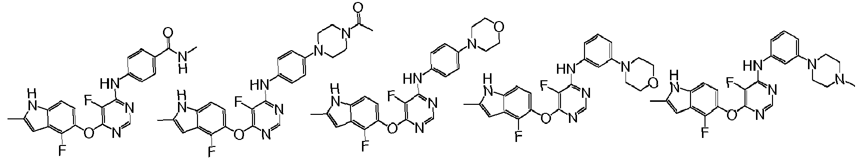

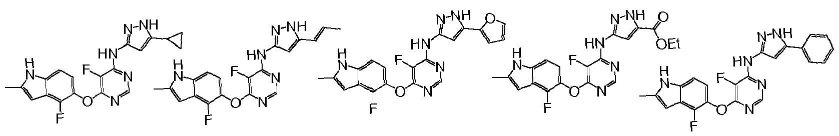

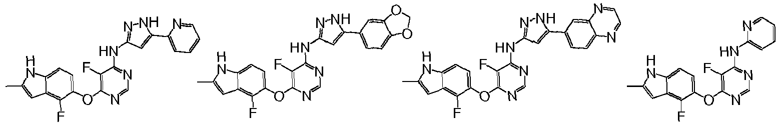

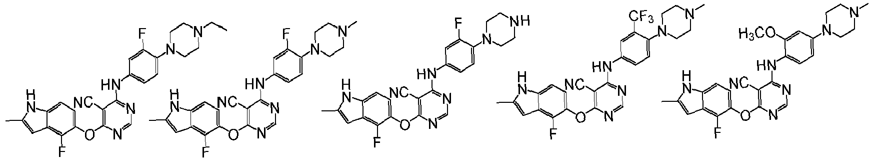

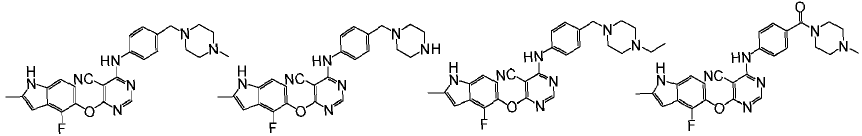

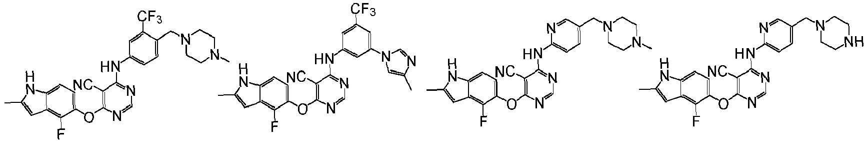

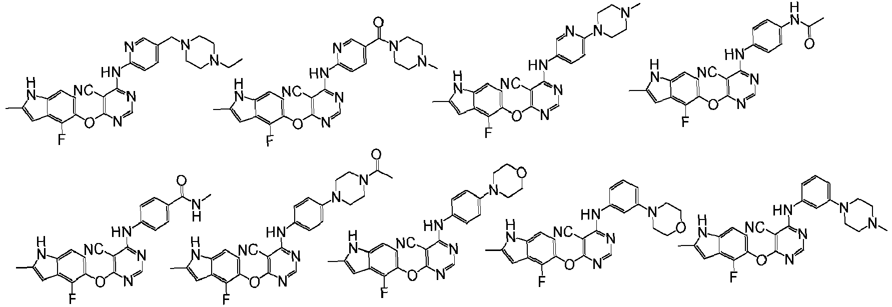

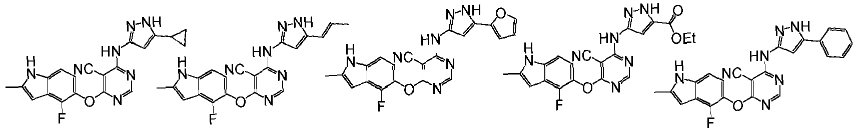

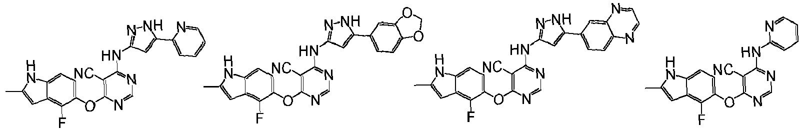

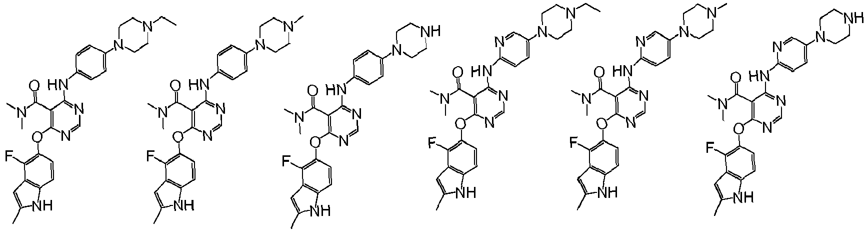

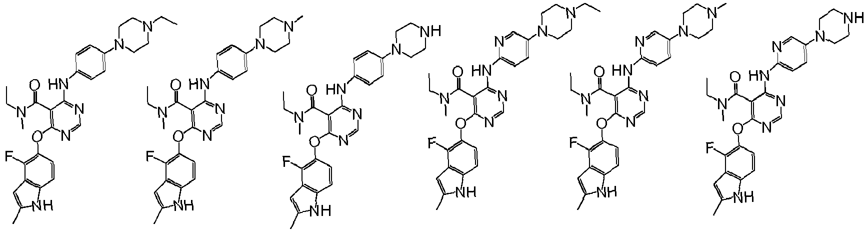

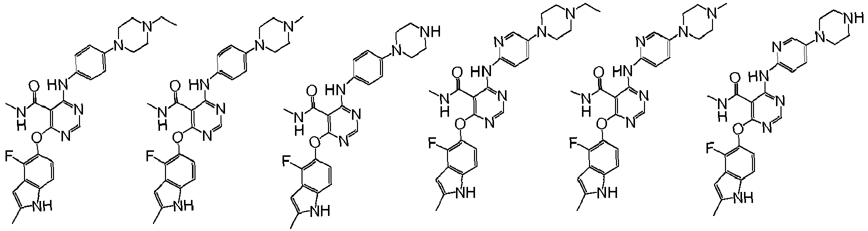

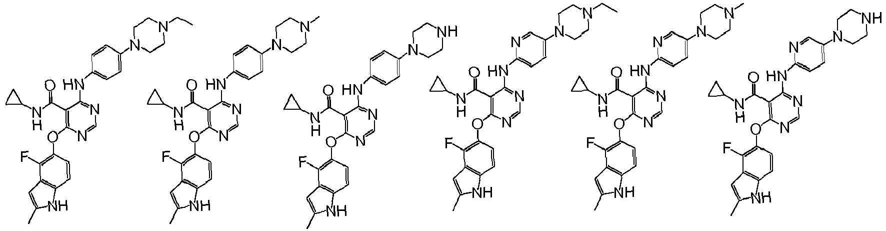

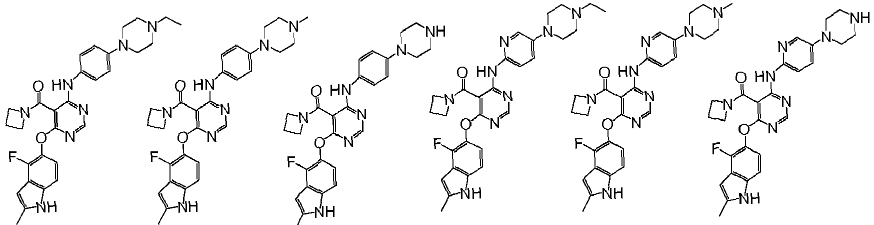

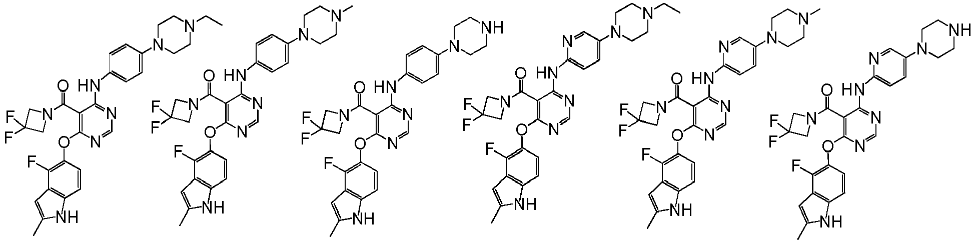

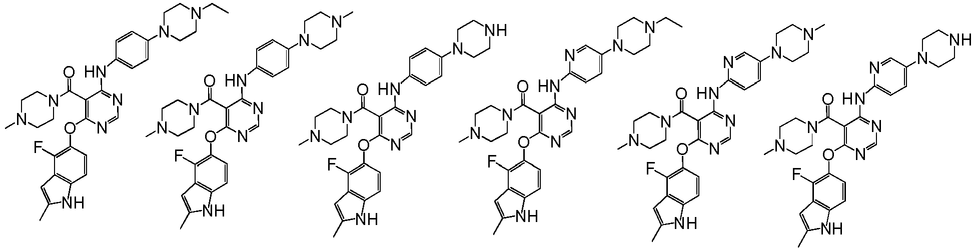

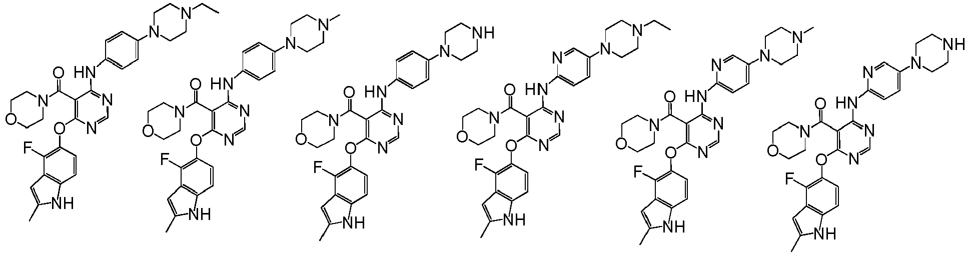

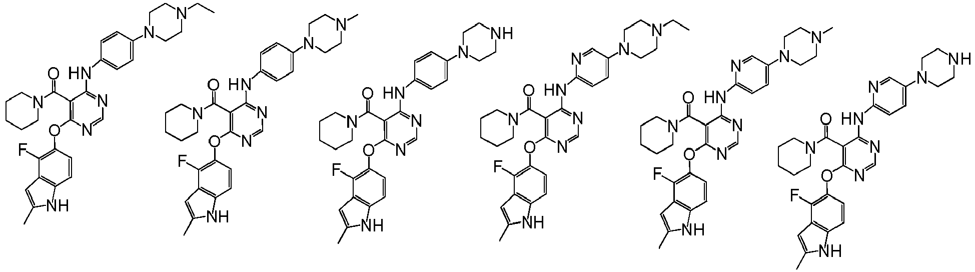

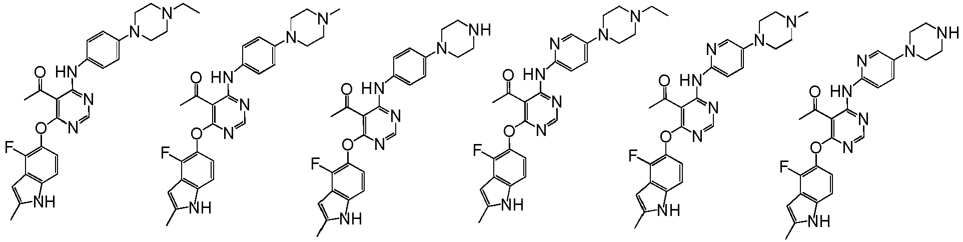

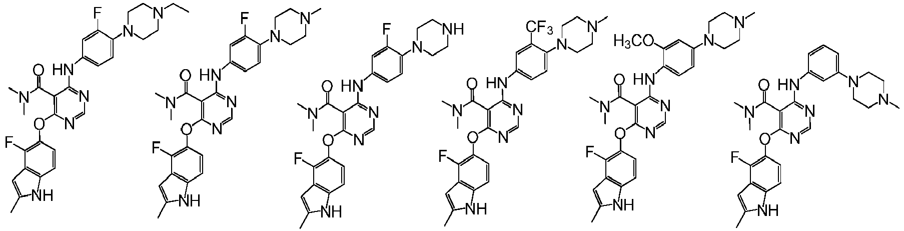

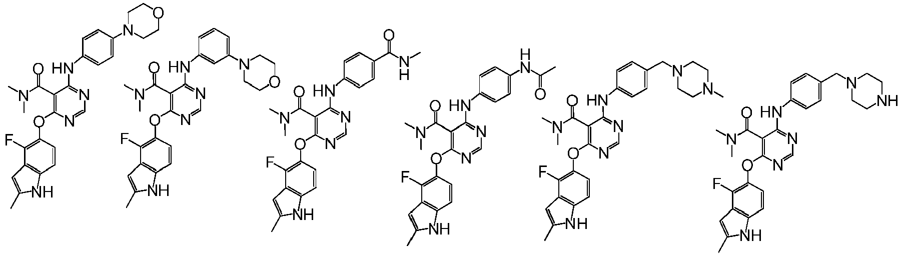

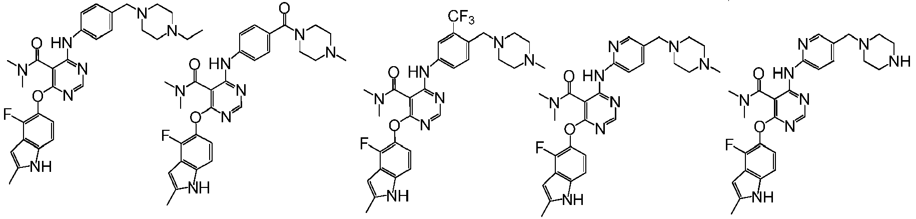

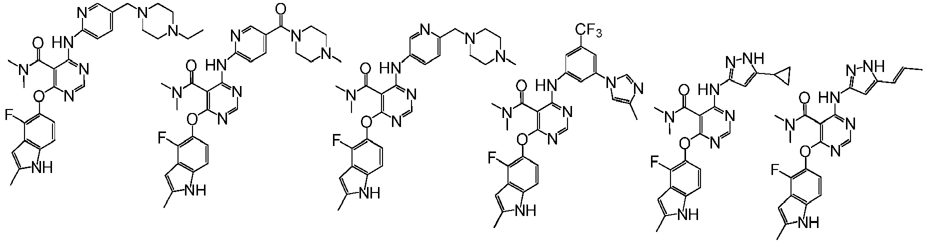

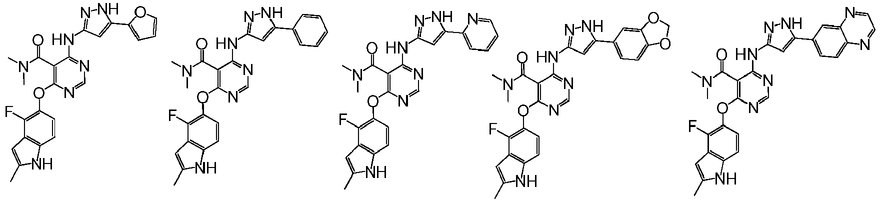

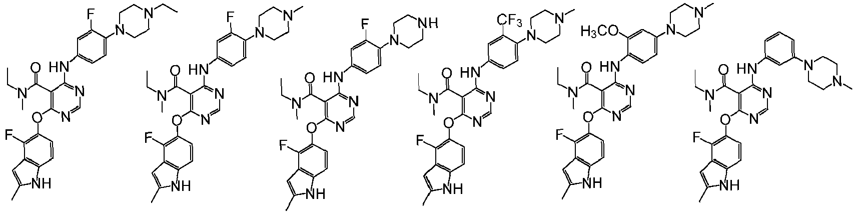

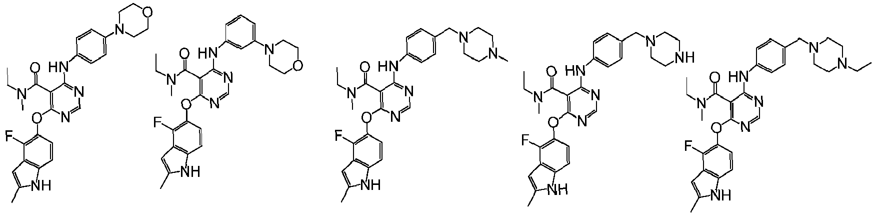

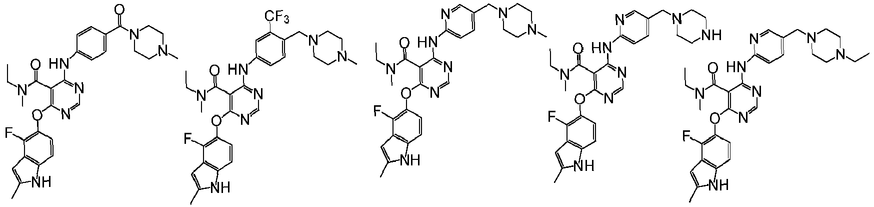

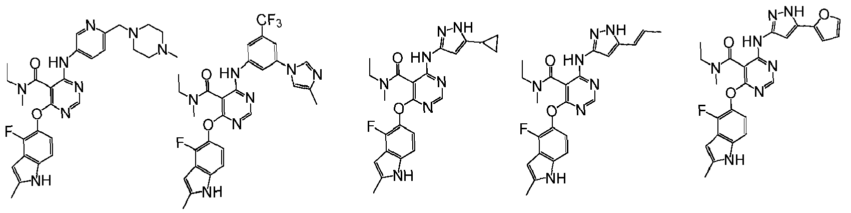

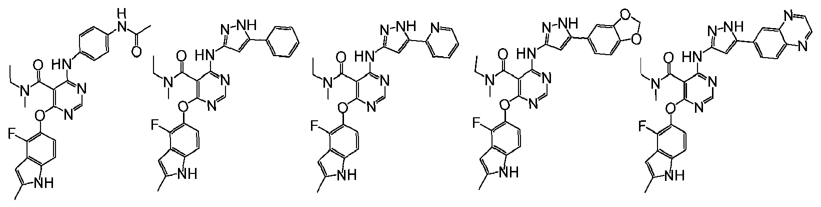

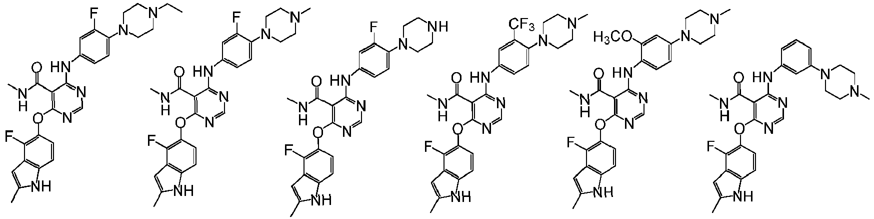

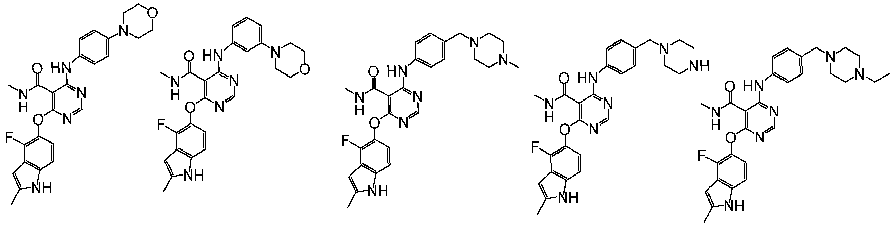

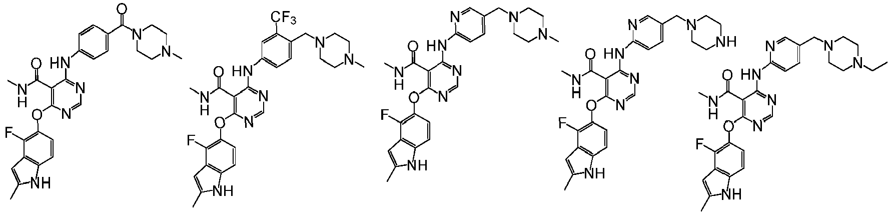

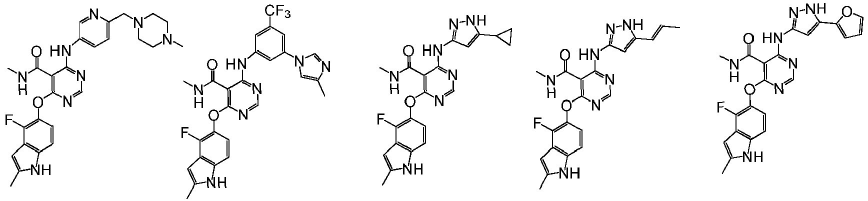

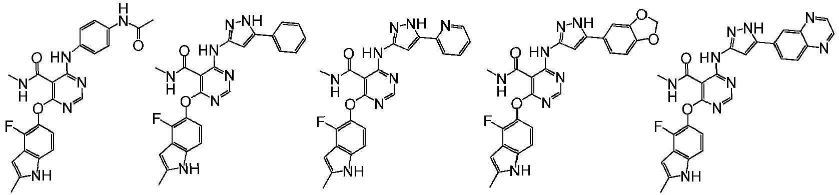

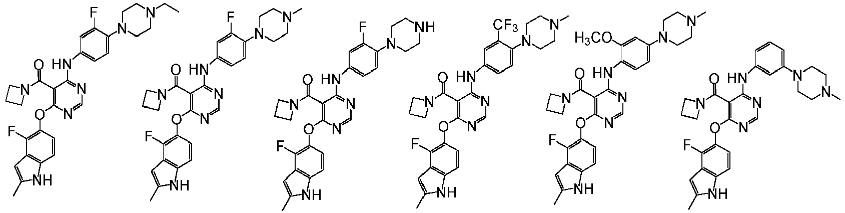

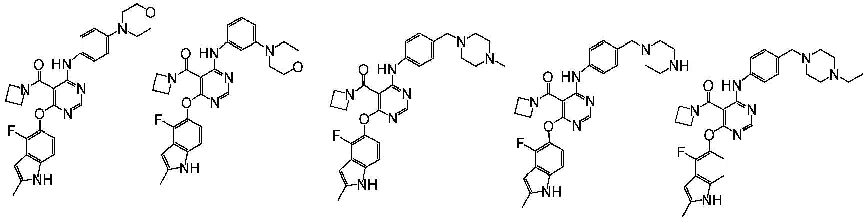

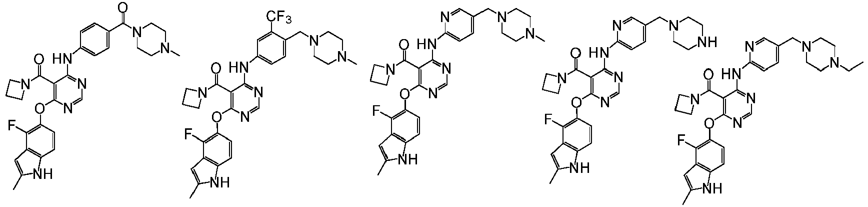

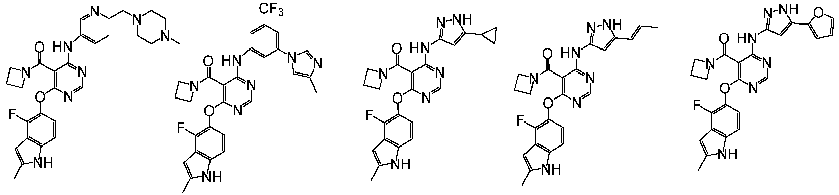

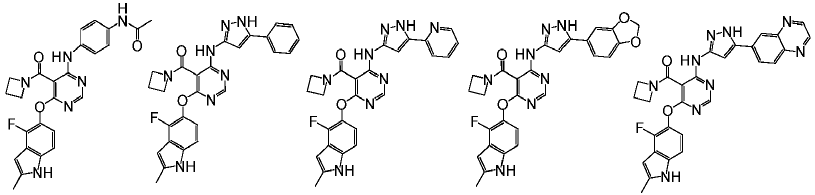

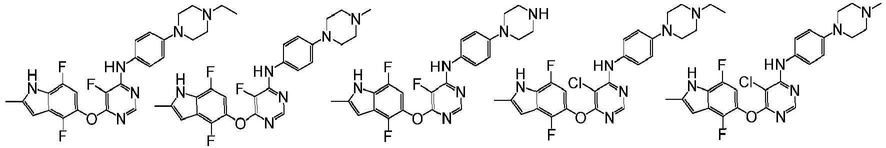

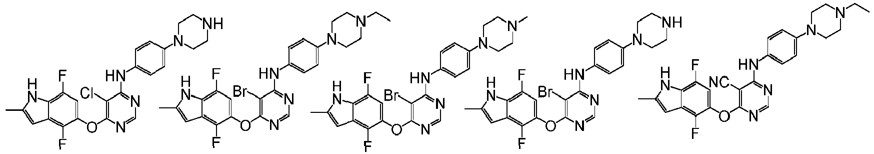

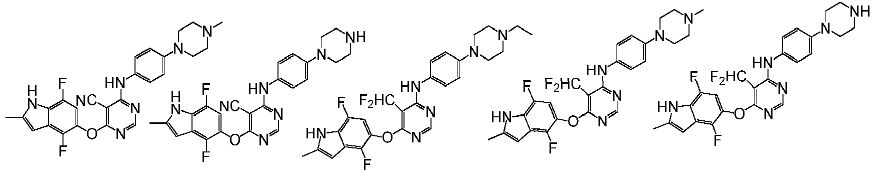

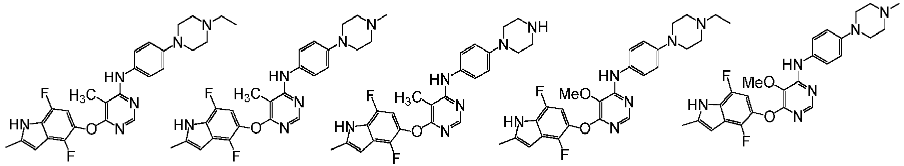

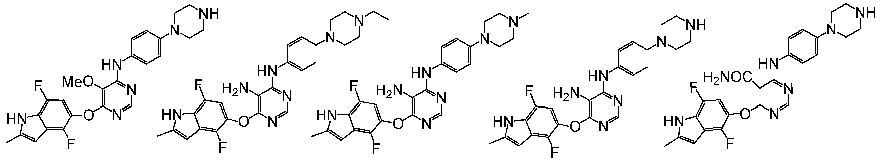

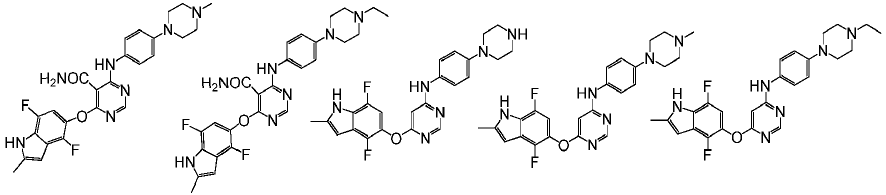

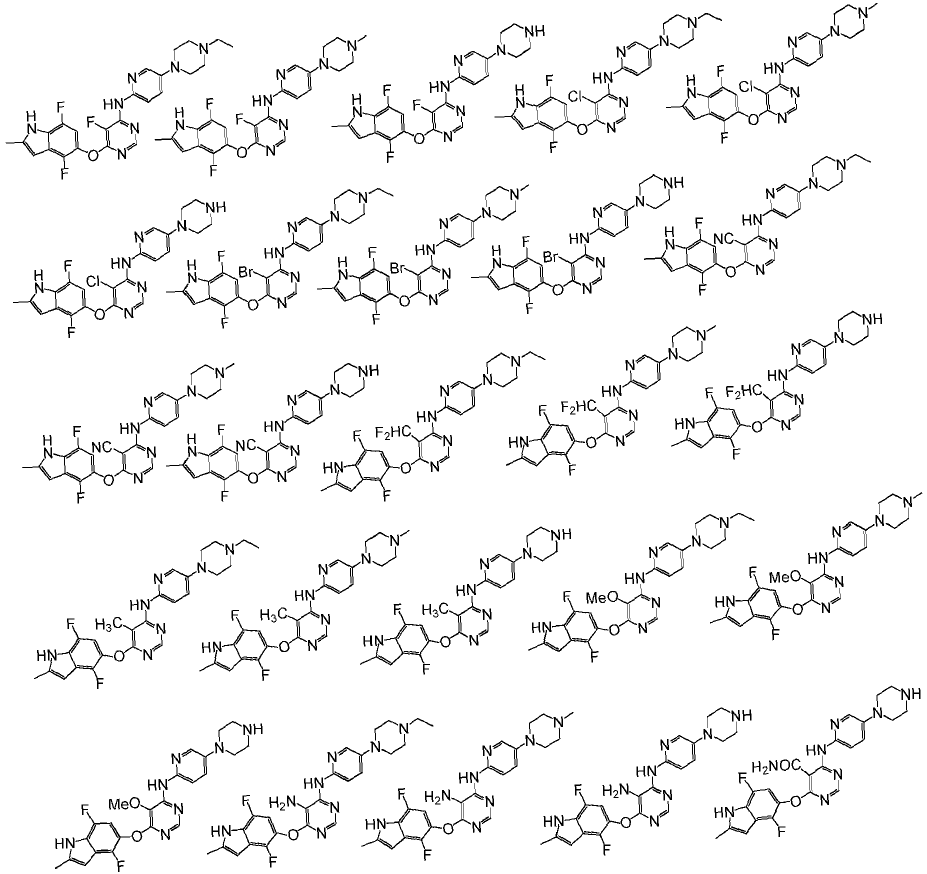

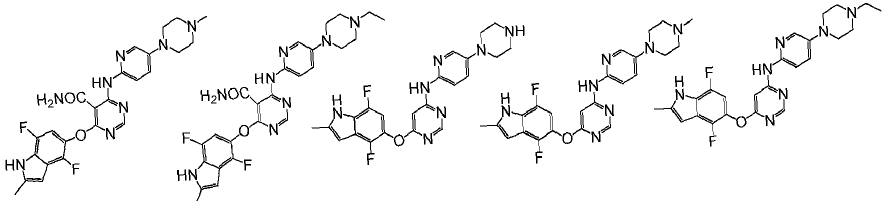

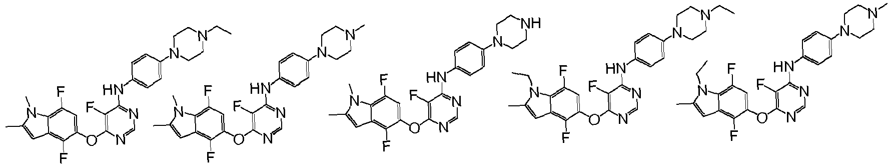

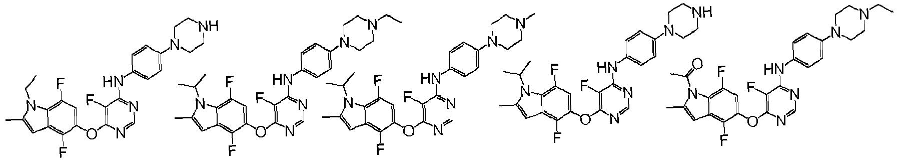

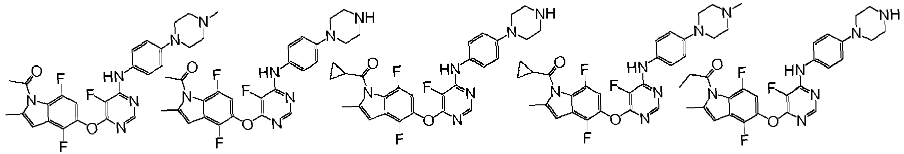

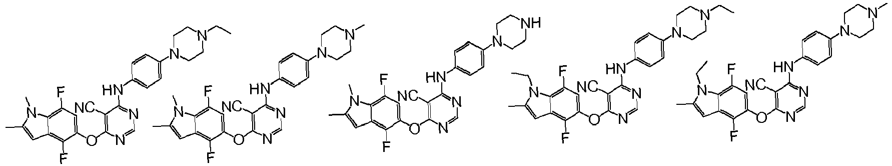

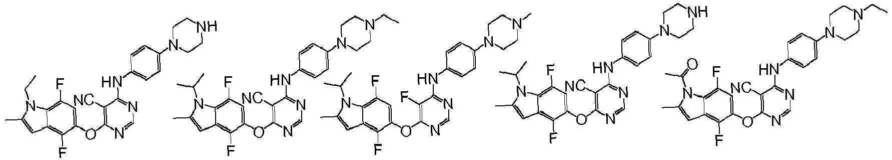

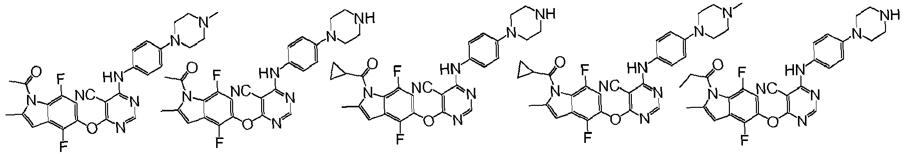

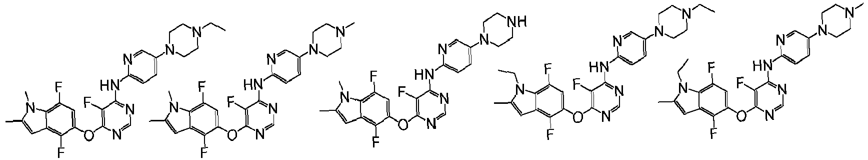

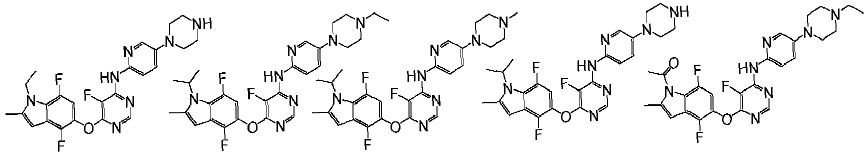

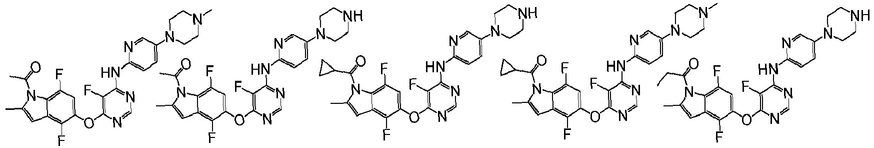

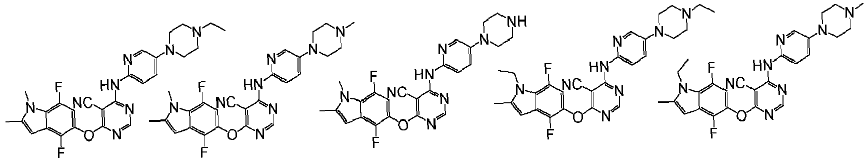

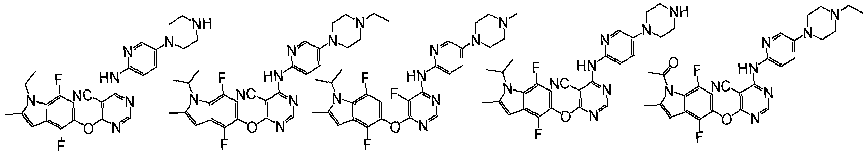

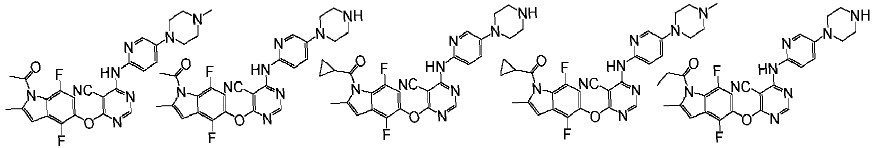

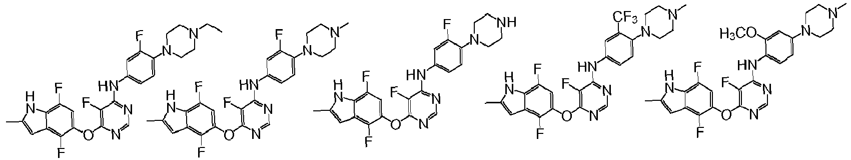

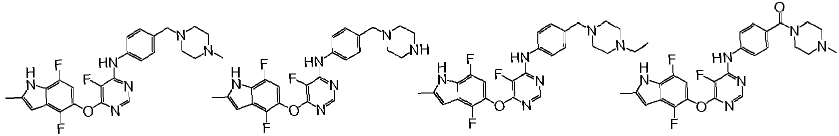

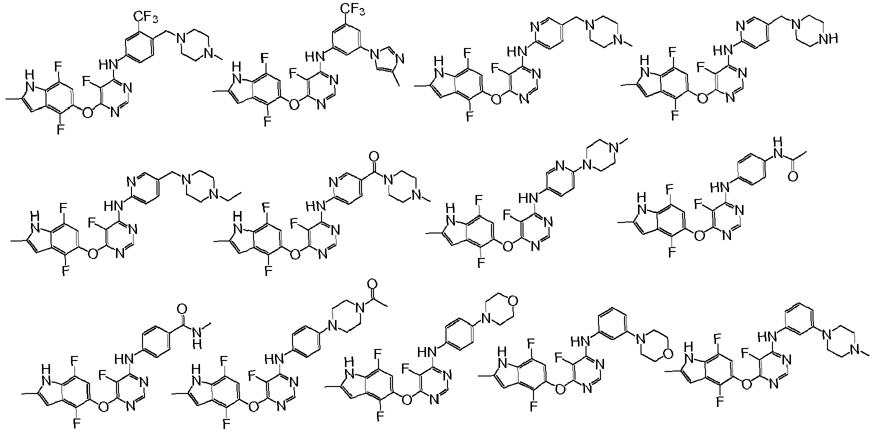

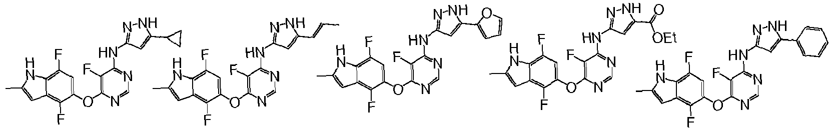

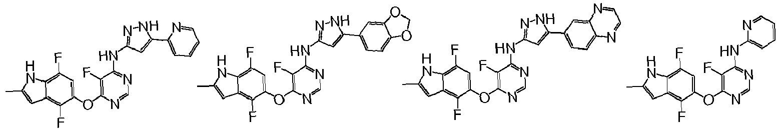

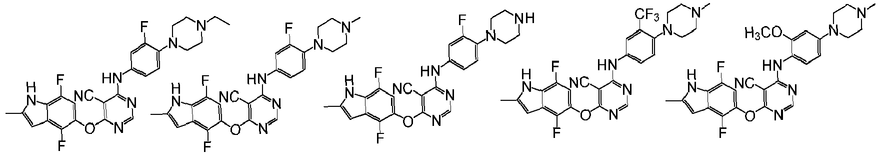

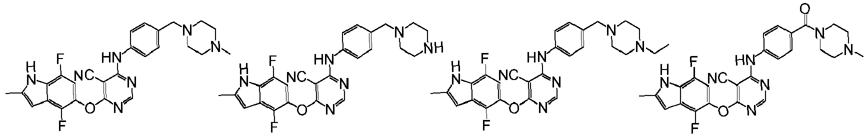

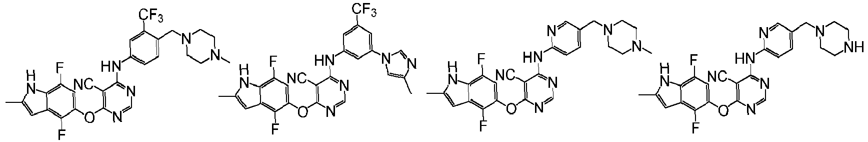

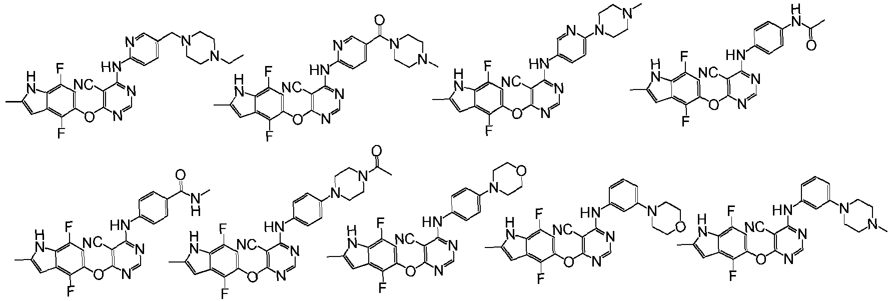

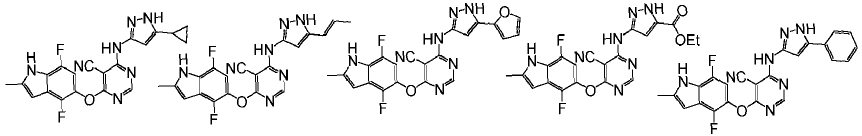

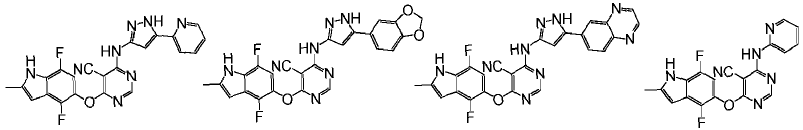

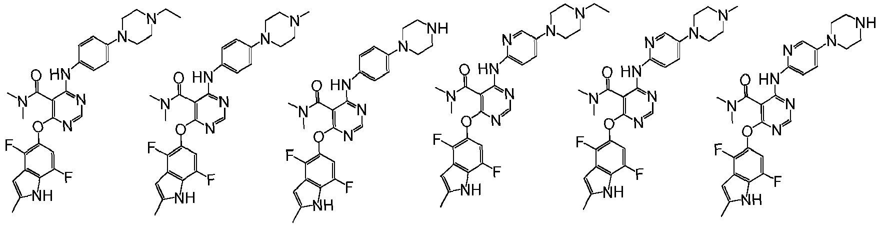

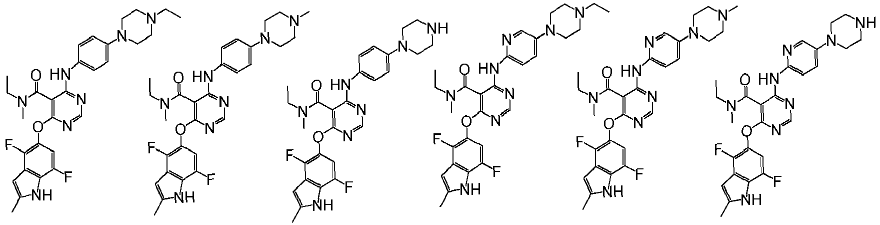

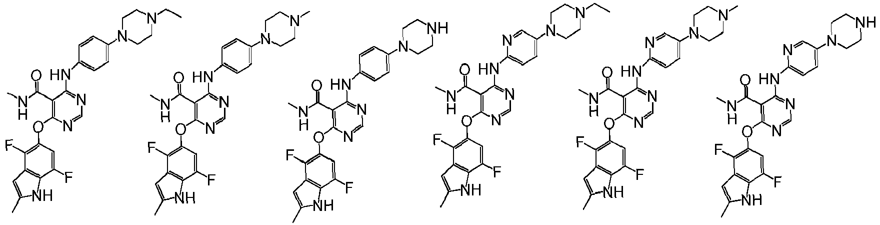

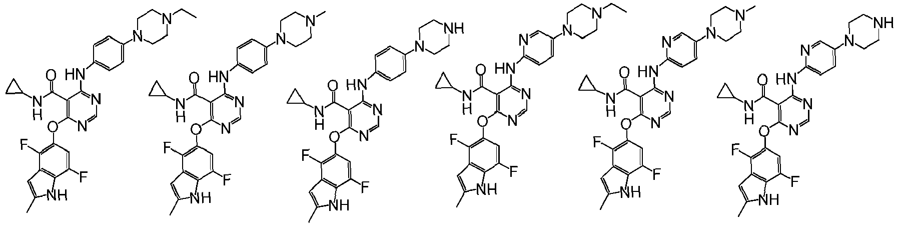

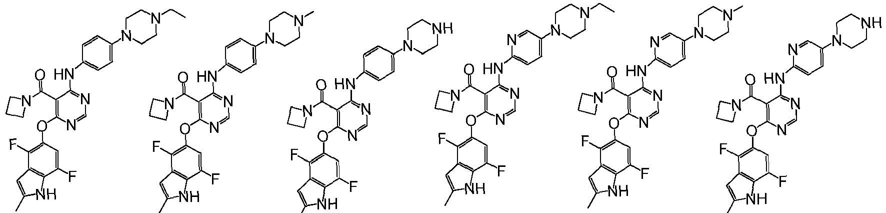

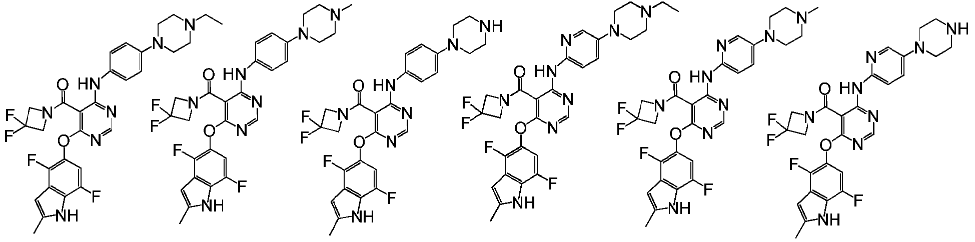

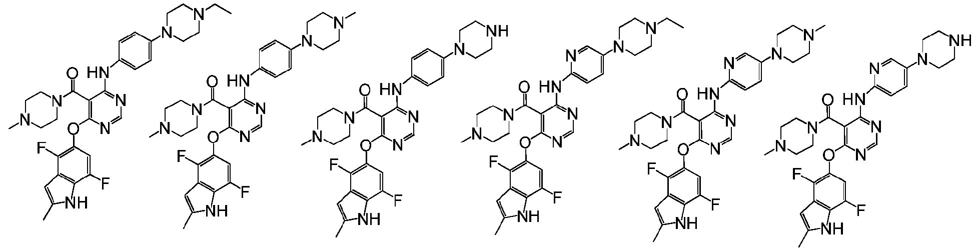

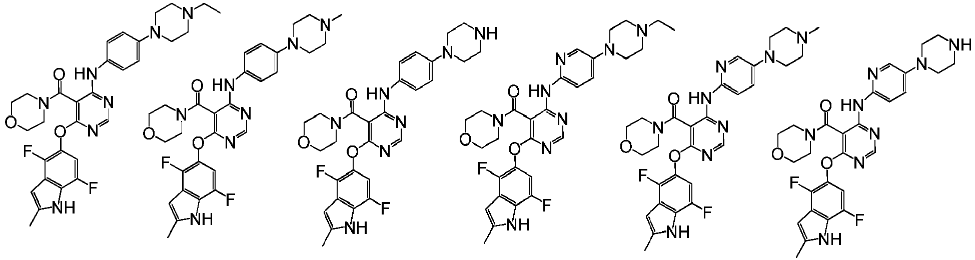

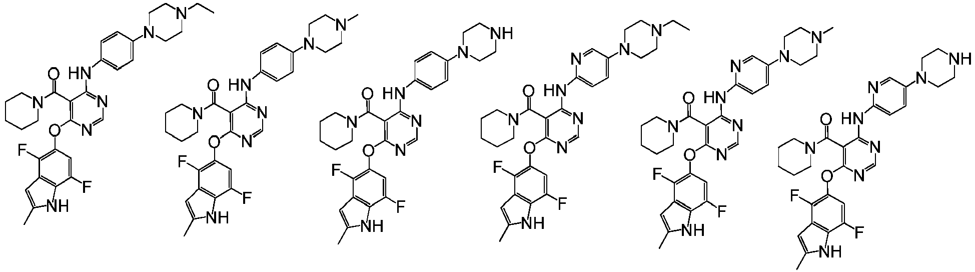

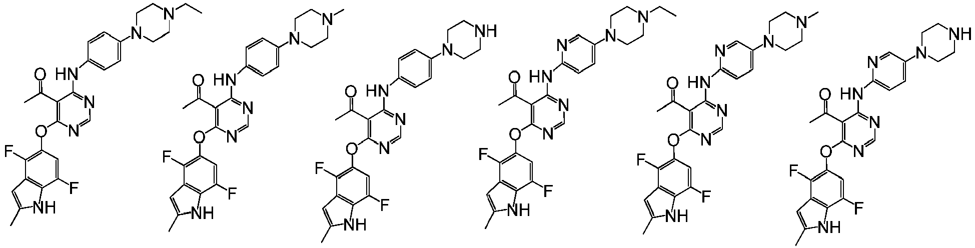

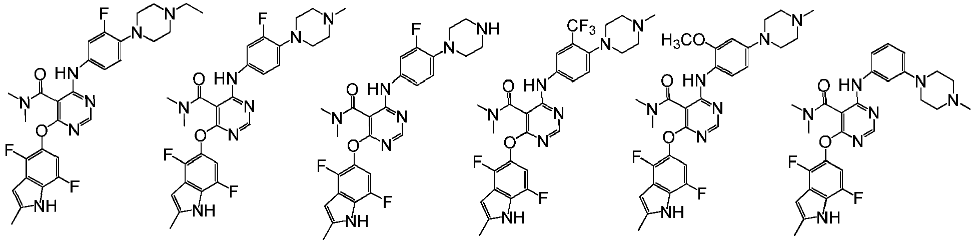

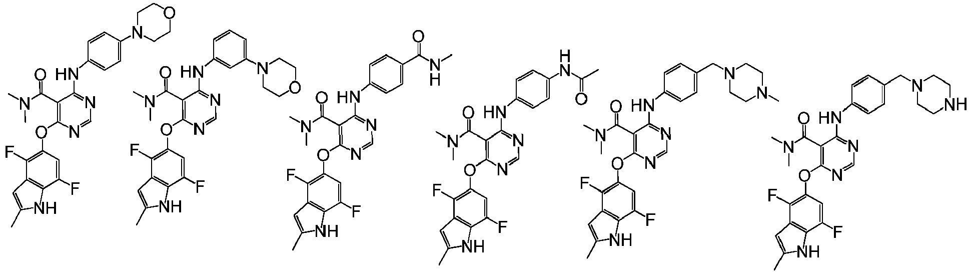

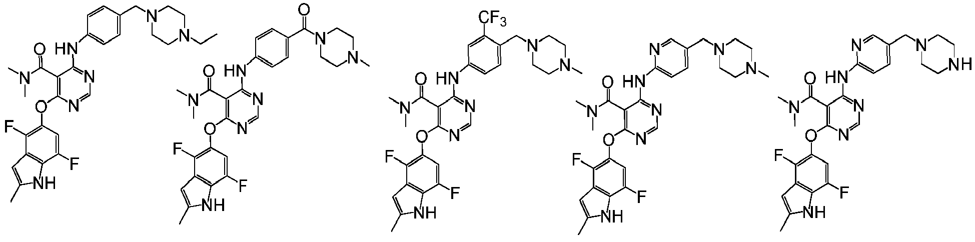

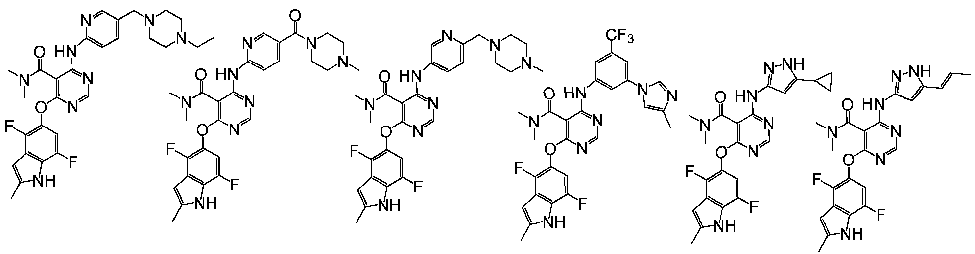

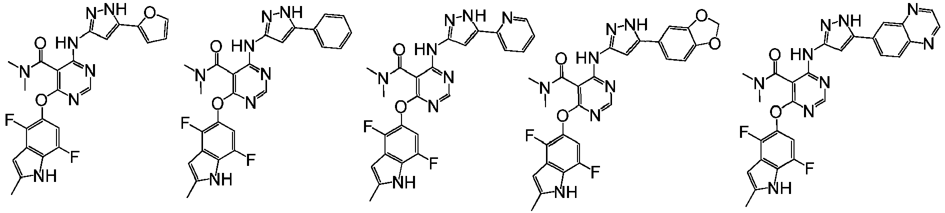

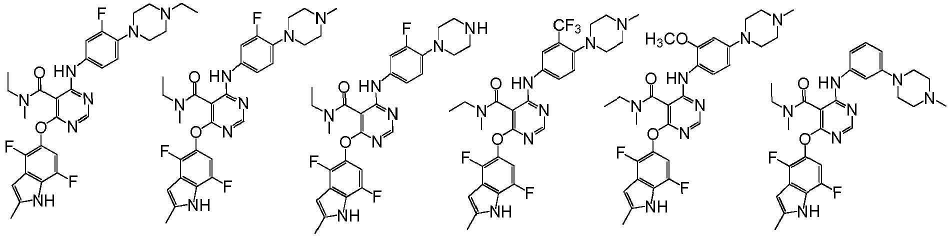

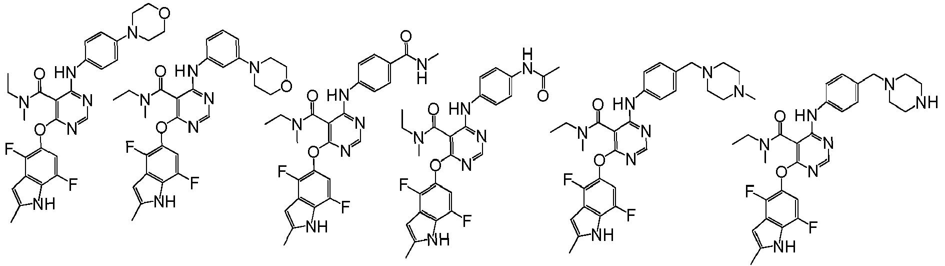

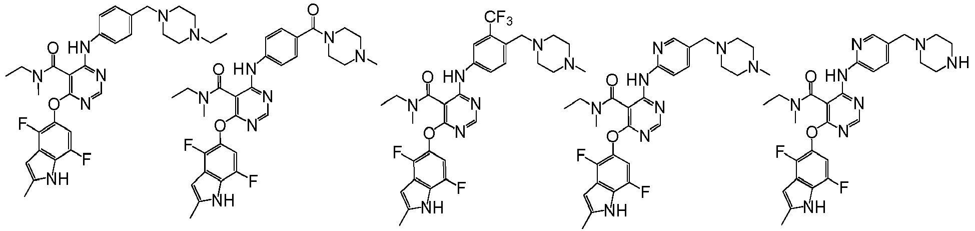

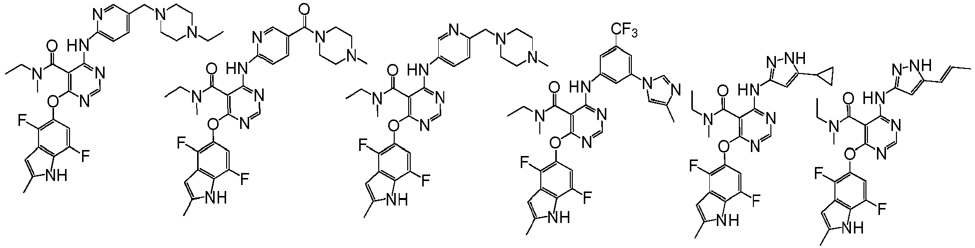

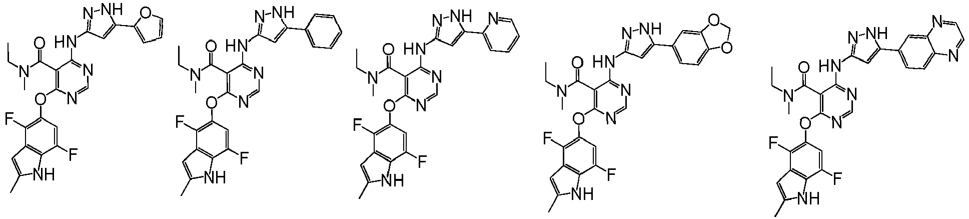

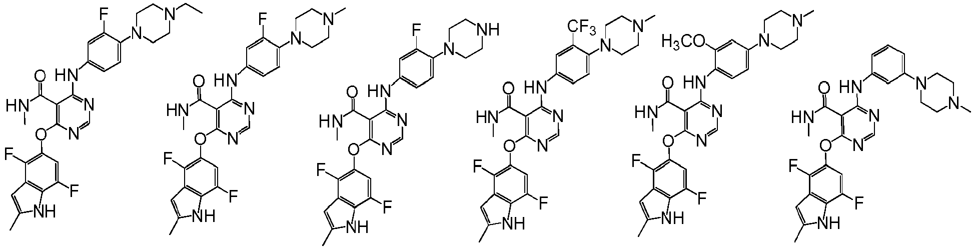

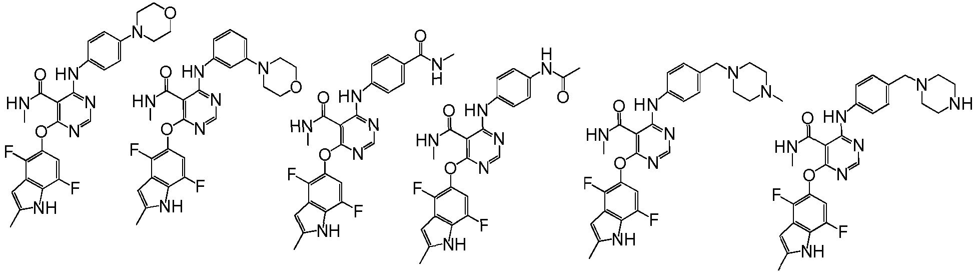

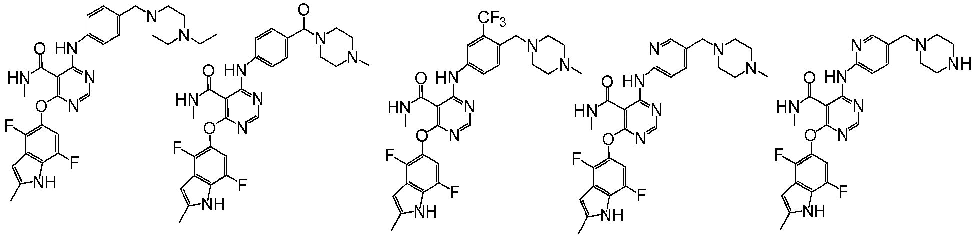

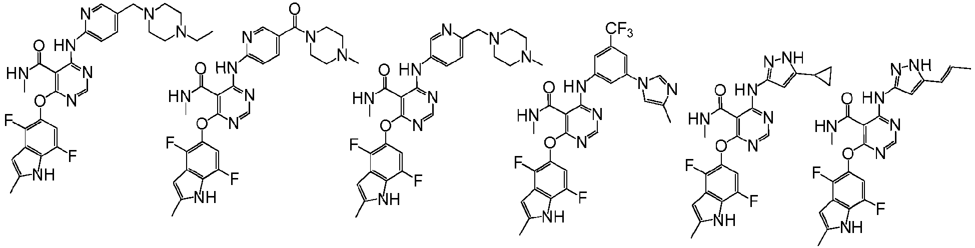

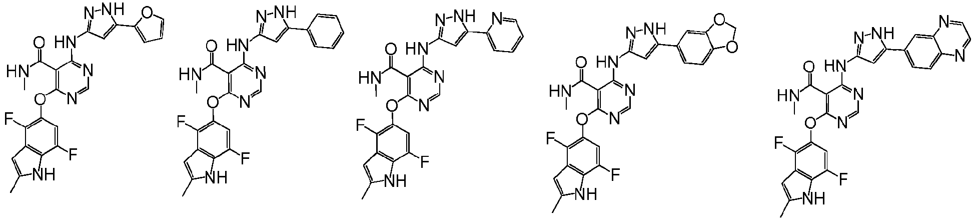

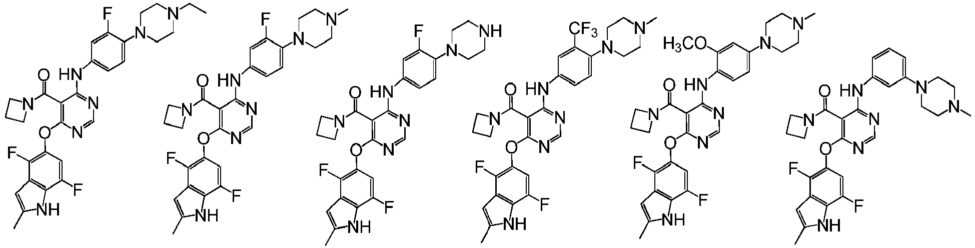

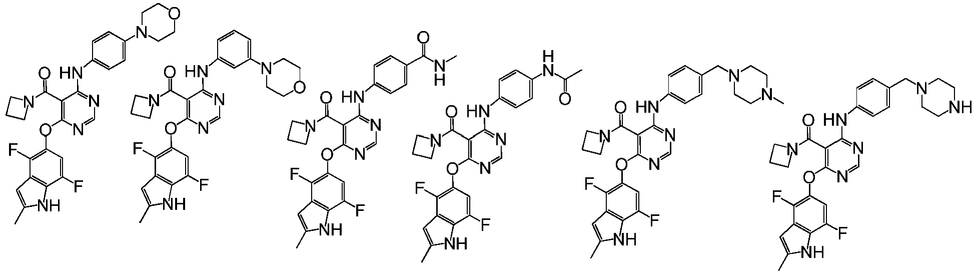

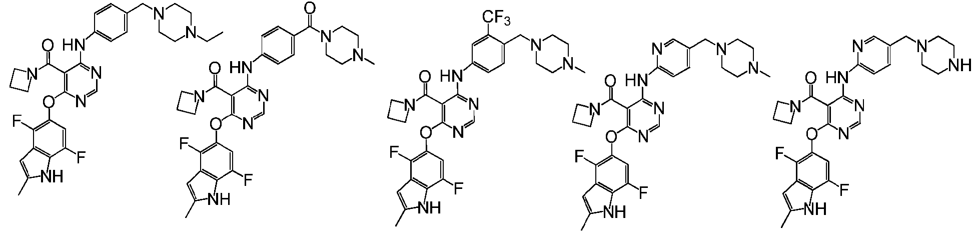

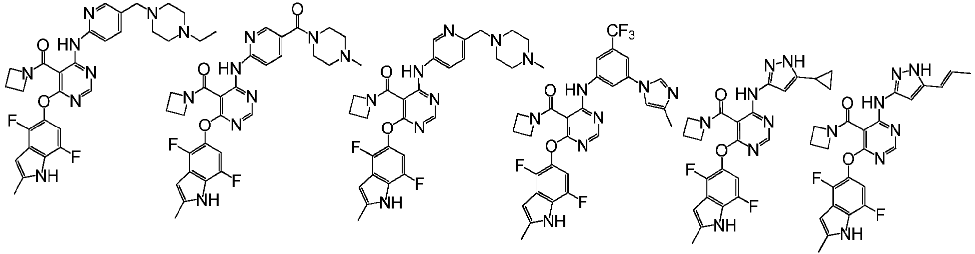

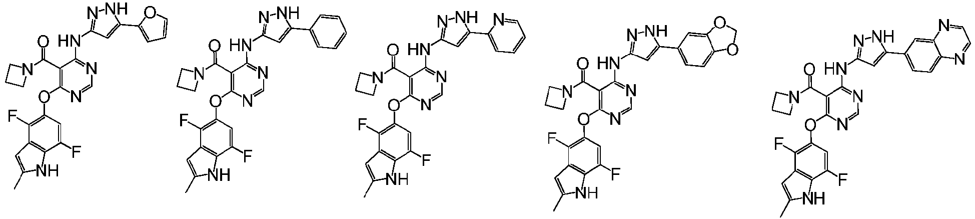

In another embodiment, a method of preparing the inventive compounds is provided. The compounds of the present invention can be generally prepared using 4, 6-dichloro-pyrimidine, with various substituents on position"5". Compound (I) may contain various stereoisomers, geometric isomers, tautomeric isomers, and the like. All of possible isomers and their mixtures are included in the present invention, and the mixing ratio is not particularly limited.

The pyrimidine derivative compounds of Formula (I) in this invention can be synthesized from commercially available precursors using established protocols. By way of example, a synthetic route similar to that shown in any of the following Schemes may be used, together with synthetic methods known in the art of synthetic organic chemistry, or variations thereon as appreciated by those skilled in the art. Each variable in the following schemes refers to any group consistent with the description of the compounds provided herein.

In the Schemes that follow the term "reduction" refers to the process of reducing a nitro functionality to an amino functionality, or the process of transforming an ester functionality to an alcohol. The reduction of a nitro group can be carried out in a number of ways well known to those skilled in the art of organic synthesis including, but not limited to, catalytic hydrogenation, reduction with SnCI2 and reduction with titanium bichloride. In the Schemes that follow, the term "hydrolyze" refers to the reaction of a substrate or reactant with water. More specifically, "hydrolyze" refers to the conversion of an ester or nitrite functionality into a carboxylic acid. This process can be catalyzed by a variety of acids or bases well known to those skilled in the art of organic synthesis.

The compounds of Formula (I) may be prepared by use of known chemical reactions and procedures. The following general preparative methods are presented to aid one of skill in the art in synthesizing the inhibitors, with more detailed examples being presented in the experimental section describing the working examples.

Propenyl-pyrazol amine as defined in formula (III) is not commercially available. It can be prepared by several methods as described earlier (see, e.g., U.S. provisional application number 61/555,738).

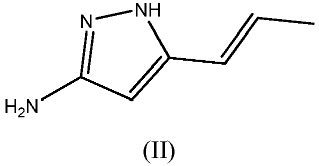

Precursors of substituted indol-5-ol as defined in formula (III) can be purchased from suppliers, or synthesized from commercially available precursors using established protocols. (WO 2004/009542, P33-38; Journal of Medicinal Chemistry, 2006, Vol 49, No. 7, P2143-2146; Org. Lett. Vol 10, No 12, 2008, P 2369-2372; WO 00/47212, P245-250; WO 2009036055 A1, P57).

Especially, precursor 47-difluoroindol-5-ol as defined in formula (IIIa) was not reported before and can be prepared by several methods as described earlier (WO2014145403 A1).

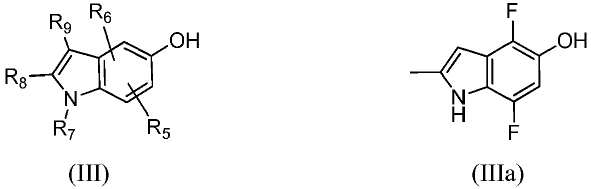

Precursors of 5-substituted 4, 6-dichloro-pyrimidines as defined in formula (IV) can be purchased from suppliers. Especially, precursor as defined in formula (IVa) can be synthesized from commercially available precursors using established protocols (PCT Int. Appl., 2010141406, 09 Dec 2010, Compound 310F).

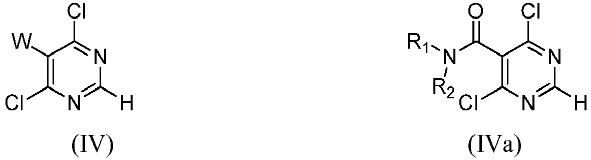

Generally, precursors of ArNH2 can be purchased from suppliers. Precursors of ArNH2 as defined in formula (V) can be purchased from suppliers, or synthesized from commercially available precursors using established protocols. (J. Med. Chem. 2010, 53, 7938-7957, specifically, P7949).

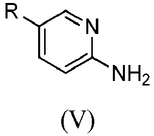

The preparation of the compounds of formula (I) in this invention can be carried out by methods listed in scheme 1.

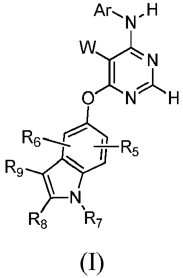

As shown in scheme 1, the pyrimidine derivative (I) can be synthesized by the reaction of 5-substituted 4,6-dichloropyrimidine with a sequence of substituted idole-5-ol to give monochlororopyrimidine intermediate of compound b, which can react with ArNH2 to produce the final compound (I). The reaction can be stepwise or in one pot. Alternative sequence can also be used to make pyrimidine derivatives. 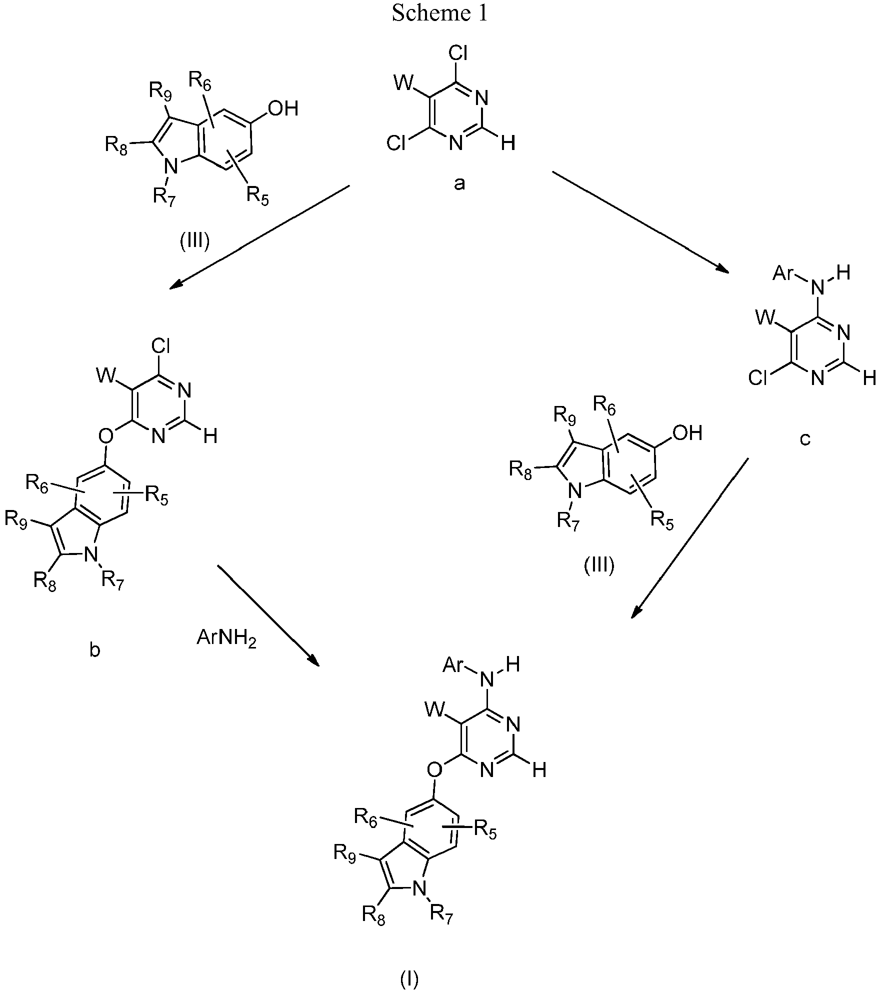 As shown in scheme 2, the final compounds as defined in (I-b), can be synthesized from the corresponding precursors, where W is "CN".

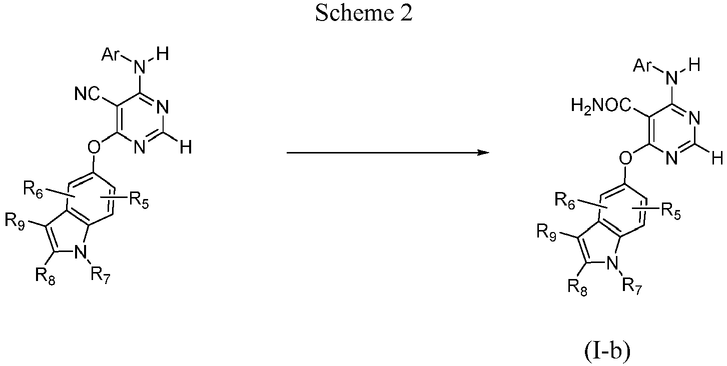

The reaction is preferably conducted in the presence of an inert solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane, heptane, ligroin and petroleum ether; aromatic hydrocarbons, such as benzene, toluene and xylene; halogenated hydrocarbons, especially aromatic and aliphatic hydrocarbons, such as methylene chloride, chloroform, carbon tetrachloride, dichloroethane, chlorobenzene and the dichlorobenzenes; esters, such as ethyl formate, ethyl acetate, propyl acetate, butyl acetate and diethyl carbonate; ethers, such as diethyl ether, diisopropyl ether, tetrahydrofuran, dioxane. dimethoxyethane and diethylene glycol dimethyl ether; ketones, such as acetone, methyl ethyl ketone, methyl isobutyl ketone, isophorone and cyclohexanone; nitro compounds, which may be nitroalkanes or nitroaranes, such as nitroethane and nitrobenzene; nitriles, such as acetonitrile and isobutyronitrile; amides, which may be fatty acid amides, such as formamide, dimethylformamide, dimethylacetamide and hexamethylphosphoric triamide; and sulphoxides, such as dimethyl sulphoxide and sulpholane.

The reaction can take place over a wide range of temperatures, and the precise reaction temperature is not critical to the invention. In general, we find it convenient to carry out the reaction at a temperature of from -50°C to 100°C.

The present invention provides compositions of matter that are formulations of one or more active drugs and a pharmaceutically-acceptable carrier. In this regard, the invention provides a composition for administration to a mammalian subject, which may include a compound of formula I, or its pharmaceutically acceptable salts.

Pharmaceutically acceptable salts of the compounds of this invention include those derived from pharmaceutically acceptable inorganic and organic acids and bases. Examples of suitable acid salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, formate, fumarate, glucoheptanoate, glycerophosphate, glycolate, hemisulfate, heptanoate, hexanoate, hydrochloride, hydrobromide, hydroiodide, 2-hydroxyethanesulfonate, lactate, maleate, malonate, methanesulfonate, 2-naphthalenesulfonate, nicotinate, nitrate, oxalate, palmoate, pectinate, persulfate, 3-phenylpropionate, phosphate, picrate, pivalate, propionate, salicylate, succinate, sulfate, tartrate, thiocyanate, tosylate and undecanoate. Other acids, such as oxalic, while not in themselves pharmaceutically acceptable, may be employed in the preparation of salts useful as intermediates in obtaining the compounds of the invention and their pharmaceutically acceptable acid addition salts.

Salts derived from appropriate bases include alkali metal (e.g., sodium and potassium), alkaline earth metal (e.g., magnesium), ammonium and N+(C1-4 alkyl)4 salts. This invention also envisions the quaternization of any basic nitrogen-containing groups of the compounds disclosed herein. Water or oil-soluble or dispersible products may be obtained by such quaternization.

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously.

The pharmaceutically acceptable compositions of this invention may be orally administered in any orally acceptable dosage form including, but not limited to, capsules, tablets, troches, elixirs, suspensions, syrups, wafers, chewing gums, aqueous suspensions or solutions.

The oral compositions may contain additional ingredients such as: a binder such as microcrystalline cellulose, gum tragacanth or gelatin; an excipient such as starch or lactose, a disintegrating agent such as alginic acid, corn starch and the like; a lubricant such as magnesium stearate; a glidant such as colloidal silicon dioxide; and a sweetening agent such as sucrose or saccharin or flavoring agent such as peppermint, methyl salicylate, or orange flavoring. When the dosage unit form is a capsule, it may additionally contain a liquid carrier such as a fatty oil. Other dosage unit forms may contain other various materials which modify the physical form of the dosage unit, such as, for example, a coating. Thus, tablets or pills may be coated with sugar, shellac, or other enteric coating agents. A syrup may contain, in addition to the active ingredients, sucrose as a sweetening agent and certain preservatives, dyes and colorings and flavors. Materials used in preparing these various compositions should be pharmaceutically or veterinarally pure and non-toxic in the amounts used.

For the purposes of parenteral therapeutic administration, the active ingredient may be incorporated into a solution or suspension. The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic.

The pharmaceutical forms suitable for injectable use include sterile solutions, dispersions, emulsions, and sterile powders. The final form should be stable under conditions of manufacture and storage. Furthermore, the final pharmaceutical form should be protected against contamination and should, therefore, be able to inhibit the growth of microorganisms such as bacteria or fungi. A single intravenous or intraperitoneal dose can be administered. Alternatively, a slow long-term infusion or multiple short-term daily infusions may be utilized, typically lasting from 1 to 8 days. Alternate day dosing or dosing once every several days may also be utilized.

Sterile, injectable solutions may be prepared by incorporating a compound in the required amount into one or more appropriate solvents to which other ingredients, listed above or known to those skilled in the art, may be added as required. Sterile injectable solutions may be prepared by incorporating the compound in the required amount in the appropriate solvent with various other ingredients as required. Sterilizing procedures, such as filtration, may then follow. Typically, dispersions are made by incorporating the compound into a sterile vehicle which also contains the dispersion medium and the required other ingredients as indicated above. In the case of a sterile powder, the preferred methods include vacuum drying or freeze drying to which any required ingredients are added.

Suitable pharmaceutical carriers include sterile water; saline, dextrose; dextrose in water or saline; condensation products of castor oil and ethylene oxide combining about 30 to about 35 moles of ethylene oxide per mole of castor oil; liquid acid; lower alkanols; oils such as corn oil; peanut oil, sesame oil and the like, with emulsifiers such as mono- or di-glyceride of a fatty acid, or a phosphatide, e.g., lecithin, and the like; glycols; polyalkylene glycols; aqueous media in the presence of a suspending agent, for example, sodium carboxymethylcellulose; sodium alginate; poly(vinylpyrolidone) ; and the like, alone, or with suitable dispensing agents such as lecithin; polyoxyethylene stearate; and the like. The carrier may also contain adjuvants such as preserving stabilizing, wetting, emulsifying agents and the like together with the penetration enhancer. In all cases, the final form, as noted, must be sterile and should also be able to pass readily through an injection device such as a hollow needle. The proper viscosity may be achieved and maintained by the proper choice of solvents or excipients. Moreover, the use of molecular or particulate coatings such as lecithin, the proper selection of particle size in dispersions, or the use of materials with surfactant properties may be utilized.

In accordance with the invention, there are provided compositions containing pyrimidine derivatives and methods useful for the in vivo delivery of pyrimidine derivatives in the form of nanoparticles, which are suitable for any of the aforesaid routes of administration.

United States Patent Nos. 5,916,596, 6,506,405 and 6,537,579 teach the preparation of nanoparticles from the biocompatible polymers, such as albumin. Thus, in accordance with the present invention, there are provided methods for the formation of nanoparticles of the present invention by a solvent evaporation technique from an oil-in-water emulsion prepared under conditions of high shear forces (e.g., sonication, high pressure homogenization, or the like).

Alternatively, the pharmaceutically acceptable compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

The pharmaceutically acceptable compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the pharmaceutically acceptable compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutically acceptable compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

The pharmaceutically acceptable compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, the pharmaceutically acceptable compositions of this invention are formulated for oral administration.

In accordance with the invention, the compounds of the invention may be used to treat diseases associated with cellular proliferation or hyperproliferation, such as cancers which include but are not limited to tumors of the nasal cavity, paranasal sinuses, nasopharynx, oral cavity, oropharynx, larynx, hypopharynx, salivary glands, and paragangliomas. The compounds of the invention may also be used to treat cancers of the liver and biliary tree (particularly hepatocellular carcinoma), intestinal cancers, particularly colorectal cancer, ovarian cancer, small cell and non-small cell lung cancer, breast cancer, sarcomas (including fibrosarcoma, malignant fibrous histiocytoma, embryonal rhabdomysocarcoma, leiomysosarcoma, neuro-fibrosarcoma, osteosarcoma, synovial sarcoma, liposarcoma, and alveolar soft part sarcoma), neoplasms of the central nervous systems (particularly brain cancer), and lymphomas (including Hodgkin's lymphoma, lymphoplasmacytoid lymphoma, follicular lymphoma, mucosa-associated lymphoid tissue lymphoma, mantle cell lymphoma, B-lineage large cell lymphoma, Burkitt's lymphoma, and T-cell anaplastic large cell lymphoma).

The compounds and methods of the present disclosure, either when administered alone or in combination with other agents (e.g., chemotherapeutic agents or protein therapeutic agents described below) are also useful in treating a variety of disorders, including but not limited to, for example: stroke, cardiovascular disease, myocardial infarction, congestive heart failure, cardiomyopathy, myocarditis, ischemic heart disease, coronary artery disease, cardiogenic shock, vascular shock, pulmonary hypertension, pulmonary edema (including cardiogenic pulmonary edema), pleural effusions, rheumatoid arthritis, diabetic retinopathy, retinitis pigmentosa, and retinopathies, including diabetic retinopathy and retinopathy of prematurity, inflammatory diseases, restenosis, asthma, acute or adult respiratory distress syndrome (ARDS), lupus, vascular leakage, protection from ischemic or reperfusion injury such as ischemic or reperfusion injury incurred during organ transplantation, transplantation tolerance induction; ischemic or reperfusion injury following angioplasty; arthritis (such as rheumatoid arthritis, psoriatic arthritis or osteoarthritis); multiple sclerosis; inflammatory bowel disease, including ulcerative colitis and Crohn's disease; lupus (systemic lupus crythematosis); graft vs. host diseases; T- cell mediated hypersensitivity diseases, including contact hypersensitivity, delayed- type hypersensitivity, and gluten-sensitive enteropathy (Celiac disease); Type 1 diabetes; psoriasis; contact dermatitis (including that due to poison ivy); Hashimoto's thyroiditis; Sjogren's syndrome; Autoimmune Hyperthyroidism, such as Graves' disease; Addison's disease (autoimmune disease of the adrenal glands); autoimmune polyglandular disease (also known as autoimmune polyglandular syndrome); autoimmune alopecia; pernicious anemia; vitiligo; autoimmune hypopituatarism; Guillain-Barre syndrome; other autoimmune diseases; cancers, including those where kineses such as Src-family kineses are activated or overexpressed, such as colon carcinoma and thymoma, or cancers where kinase activity facilitates tumor growth or survival; glomerulonephritis, serum sickness; uticaria; allergic diseases such as respiratory allergies (asthma, hayfever, allergic rhinitis) or skin allergies; mycosis fungoides; acute inflammatory responses (such as acute or adult respiratory distress syndrome and ischemialreperfusion injury); dermatomyositis; alopecia areata; chronic actinic dermatitis; eczema; Behcet's disease; Pustulosis palmoplanteris; Pyoderma gangrenum; Sezary's syndrome; atopic dermatitis; systemic schlerosis; morphea; peripheral limb ischemia and ischemic limb disease; bone disease such as osteoporosis, osteomalacia, hyperparathyroidism, Paget's disease, and renal osteodystrophy; vascular leak syndromes, including vascular leak syndromes induced by chemotherapies or immunomodulators such as IL-2; spinal cord and brain injury or trauma; glaucoma; retinal diseases, including macular degeneration; vitreoretinal disease; pancreatitis; vasculatides, including vasculitis, Kawasaki disease, thromboangiitis obliterans, Wegener s granulomatosis, and Behcet's disease; scleroderma; preeclampsia; thalassemia; Kaposi's sarcoma; von Hippel Lindau disease; and the like.

In accordance with the invention, the compounds of the invention may be used to treat diseases associated with undesired cellular proliferation or hyperproliferation comprising identifying the animal afflicted with said disease or condition and administering to said afflicted animal a composition comprising the compound of formula (I), wherein the disease or condition is associated with a kinase.

The invention also provides methods of treating an animal afflicted with the above diseases and conditions. The amount of the compounds of the present invention that may be combined with the carrier materials to produce a composition in a single dosage form will vary depending upon the host treated, the particular mode of administration. Preferably, the compositions should be formulated so that a dosage of between 0.01-100 mg/kg body weight/day of the inhibitor can be administered to a patient receiving these compositions.

In one aspect, the invention compounds are administered in combination with chemotherapeutic agent, an anti-inflammatory agent, antihistamines, chemotherapeutic agent, immunomodulator, therapeutic antibody or a protein kinase inhibitor, e.g., a tyrosine kinase inhibitor, to a subject in need of such treatment.

The method includes administering one or more of the inventive compounds to the afflicted mammal. The method may further include the administration of a second active agent, such as a cytotoxic agent, including alkylating agents, tumor necrosis factors, intercalators, microtubulin inhibitors, and topoisomerase inhibitors. The second active agent may be co-administered in the same composition or in a second composition. Examples of suitable second active agents include, but are not limited to, a cytotoxic drug such as Acivicin; Aclarubicin; Acodazole Hydrochloride; AcrQnine; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorambucil; Cirolemycin; Cisplatin; Cladribine; Crisnatol Mesylate; Cyclophosphamide; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflomithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Ethiodized Oil 131; Etoposide; Etoposide Phosphate; Etoprine; Fadrozole Hydrochloride; Fazarabine; Fenretinide; Floxuridine; Fludarabine Phosphate; Fluorouracil; Flurocitabine; Fosquidone; Fostriecin Sodium; Gemcitabine; Gemcitabine Hydrochloride; Gold Au 198; Hydroxyurea; Idarubicin Hydrochloride; Ifosfamide; Ilmofosine; Interferon Alfa-2a; Interferon Alfa-2b; Interferon Alfa-nl; Interferon Alfa-n3; Interferon Beta- □ a; Interferon Gamma- Ib; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Safmgol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Strontium Chloride Sr 89; Sulofenur; Talisomycin; Taxane; Taxoid; Tecogalan Sodium; Tegafur; Teloxantrone Hydrochloride; Temoporfin; Teniposide; Teroxirone; Testolactone; Thiamiprine; Thioguanine; Thiotepa; Tiazofurin; Tirapazamine; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; and Zorubicin Hydrochloride.

In accordance with the invention, the compounds and compositions may be used at sub-cytotoxic levels in combination with other agents in order to achieve highly selective activity in the treatment of non-neoplastic disorders, such as heart disease, stroke and neurodegenerative diseases (Whitesell et al., Curr Cancer Drug Targets (2003), 3(5), 349-58).

The exemplary therapeutical agents that may be administered in combination with invention compounds include EGFR inhibitors, such as gefitinib, erlotinib, and cetuximab. Her2 inhibitors include canertinib, EKB-569, and GW-572016. Also included are Src inhibitors, dasatinib, as well as Casodex (bicalutamide), Tamoxifen, MEK-1 kinase inhibitors, MARK kinase inhibitors, PI3 inhibitors, and PDGF inhibitors, such as imatinib, Hsp90 inhibitors, such as 17-AAG and 17-DMAG. Also included are anti-angiogenic and antivascular agents which, by interrupting blood flow to solid tumors, render cancer cells quiescent by depriving them of nutrition. Castration, which also renders androgen dependent carcinomas non-proliferative, may also be utilized. Also included are IGF1R inhibitors, inhibitors of non- receptor and receptor tyrosine kineses, and inhibitors of integrin.

The pharmaceutical composition and method of the present invention may further combine other protein therapeutic agents such as cytokines, immunomodulatory agents and antibodies. As used herein the term "cytokine" encompasses chemokines, interleukins, lymphokines, monokines, colony stimulating factors, and receptor associated proteins, and functional fragments thereof. As used herein, the term "functional fragment" refers to a polypeptide or peptide which possesses biological function or activity that is identified through a defined functional assay. The cytokines include endothelial monocyte activating polypeptide II (EMAP- II), granulocyte-macrophage-CSF (GM-CSF), granulocyte-CSF (G- CSF), macrophage- CSF (M-CSF), IL-1, IL-2, IL-3, IL- 4, IL-5, IL-6, IL-12, and IL-13, interferons, and the like and which is associated with a particular biologic, morphologic, or phenotypic alteration in a cell or cell mechanism.

Other therapeutic agents for the combinatory therapy include cyclosporins (e.g., cyclosporin A), CTLA4-Ig, antibodies such as ICAM-3, anti-IL-2 receptor (Anti-Tac), anti-CD45RB, anti-CD2, anti-CD3 (OKT-3), anti-CD4, anti-CD80, anti-CD86, agents blocking the interaction between CD40 and gp39, such as antibodies specific for CD40 and for gpn39 (i.e., CD154), fusion proteins constructed from CD40 and gp39 (CD40Ig and CD8gp39), inhibitors, such as nuclear translocation inhibitors, of NF-kappa B function, such as deoxyspergualin (DSG), cholesterol biosynthesis inhibitors such as HM:G CoA reductase inhibitors (lovastatin and simvastatin), non-steroidal antiinflammatory drugs (NSAIDs) such as ibuprofen and cyclooxygenase inhibitors such as rofecoxib, steroids such as prednisone or dexamethasone, gold compounds, antiproliferative agents such as methotrexate, FK506 (tacrolimus, Prograf), mycophenolate mofetil, cytotoxic drugs such as azathioprine and cyclophosphamide, TNF-a inhibitors such as tenidap, anti-TNF antibodies or soluble TNF receptor, and rapamycin (sirolimus or Rapamune) or derivatives thereof.

When other therapeutic agents are employed in combination with the compounds of the present invention they may be used for example in amounts as noted in the Physician Desk Reference (PDR) or as otherwise determined by one having ordinary skill in the art.

The following demonstrates the chemical structure and synthesis of 2 "intermediates" useful in the synthesis of 61 "compounds." The 61 compounds will be tested for their ability to inhibit a range of kinases.

All synthesis were performed under anhydrous conditions (i.e. dry solvents) in an atmosphere of argon, except where stated, using oven-dried apparatus and employing standard techniques in handling air-sensitive materials. Aqueous solutions of sodium bicarbonate (NaHCO3) and sodium chloride (brine) were saturated.
Analytical thin layer chromatography (TLC) was carried out on Merck Kiesel gel 60 F254 plates with visualization by ultraviolet and/or anisaldehyde, potassium permanganate or phosphomolybdic acid dips.
NMR spectra: 1H Nuclear magnetic resonance spectra were recorded at 400 MHz. Data are presented as follows: chemical shift, multiplicity (s = singlet, d = doublet, t = triplet, q = quartet, qn = quintet, dd = doublet of doublets, m = multiplet, bs = broad singlet), coupling constant (J/Hz) and integration. Coupling constants were taken and calculated directly from the spectra and are uncorrected.
Low resolution mass spectra: Electrospray (ES+) ionization was used. The protonated parent ion (M+H) or parent sodium ion (M+Na) or fragment of highest mass is quoted. Analytical gradient consisted of 10% ACN in water ramping up to 100% ACN over 5 minutes unless otherwise stated.

High performance liquid chromatography (HPLC) was use to analyze the purity of derivatives. HPLC was performed on a Phenomenex Synergi Polar-RP, 4u, 80A, 150 x 4.6 mm column using a Shimadzu system equipted with SPD-M10A Phosphodiode Array Detector. Mobile phase A was water and mobile phase B was acetonitrile with a gradient from 20% to 80% B over 60 minutes and re-equilibrate at A/B (80:20) for 10 minutes. UV detection was at 220 and 54 nm.
The following demonstrates the chemical structure and synthesis of two intermediate compounds ("intermediate 1" and "intermediate 2") known by those of ordinary skill in art to have been useful in synthesizing pyrimidine derivatives which can have kinase inhibitory activity.

### Intermediate 1

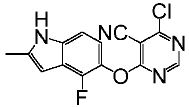

To a solution of 4-fluoro-2-methyl-1H-indol-5-ol (200 mg, 1.21 mmol) in a mixture of acetonitrile (4 mL) and N, N-dimethylformamide (1 mL) was added potassium carbonate (200 mg, 1.45 mmol). The reaction mixture was stirred for 1h at room temperature before a suspension of 4, 6-dichloropyrimidine-5-carbonitrile (221 mg, 1.27 mmol) in 3 mL of acetonitrile was added. This mixture was stirred at room temperature for 1 h. TLC was checked and the reaction was completed. The mixture was diluted with water and ethyl acetate. The layers were separated and the aqueous phase was extracted twice with ethyl acetate. The combined organic phases were washed once with water, then with brine, dried over sodium sulfate, filtered, and the filtrate was concentrated in vacuo to give the desired product as brown solids (365 mg, 99% yield). ¹H NMR (400 MHz, DMSO-d₆) δ 11.46 (br, 1H), 8.83 (s, 1H), 7.17 (d, J = 8.8 Hz, 1H), 7.00 (t, J = 7.6 Hz, 1H), 6.27 (s, 1H), 2.41 (s, 3H); ESI-MS: calcd for (C14H8ClFN4O) 302, found 303 (MH⁺).

### Intermediate 2

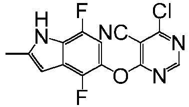

To a solution of 4,7-difluoro-2-methyl-1H-indol-5-ol (500 mg, 2.73 mmol) in a mixture of acetonitrile (9 mL) and N, N-dimethylformamide (1 mL) was added potassium carbonate (453 mg, 3.28 mmol). The reaction mixture was stirred for 30 min. at room temperature before a suspension of 2, 4-dichloro5-cyanopyrimidine (499 mg, 2.87 mmol) in acetonitrile/DMF (2.5 mL/2.5 mL) was added. This mixture was stirred at 0 °C for 2 h. TLC was checked and the reaction was completed. The mixture was diluted with water/brine and ethyl acetate. The layers were separated and the aqueous phase was extracted twice with ethyl acetate. The combined organic phases were washed once with water/brine three times, dried over sodium sulfate, filtered, and the filtrate was concentrated in vacuo to give the deired compound as purple sticky solids (890 mg, 100% yield, contained some DMF). The product was used without further purification. ¹H NMR (400 MHz, DMSO-d₆) δ 11.91 (br, 1H), 8.84 (s, 1H), 7.03 (dd, J = 5.6 Hz, J=10.4Hz, 1H), 6.35 (br, 1H), 2.39 (s, 3H); ESI-MS: calcd for (C14H7ClF2N4O) 320, found 321 (MH⁺).

The following demonstrate the chemical structure and a method of synthesizing 61 pyrimidine derivatives ("compound 1" to "compound 61") which are species the generic compound disclosed herein.

### Reference Compound 1

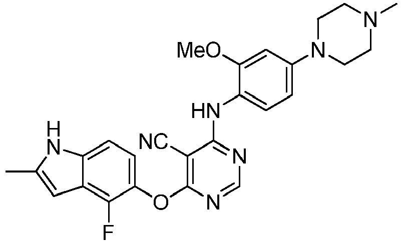

A mixture of Intermediate 1 (100 mg, 0.33 mmol), 2-methoxy-4-(4-methylpiperazin-1-yl)aniline (73 mg, 0.33 mmol),, and DIPEA (0.08 ml, 0.49 mmol) in DMSO (5 ml) was stirred at room temperature for 30 min. After checking the TLC, the mixture was added to water (100ml). After cooled with ice-bath, the solids were collected by filtration, washed by water. The crude product was purified by column chromatography (silica gel, 0-15% MeOH in DCM) to give the desired product as yellow solids (64 mg, 40% yield). ¹H NMR (400 MHz, DMSO-d₆) δ 11.34 (br, 1H), 9.45 (br s, 1H), 8.15 (s, 1H), 7.13 (m, 2H), 6.93 (m, 1H), 6.65 (m, 1H), 6.50 (m, 1H), 6.23 (s, 1H), 3.77 (s, 3H), 3.18 (m, 4H), 2.46 (m, 4H), 2.39 (s, 3H), 2.23 (s, 3H); ESI-MS: calcd for C26H26FN7O2 487, found 488 (MH⁺). HPLC: retention time: 18.63 min. purity: 96%.

### Reference Compound 2

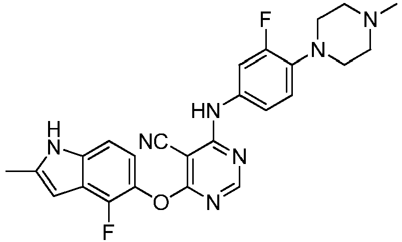

A mixture Intermediate 1 (100 mg, 0.33 mmol), 3-fluoro-4-(4-methylpiperazin-1-yl)aniline (69 mg, 0.33 mmol),, and DIPEA (0.15 ml, 0.82 mmol) in DMSO (5 ml) was stirred at room temperature for 30 min. After checking the TLC, the mixture was added to water (100ml). After cooled with ice-bath, the solids were collected by filtration, washed by water. The crude product was purified by column chromatography (silica gel, 0-15% MeOH in DCM) to give the desired product as light brown solids (90 mg, 57% yield). ¹H NMR (400 MHz, DMSO-d₆) δ 11.36 (br, 1H), 10.0 (s, 1H), 8.30 (s, 1H), 7.41 (m, 1H), 7.26 (m, 1H), 7.13 (m, 1H), 6.98 (m, 2H), 6.24 (br s, 1H), 3.00 (m, 4H), 2.50 (m, 4H), 2.40 (s, 3H), 2.23 (s, 3H); ESI-MS: calcd for C25H23F2N7O 475, found 476 (MH⁺). HPLC: retention time: 19.15 min. purity: 95%.

### Reference Compound 3

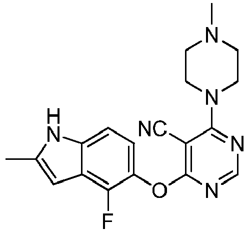

A mixture Intermediate 1 (100 mg, 0.33 mmol), 4-((4-methylpiperazin-1-yl)methyl)aniline (68 mg, 0.33 mmol), and DIPEA (0.15 ml, 0.82 mmol) in DMSO (5 ml) was stirred at room temperature for 30 min. After checking the TLC, the mixture was added to water (100ml). After cooled with ice-bath, the solids were collected by filtration, washed by water. The crude product was purified by column chromatography (silica gel, 0-15% MeOH in DCM) to give the desired product as off white solids (41 mg, 26% yield) ¹H NMR (400 MHz, DMSO-d₆) δ 11.34 (br s, 1H), 8.24 (m, 1H), 7.12 (m, 1H), 6.92 (m, 1H), 6.22 (s, 1H), 3.95 (m, 4H), 2.41s (m, 4H), 2.39 (s, 3H), 2.24 (s, 3H); ESI-MS: calcd for C19H19FN6O 366, found 367 (MH⁺). HPLC: retention time: 12.36 min. purity: 98%.

### Reference Compound 4

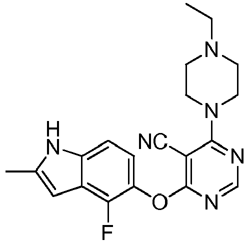

A mixture Intermediate 1 (100 mg, 0.33 mmol), 4-((4-ethylpiperazin-1-yl)methyl)aniline (72 mg, 0.33 mmol), and DIPEA (0.15 ml, 0.82 mmol) in DMSO (5 ml) was stirred at room temperature for 30 min. After checking the TLC, the mixture was added to water (100ml). After cooled with ice-bath, the solids were collected by filtration, washed by water. The crude product was purified by column chromatography (silica gel, 0-15% MeOH in DCM) to give the desired product as off white solids (18 mg, 11% yield) ¹H NMR (400 MHz, DMSO-d₆) δ 11.34 (br s, 1H), 8.24 (s, 1H), 7.12 (m, 1H), 6.92 (m, 1H), 6.23 (s, 1H), 3.96 (m, 4H), 2.52 (m, 3H), 2.45 (m, 4H), 1.23 (m, 2H), 1.050 (m, 3H); ESI-MS: calcd for C20H21FN6O 380, found 381 (MH⁺). HPLC: retention time: 13.77 min. purity: 93%.

### Reference Compound 5

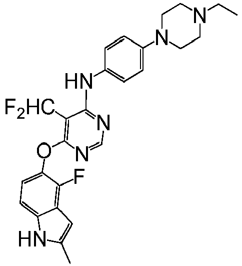

Step 1: To a solution of flouroindole (415 mg, 2.51 mmol) and 4,6-dichloro-5-(difluoromethyl)pyridine (500 mg, 2.51 mmol) in DMSO (3ml) was added potassium carbonate (695 mg, 5.03 mmol) at room temperature and the mixture was heated at room temperature for oernight. TLC was checked and the starting material was consumed. The reaction mixture was added to a flask with water/brine (50ml/50 ml) and the mixture was stirred at room temperature for 1 hour then colled with ice bath. The solids were collected by filtration and washed by water to obtain the desired product as yellow solids (803 mg, 98% yield). No further purification was performed and the product was used for the next step reaction. ¹H NMR(400 MHz, DMSO-d₆) δ 11.39 (br, 1H), 8.71 (s, 1H), 7.51 (t, J=52.4 Hz, 1H), 7.15 (d, J = 8.8 Hz, 1H), 6.96 (t, J = 7.6 Hz, 1H), 6.25 (s, 1H), 2.40 (s, 3H); ESI-MS: calcd for (C14H9ClF3N3O) 327, found 328 (MH⁺).

**Step 2:** A mixture of above intermediate (125 mg, 0.61 mmol), 4-(4-Ethylpiperazine-1-yl))aniline (200 mg, 0.61 mmol), and DIPEA (0.27 ml, 1.52 mmol) in DMSO (3.0 ml) was stiired at 100 °C for 2 h, then at room temperature forovernight. TLC was checked and the reaction was completed. The mixture was added to water/sat. NH₄Cl (50 ml/50ml) and stirred at room temperature for 30 min. The pH odf the mixture was adjusted to ∼ 6 using 2N HCl. Cooled at 4 °C and the solids were collected by filtration, washed by water to give the sticky fine crude product. The crude product was dissolved into DCM/MeOH(2ml/2ml), dried over sodium sulfate and concentrated. The crude product was purified on column (0-10% MeOH in DCM) to give the desired product as yellow solids (103 mg, 34% yield). ¹H NMR (400 MHz, DMSO-d₆) δ 11.32 (br, 1H), 10.49 (br, 1H), 8.93 (s, 1H), 8.12 (s, 1H), 7.36 (d, J = 8.8 Hz, 2H), 7.10 (d, J = 8.8 Hz,1H), 7.00 (d, J = 9.2 Hz, 2H), 6.86 (t, J = 7.6 Hz, 1H), 6.21 (s, 1H), 3.80 (br, 2H), 3.55 (br, 2H), 3.10 (m, 6H), 2.39 (s, 3H), 1.28 (t, J=7.2Hz, 3H); ESI-MS: calcd for (C26H27F3N6O) 496, found 497 (MH⁺).

### Reference Compound 6

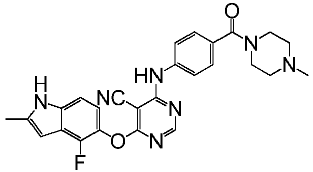

A flask was charged with intermiediate 1 (150mg, 0.5 mmol), 1-(4-aminobenzoyl)-4-methyl piperazine (109 mg, 0.5 mmol), TFA (50uL), isopropanol (3mL). The reaction was heated to 100°C for 4h. The reaction mixture was basified with a saturated aqueous sodium bicarbonate solution and then was extracted with DCM/ (10 mlx3). The combined organic was washed by brine, dried over sodium sulfate and concentrated. The crude product was purified with flash chromatography (0-10% MeOH-in DCM) to afford the desired product as light yellow solids (160 mg, 66% yield). ¹H NMR (400 MHz, DMSO-d₆) δ 11.36 (br, 1H), 10.17 (br, 1H), 8.34 (s, 1H), 7.63 (d, J = 8.8 Hz, 2H), 7.39 (d, J = 8.8 Hz, 2H), 7.13 (d, J = 8.4 Hz,1H), 6.96 (t, J = 7.6 Hz, 1H), 6.24 (s, 1H), 3.80-3.40 (br, 4H), 2.40 (s, 3H), 2.36-2.24 (br, 4H), 2.19 (s, 3H); ESI-MS: calcd for (C26H24FN7O2) 485, found 486 (MH⁺).

### Reference Compound 7

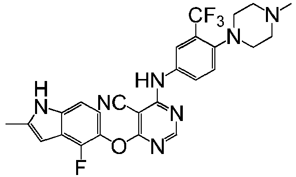

A flask was charged with intermiediate 1 (100mg, 0.33 mmol), 4-(4-methylpiperazin-1-yl)-3-(trifluoromethyl)aniline (86 mg, 0.33 mmol), TFA (50uL), isopropanol (3mL). The reaction was heated to 100°C for 4h. The reaction mixture was basified with a saturated aqueous sodium bicarbonate solution and then was extracted with DCM/ (10mlx3). The combined organic was washed by brine, dried over sodium sulfate and concentrated. The crude product was purified with flash chromatography (0-10% MeOH-in DCM) to afford the desired product as light yellow solids (103 mg, 60% yield). ¹H NMR (400 MHz, DMSO-d₆) δ 11.36 (br, 1H), 10.18 (br, 1H), 8.33 (s, 1H), 7.90-7.80 (m, 2H), 7.55 (d, J = 8.8 Hz, 1H), 7.13 (d, J = 8.8 Hz,1H), 6.95 (t, J = 7.2 Hz, 1H), 6.24 (s, 1H), 2.85 (m, 4H), 2.49-2.32 (m, 7H), 2.23 (s, 3H); ESI-MS: calcd for (C26H23F4N7O) 525, found 526 (MH⁺).

### Reference Compound 8

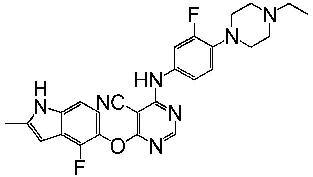

A flask was charged with intermiediate 1 (100mg, 0.33 mmol), 4-(4-eethylpiperazin-1-yl)-3-fluoroaniline (74 mg, 0.33 mmol), TFA (50uL), isopropanol (3mL). The reaction was heated to 100°C for 4h. The reaction mixture was basified with a saturated aqueous sodium bicarbonate solution and then was extracted with DCM/ (10mlx3). The combined organic was washed by brine, dried over sodium sulfate and concentrated. The crude product was purified with flash chromatography (0-10% MeOH-in DCM) to afford the desired product as light yellow solids (120 mg, 74% yield). ¹H NMR (400 MHz, DMSO-d₆) δ 11.36 (br, 1H), 9.99 (br, 1H), 8.29 (s, 1H), 7.39 (dd, J=2.0Hz, J=14.4Hz, 1H), 7.26 (d, J = 8.8 Hz, 1H), 7.13 (d, J = 8.4 Hz,1H), 7.01 (t, J=9.2Hz, 1H), 6.95 (t, J = 8.0 Hz, 1H), 6.24 (s, 1H), 3.00 (m, 4H), 2.60-2.45 (m, 4H), 2.40 (m, 5H), 1.02 (t, J=7.2Hz, 3H); ESI-MS: calcd for (C26H25F2N7O) 489, found 490 (MH⁺).

### Reference Compound 9

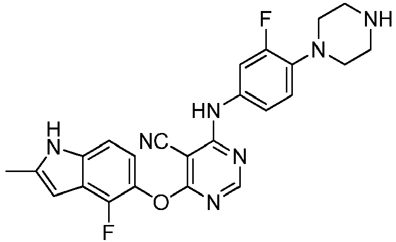

A mixture intermiediate 1 (100 mg, 0.33 mmol), tert-butyl 4-(4-amino-2-fluorophenyl)piperazine-1-carboxylate (97 mg, 0.33 mmol),, and DIPEA (0.15 ml, 0.82 mmol) in DMSO (5 ml) was stirred at room temperature for 30 min. After checking the TLC, the mixture was added to water (100ml). After cooled with ice-bath, the solids were collected by filtration, washed by water. After air-drying at room temperature overnight, the solids were suspended into DCM/MeOH (10/1, 5 mL) and 1 ml of TFA was added. The mixture was stirred at room temperature for overnight. After concentrated, the residue was dissolved into DCM/MeOH (8/2, 15 ml) and sat. Sodium bicarbonate solution was added to pH about 7. The organic was dried over sodium sulfate and concentrated. The crude product was purified by column chromatography (silica gel, 0-15% MeOH in DCM) to give the desired product as off white solids (60 mg, 39% yield). ¹H NMR (400 MHz, DMSO-d₆) δ 11.36 (br, 1H), 8.30 (s, 1H), 7.43 (m, 1H), 7.28 (m, 1H), 7.14 (m, 1H), 7.02 (m, 1H), 6.94 (m, 1H), 6.24 (s, 1H), 2.97 (m, 8H), 2.40 (m, 3H); ESI-MS: calcd for C24H21F2N7O 461, found 462(MH⁺). HPLC: retention time: NA

### Reference Compound 10

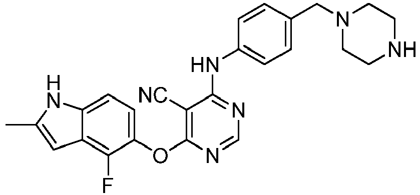

A mixture intermiediate 1 (100 mg, 0.33 mmol), tert-butyl 4-(4-aminobenzyl)piperazine-1-carboxylate (96 mg, 0.33 mmol),, and DIPEA (0.15 ml, 0.82 mmol) in DMSO (5 ml) was stirred at room temperature for 30 min. After checking the TLC, the mixture was added to water (100ml). After cooled with ice-bath, the solids were collected by filtration, washed by water. After air-drying at room temperature overnight, the solids were suspended into DCM/MeOH (10/1, 5 mL) and 1 ml of TFA was added. The mixture was stirred at room temperature for overnight. After concentrated, the residue was dissolved into DCM/MeOH (8/2, 15 ml) and sat. Sodium bicarbonate solution was added to pH about 7. The organic was dried over sodium sulfate and concentrated. The crude product was purified by column chromatography (silica gel, 0-15% MeOH in DCM) to give the desired product as light yellow solids (15 mg, 10% yield). ¹H NMR (400 MHz, DMSO-d₆) δ 11.48 (br, 1H), 10.06 (s, 1H), 8.90 (m, 2H), 8.28 (m, 1H), 7.48 (m, 2H), 7.29 (m, 2H), 7.13 (m, 1H), 6.94 (m, 1H), 6.23 (s, 1H), 3.53 (m, 2H), 3.09 (m, 4H), 2.54 (m, 4H), 2.40 (m, 3H); ESI-MS: calcd for C25H24FN7O 457, found 458 (MH⁺). HPLC: retention time: 13.75 min. purity: 91%.

### Reference Compound 11

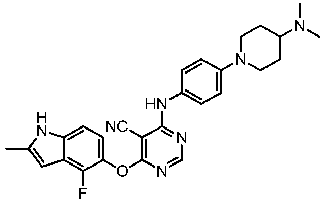

**Step 1:** A mixture of 1-fluoro-4-nitrobenzene (3.00 g, 21.3 mmol), N,N-dimethylpiperidin-4-amine dihydrochloride (4.70 g, 23.4 mmol) and DIPEA (15 mL, 86.1 mmol) was stirred at 95 °C for 18 h. The mixture was cooled to room temperature, diluted with 1:1 EtOAc/hexanes (100 mL), washed twice with aq. calcium gluconate (100 mL each, 50% saturation), and the organic layer was separated and dried over anhydrous Na₂SO₄ and concentrated to yield the desired product as a red oil (4.1 g, 77% yield). ¹H NMR (DMSO-d6, 400 MHz) δ 8.03 (d, J = 9.6 Hz, 1H), 7.01 (d, J = 9.6 Hz, 1H), 4.04-4.01 (m, 2H), 3.02-2.95 (m, 2H), 2.39-2.32 (m, 1H), 2.17 (s, 6H), 1.85-1.81 (m, 2H), 1.44-1.34 (m, 2H); MS (ESI): calcd for C13H19N3O2: 249, found: 250 (MH⁺).

**Step 2:** A mixture of N,N-dimethyl-1-(4-nitrophenyl)piperidin-4-amine (1.5 g, 6.0 mmol), tin (II) chloride dihydrate (6.8 g, 30 mmol) and methanol (100 mL) was stirred at 70 °C for 19 h. The mixture was cooled to room temperature, diluted with 4:1 EtOAc/hexanes (200 mL), washed with aq. 5M NaOH (200 mL), and the organic layer was separated and dried over anhydrous Na₂SO₄ and concentrated. The resulting residue was purified by flash column chromatography on silica gel using 0-20% MeOH in DCM (v/v) as eluent to afford the desired product 1-(4-aminophenyl)-N,N-dimethylpiperidin-4-amine as a light yellow solid (340 mg, 26% yield). ¹H NMR (DMSO-d6, 400 MHz) δ 6.67 (d, J = 8.8 Hz, 2H), 6.47 (d, J = 8.8 Hz, 2H), 4.53 (br s, 2H), 3.37-3.34 (m, 2H), 2.48-2.42 (m, 2H), 2.18 (s, 6H), 2.11-2.05 (m, 1H), 1.80-1.77 (m, 2H), 1.51-1.41 (m, 2H); MS (ESI): calcd for C13H21N3: 219, found: 220 (MH⁺).

Step 3: To a mixture of 4-chloro-6-((4-fluoro-2-methyl-1H-indol-5-yl)oxy)pyrimidine-5-carbonitrile (200 mg, 0.661 mmol) and 1-(4-aminophenyl)-N,N-dimethylpiperidin-4-amine (142 mg, 0.647 mmol) in anhydrous DMSO (2.0 mL) was added TEA (0.28 mL; 1.98 mmol), and the resulting biphasic mixture was efficiently stirred at room temperature under argon atmosphere for 19 h. The resulting mixture was diluted with 4:1 EtOAc/hexanes (100 mL) and washed with aq. NH₄Cl (ca. 100 mL each; 50% NH₄Cl saturation) followed by brine (100 mL; 50% saturation). The organic layer was separated and dried over anhydrous Na₂SO₄ and concentrated. The resulting residue was purified by crystallization out of EtOAc to afford the desired product as a yellow solid (254 mg, 81% yield). ¹H NMR (DMSO-d6, 400 MHz) δ 11.35 (br s, 1H), 9.83 (br s, 1H), 8.21 (s, 1H), 7.30-7.28 (m, 2H), 7.13-7.11 (m, 1H), 6.95-6.91 (m, 2H), 6.23 (s, 1H), 3.71-3.68 (m, 2H), 2.69-2.62 (m, 2H), 2.40 (s, 3H), 2.24-2.21 (m, 1H), 2.19 (s, 6H), 1.84-1.81 (m, 2H), 1.52-1.42 (m, 2H); MS (ESI): calcd for C27H28FN7O: 485, found: 486 (MH⁺).

### Reference Compound 12

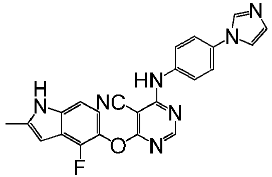

A mixture of intermiediate 1 (150 mg, 0.50 mmol), 4-imidazole-1-yl-phenylamine (91 mg, 0.57mmol), and DIPEA (0.22 ml, 1.245 mmol) in DMSO (3.0 ml) was stiired at roomtemperaure for overnight. TLC was checked and the reaction was completed. The mixture was added to sat. NH₄Cl/water (25ml/50ml ml) and stirred at room temperature for 30 min. The pH of the mixture was adjusted to about 6 using 2N HCl. After coled with ice for 1h, the solids were collected by filtration, washed by water to give the crude product. The crude product was purified by column on slica gel (0-10% MeOH in DCM) to give the desired product as yellow solids (140 mg, 66% yield).¹H NMR (400 MHz, DMSO-d₆) δ 11.36 (br, 1H), 10.16 (s, 1H), 8.31 (s, 1H), 8.24 (s, 1H), 7.66 (m, 5H), 7.14 (m, 2H), 6.95 (t, J = 7.6 Hz, 1H), 6.24 (s, 1H), 2.40 (s, 3H); ESI-MS: calcd for (C23H16FN7O) 425, found 426 (MH⁺).

### Reference Compound 13

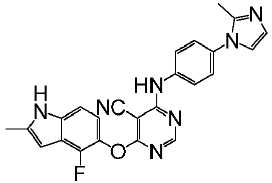

A mixture of intermiediate 1 (150 mg, 0.50 mmol), 4-(2-methylimidazole-1-yl-phenylamine (99 mg, 0.57mmol), and DIPEA (0.22 ml, 1.245 mmol) in DMSO (3.0 ml) was stiired at roomtemperaure for overnight. TLC was checked and the reaction was completed. The mixture was added to sat. NH₄Cl/water (25ml/50ml ml) and stirred at room temperature for 30 min. The pH of the mixture was adjusted to about 6 using 2N HCl. After coled with ice for 1h, the solids were collected by filtration, washed by water to give the crude product. The crude product was purified by column on slica gel (0-10% MeOH in DCM) to give the desired product as yellow solids (108 mg, 50% yield).¹H NMR (400 MHz, DMSO-d₆) δ 11.36 (br, 1H), 10.16 (br, 1H), 8.33 (s, 1H), 7.70 (d, J=8.8Hz, 2H), 7.42 (d, J=8.8Hz, 2H), 7.28 (br, 1H), 7.14 (d, J=8.4Hz, 1H), 6.96 (t, J = 7.6 Hz, 1H), 6.25 (s, 1H), 2.41 (s, 3H), 2.30 (s, 3H); ESI-MS: calcd for (C24H18FN7O) 439, found 440 (MH⁺).

### Reference Compound 14

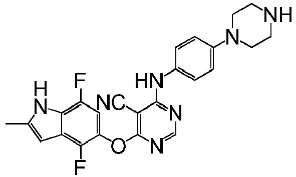

A mixture of intermiediate 2 (150 mg, 0.47 mmol), tert-butyl 4-(4-aminophenyl)piperazine-1-carboxylate (149mg, 0.54mmol), and DIPEA (0.21 ml, 1.17 mmol) in DMSO (3.0 ml) was stiired at roomtemperaure for overnight. TLC was checked and the reaction was completed. The mixture was added to sat. NH₄Cl/water (25ml/50ml ml) and stirred at room temperature for 30 min. The pH of the mixture was adjusted to about 6 using 2N HCl. After coled with ice for 1h, the solids were collected by filtration, washed by water to give the crude product. The crude product was suspended in DCM (10 ml) and 1 ml of TFA was added (the mixture become clear solution). The mixture was stirred at room temperature for overnight. Potassium phosphate in water was added to the mixture (pH about 8), and extracted with DCM/MeOH. The combined organic was washed by brine, concentrated and purified by column on slica gel (5-15% MeOH in DCM) to give the desired product as yellow solids (97mg, 45% yield).¹H NMR (400 MHz, DMSO-d₆) δ 11.83 (br, 1H), 9.90 (br, 1H), 8.24 (s, 1H), 7.32 (d, J=8.8Hz, 2H), 7.00 (dd, J =5.6Hz, J=10.4Hz,1H), 6.94 (d, J=8.8Hz, 2H), 6.34 (s, 1H), 3.14 (m, 4H), 2.99 (m, 4H), 2.41 (s, 3H) (NH may be berried under solvent peak); ESI-MS: calcd for (C24H21F2N7O) 461, found 462 (MH⁺).

### Reference Compound 15

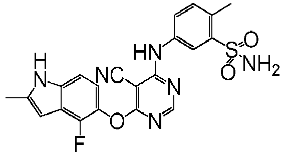

A flask was charged with intermediate 1 (100mg, 0.33 mmol), 5-Amino-2-methylbenzenesulfonamide (77 mg, 0.41 mmol), TFA (50uL), isopropanol (3mL). The reaction was heated to 100°C for 4h. The reaction mixture was basified with a saturated aqueous sodium bicarbonate solution and then was extracted with DCM/ (10mlx3). The combined organic was washed by brine, dried over sodium sulfate and concentrated. The crude product was purified with flash chromatography (0-10% MeOH-in DCM) to afford the desired product as light yellow solids (79 mg, 53% yield). ¹H NMR (400 MHz, DMSO-d₆) δ 11.36 (br, 1H), 10.25 (br, 1H), 8.30 (s, 1H), 8.07 (br, 1H), 7.69 (dd, J=2.4Hz, J=8.4Hz, 1H), 7.39 (br, 2H), 7.37 (d, J = 8.4 Hz, 1H), 7.13 (d, J = 8.8 Hz,1H), 6.95 (t, J = 7.6 Hz, 1H), 6.24 (s, 1H), 2.57 (s, 3H), 2.40 (s, 3H); ESI-MS: calcd for (C21H17FN6O3S) 452, found 453 (MH⁺).

### Reference Compound 16

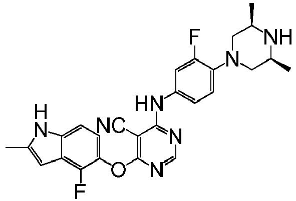

A flask was charged with intermiediate 1 (100mg, 0.33 mmol), 4-((3S,5R)-3,5-dimethylpiperazin-1-yl)-3-fluoroaniline (92 mg, 0.41 mmol), TFA (50uL), isopropanol (3mL). The reaction was heated to 100°C for 4h. The reaction mixture was basified with a saturated aqueous sodium bicarbonate solution and then was extracted with DCM/ (10mlx3). The combined organic was washed by brine, dried over sodium sulfate and concentrated. The crude product was purified with flash chromatography (0-10% MeOH-in DCM) to afford the desired product as yellow solids (118 mg, 73% yield). ¹H NMR (400 MHz, DMSO-d₆) δ 11.35 (br, 1H), 9.98 (br, 1H), 8.29 (s, 1H), 7.40 (dd, J=2.4Hz, J=14.4Hz, 1H), 7.26 (dd, J=2.0Hz, J=8.4Hz, 1H), 7.12 (d, J = 8.8 Hz, 1H), 6.95 (m, 2H), 6.24 (s, 1H), 3.17 (d, J=9.6Hz, 2H), 2.91 (m, 2H), 2.40 (s, 3H), 2.19 (m, 3H), 1.00 (s, 3H), 0.98 (s, 3H); ESI-MS: calcd for (C26H25F2N7O) 489, found 490 (MH⁺).

### Compound 17

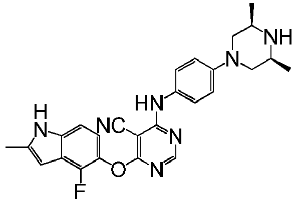

A flask was charged with intermiediate 1 (100mg, 0.33 mmol), 4-((3S,5R)-3,5-dimethylpiperazin-1-yl)aniline (85 mg, 0.41 mmol), TFA (50uL), isopropanol (5mL). The reaction was heated to 100°C for 4h. The reaction mixture was basified with a saturated aqueous sodium bicarbonate solution and then was extracted with DCM/ (10mlx3). The combined organic was washed by brine, dried over sodium sulfate and concentrated. The crude product was purified with flash chromatography (0-10% MeOH-in DCM) to afford the desired product as yellow solids (122 mg, 78% yield). ¹H NMR (400 MHz, DMSO-d₆) δ 11.36 (br, 1H), 9.84 (br, 1H), 8.21 (s, 1H), 7.28 (d, J=8.8Hz, 2H), 7.12 (d, J = 8.8 Hz, 1H), 6.92 (m, 3H), 6.24 (s, 1H), 3.51 (m, 2H), 2.84 (m, 2H), 2.40 (s, 3H), 2.11 (m, 3H), 1.03 (s, 3H), 1.01 (s, 3H); ESI-MS: calcd for (C26H26FN7O) 471, found 472 (MH⁺).

### Reference Compound 18

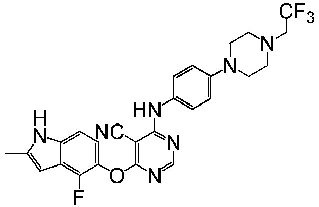

A flask was charged with intermiediate 1 (100mg, 0.33 mmol), 4-(4-(2,2,2-trifluoroethyl)piperazin-1-yl)aniline (86 mg, 0.33 mmol), TFA (50uL), isopropanol (5mL). The reaction was heated to 100°C for 4h. The reaction mixture was basified with a saturated aqueous sodium bicarbonate solution and then was extracted with DCM/ (10mlx3). The combined organic was washed by brine, dried over sodium sulfate and concentrated. The crude product was purified with flash chromatography (0-10% EtOAc in DCM) to afford the desired product as yellow solids (73 mg, 42% yield). ¹H NMR (400 MHz, DMSO-d₆) δ 11.36 (br, 1H), 9.86 (br, 1H), 8.21 (s, 1H), 7.31 (d, J=8.8Hz, 2H), 7.12 (d, J = 8.4 Hz, 1H), 6.94 (m, 3H), 6.24 (s, 1H), 3.23 (q, J=10Hz, 2H), 3.13 (m, 4H), 2.76 (m, 4H), 2.34 (s, 3H); ESI-MS: calcd for (C26H23F4N7O) 525, found 526 (MH⁺).

### Compound 19

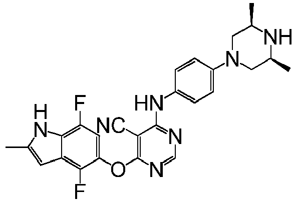

A flask was charged with intermiediate 2 (100mg, 0.31 mmol), 4-((3S,SR)-3,5-dimethylpiperazin-1-yl)aniline (80 mg, 0.39 mmol), TFA (50uL), isopropanol (5mL). The reaction was heated to 100°C for 4h. The reaction mixture was basified with a saturated aqueous sodium bicarbonate solution and then was extracted with DCM/ (10mlx3). The combined organic was washed by brine, dried over sodium sulfate and concentrated. The crude product was purified with flash chromatography (0-10% MeOH-in DCM) to afford the desired product as yellow solids (146 mg, 95% yield). ¹H NMR (400 MHz, DMSO-d₆) δ 11.83 (br, 1H), 9.86 (br, 1H), 8.23 (s, 1H), 7.30 (d, J=8.4Hz, 2H), 7.00 (dd, J=5.6Hz, J = 10.4 Hz, 1H), 6.90 (d, J=8.8Hz, 2H), 6.34 (s, 1H), 3.51 (m, 2H), 2.84 (m, 2H), 2.41 (s, 3H), 2.11 (m, 2H), 1.02 (d, J=6.4Hz, 6H); ESI-MS: calcd for (C26H25F2N7O) 489, found 490 (MH⁺).

### Reference Compound 20

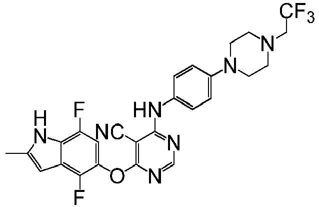

A flask was charged with intermiediate 2 (100mg, 0.31 mmol), 4-(4-(2,2,2-trifluoroethyl)piperazin-1-yl)aniline (101 mg, 0.39 mmol), TFA (50uL), isopropanol (5mL). The reaction was heated to 100°C for 4h. The reaction mixture was basified with a saturated aqueous sodium bicarbonate solution and then was extracted with DCM/ (10mlx3). The combined organic was washed by brine, dried over sodium sulfate and concentrated. The crude product was purified with flash chromatography (0-10% EtOAc in DCM) to afford the desired product as yellow solids (101 mg, 60% yield). ¹H NMR (400 MHz, DMSO-d₆) δ 11.83 (br, 1H), 9.88 (br, 1H), 8.23 (s, 1H), 7.30 (d, J=8.8Hz, 2H), 7.00 (dd, J=5.6Hz, J = 10.8Hz, 1H), 6.94 (d, J=9.2Hz, 2H), 6.34 (s, 1H), 3.25 (q, J=10.0Hz, 2H), 3.14 (m, 4H), 2.76 (m, 4H), 2.41 (s, 3H); ESI-MS: calcd for (C26H22F5N7O) 543, found 544 (MH⁺).

### Reference Compound 21

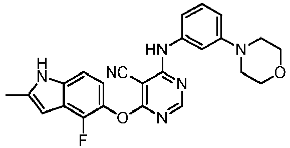

To a mixture of 4-chloro-6-((4-fluoro-2-methyl-1H-indol-5-yl)oxy)pyrimidine-5-carbonitrile (200 mg, 0.661 mmol) and 3-morpholinoaniline (124 mg, 0.694 mmol) in anhydrous DMSO (2.0 mL) was added TEA (0.28 mL; 1.98 mmol), and the resulting biphasic mixture was efficiently stirred at room temperature under argon atmosphere for 18 h. The resulting mixture was diluted with EtOAc (100 mL) and washed with aq. NH₄Cl (ca. 100 mL each; 50% NH₄Cl saturation) followed by brine (100 mL; 50% saturation). The organic layer was separated and dried over anhydrous Na₂SO₄ and concentrated. The resulting residue was purified by crystallization out of EtOAc to afford the desired product as a brown solid (123 mg, 42% yield). ¹H NMR (DMSO-d6, 400 MHz) δ 11.36 (br s, 1H), 9.89 (br s, 1H), 8.28 (s, 1H), 7.23-7.19 (m, 1H), 7.14-7.10 (m, 2H), 7.05-7.03 (m, 1H), 6.96-6.92 (m, 1H), 6.79-6.77 (m, 1H), 3.75-3.73 (m, 4H), 3.11-3.09 (m, 4H), 2.40 (s, 3H); MS (ESI): calcd for C24H21FN6O2: 444, found: 445 (MH⁺).

### Reference Compound 22

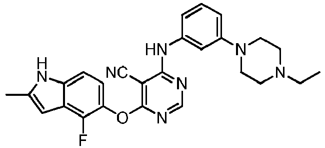

To a mixture of 4-chloro-6-((4-fluoro-2-methyl-1H-indol-5-yl)oxy)pyrimidine-5-carbonitrile (200 mg, 0.661 mmol) and 3-(4-ethylpiperazin-1-yl)aniline (193 mg, 0.694 mmol) in anhydrous DMSO (2.0 mL) was added TEA (0.28 mL; 1.98 mmol), and the resulting biphasic mixture was efficiently stirred at room temperature under argon atmosphere for 18 h. The resulting mixture was diluted with EtOAc (100 mL) and washed with aq. NH₄Cl (ca. 100 mL each; 50% NH₄Cl saturation) followed by brine (100 mL; 50% saturation). The organic layer was separated and dried over anhydrous Na₂SO₄ and concentrated. The resulting residue was purified by crystallization out of EtOAc to afford the desired product as a brown solid (221 mg, 69% yield). ¹H NMR (DMSO-d6, 400 MHz) δ 11.36 (br s, 1H), 9.86 (br s, 1H), 8.28 (s, 1H), 7.20-7.08 (m, 3H), 7.02-6.92 (m, 2H), 6.78-6.75 (m, 1H), 3.14-3.12 (4H), 2.48 (m, 4H); obscured by DMSO signal), 2.40 (s, 3H), 2.37 (q, J = 7.2 Hz, 2H), 1.03 (t, J = 7.2 Hz, 3H); MS (ESI): calcd for C26H26FN7O: 471, found: 472 (MH⁺).

### Reference Compound 23

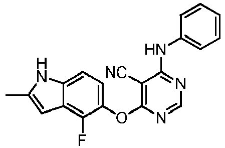

To a mixture of 4-chloro-6-((4-fluoro-2-methyl-1H-indol-5-yl)oxy)pyrimidine-5-carbonitrile (200 mg, 0.330 mmol) and aniline (33 µL, 0.363 mmol) in anhydrous DMSO (2.0 mL) was added TEA (0.14 mL; 0.99 mmol), and the resulting biphasic mixture was efficiently stirred at room temperature under argon atmosphere for 18 h. The resulting mixture was diluted with EtOAc (100 mL) and washed with aq. NH₄Cl (ca. 100 mL each; 50% NH₄Cl saturation) followed by brine (100 mL; 50% saturation). The organic layer was separated and dried over anhydrous Na₂SO₄ and concentrated. The resulting residue was purified by flash column chromatography on silica gel using 0-100% EtOAc in hexanes (v/v) to afford the desired product as an off-white solid (93 mg, 78% yield). ¹H NMR (DMSO-d6, 400 MHz) δ 11.36 (br s, 1H), 10.04 (br s, 1H), 8.29 (s, 1H), 7.53 (d, J = 7.6 Hz, 2H), 7.39-7.35 (m, 2H), 7.20-7.12 (m, 2H), 6.97-6.93 (m, 1H), 6.24 (s, 1H), 2.40 (s, 3H); MS (ESI): calcd for C20H14FN5O: 359, found: 360 (MH⁺).

### Reference Compound 24

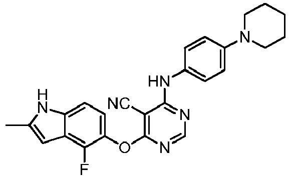

To a mixture of 4-chloro-6-((4-fluoro-2-methyl-1H-indol-5-yl)oxy)pyrimidine-5-carbonitrile (100 mg, 0.330 mmol) and aniline (33 µL, 0.363 mmol) in anhydrous DMSO (2.0 mL) was added TEA (0.14 mL; 0.99 mmol), and the resulting biphasic mixture was efficiently stirred at room temperature under argon atmosphere for 18 h. The resulting mixture was diluted with EtOAc (100 mL) and washed with aq. NH₄Cl (ca. 100 mL each; 50% NH₄Cl saturation) followed by brine (100 mL; 50% saturation). The organic layer was separated and dried over anhydrous Na₂SO₄ and concentrated. The resulting residue was purified by flash column chromatography on silica gel using 0-100% EtOAc in hexanes (v/v) to afford the desired product as an off-white solid (93 mg, 78% yield). ¹H NMR (DMSO-d6, 400 MHz) δ 11.35 (br s, 1H), 9.82 (br s, 1H), 8.21 (d, J = 0.8 Hz, 1H), 7.29 (d, J = 8.0 Hz, 2H), 7.12 (d, J = 8.8 Hz, 1H), 6.95-6.90 (m, 3H), 6.23 (d, J = 0.8 Hz, 1H), 3.14-3.11 (m, 4H), 2.40 (s, 3H), 1.65-1.51 (m, 6H); MS (ESI): calcd for C25H23FN6O: 442, found: 443 (MH⁺).

### Reference Compound 25

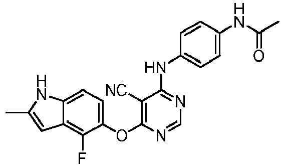

To a mixture of 4-chloro-6-((4-fluoro-2-methyl-1H-indol-5-yl)oxy)pyrimidine-5-carbonitrile (100 mg, 0.330 mmol) and N-(4-aminophenyl)acetamide (54 mg, 0.36 mmol) in anhydrous DMSO (2.0 mL) was added TEA (0.14 mL; 0.99 mmol), and the resulting biphasic mixture was efficiently stirred at room temperature under argon atmosphere for 18 h. The resulting mixture was diluted with EtOAc (100 mL) and washed with aq. NH₄Cl (ca. 100 mL each; 50% NH₄Cl saturation) followed by brine (100 mL; 50% saturation). The organic layer was separated and dried over anhydrous Na₂SO₄ and concentrated. The resulting residue was purified by crystallization out of EtOAc to afford the desired product N-(4-((5-cyano-6-((4-fluoro-2-methyl-1H-indol-5-yl)oxy)pyrimidin-4-yl)amino)phenyl)acetamide as an off-white solid (88 mg, 64% yield). ¹H NMR (DMSO-d6, 400 MHz) δ 11.37 (br s, 1H), 9.99 (br s, 1H), 9.98 (br s, 1H), 8.25 (s, 1H), 7.57-7.55 (m, 2H), 7.42-7.40 (m, 2H), 7.14-7.11 (m, 1H), 6.96-6.92 (m, 1H), 6.24 (s, 1H), 2.40 (s, 3H), 2.04 (s, 3H); MS (ESI): calcd for C22H17FN6O2: 416, found: 417 (MH⁺).

### Reference Compound 26

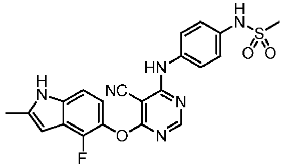

To a mixture of 4-chloro-6-((4-fluoro-2-methyl-1H-indol-5-yl)oxy)pyrimidine-5-carbonitrile (100 mg, 0.330 mmol) and N-(4-aminophenyl)methanesulfonamide (68 mg, 0.36 mmol) in anhydrous isopropanol (5.0 mL) was added TFA (0.05 mL; 0.65 mmol), and the resulting mixture was efficiently stirred at 80 °C under argon atmosphere for 22 h. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated. The resulting residue was purified by crystallization out of EtOAc afford the desired product as an off-white solid (97 mg, 65% yield). ¹H NMR (DMSO-d6, 400 MHz) δ 11.35 (br s, 1H), 10.02 (br s, 1H), 9.71 (br s, 1H), 8.27 (s, 1H), 7.49-7.46 (m, 2H), 7.21-7.11 (m, 3H), 6.96-6.92 (m, 1H), 6.24 (s, 1H), 2.98 (s, 3H), 2.40 (s, 3H); MS (ESI): calcd for C21H17FN6O3S: 452, found: 453 (MH⁺).

### Reference Compound 27

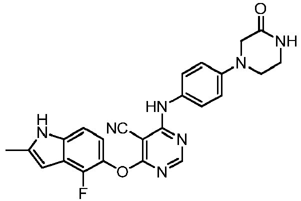

To a mixture of 4-chloro-6-((4-fluoro-2-methyl-1H-indol-5-yl)oxy)pyrimidine-5-carbonitrile (100 mg, 0.330 mmol) and 4-(4-aminophenyl)piperazin-2-one (69 mg, 0.36 mmol) in anhydrous DMSO (3.0 mL) was added TEA (0.14 mL; 0.99 mmol), and the resulting biphasic mixture was efficiently stirred at room temperature under argon atmosphere for 27 h. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated, whereupon a precipitate formed. The precipitate was filtered and washed with EtOAc to afford the desired product as a yellow solid (88 mg, 64% yield). ¹H NMR (DMSO-d6, 400 MHz) δ 11.34 (br s, 1H), 9.86 (br s, 1H), 8.22 (s, 1H), 8.04 (br s, 1H), 7.35-7.33 (m, 2H), 7.12 (d, J = 8.8 Hz, 1H), 6.95-6.91 (m, 2H), 6.24-6.23 (m, 1H), 3.71 (s, 2H), 3.41-3.38 (m, 2H), 3.32-3.29 (m, 2H; obscured by water signal), 2.40 (s, 3H); MS (ESI): calcd for C24H20FN7O2: 457, found: 458 (MH⁺).

### Reference Compound 28

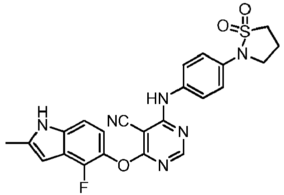

To a mixture of 4-chloro-6-((4-fluoro-2-methyl-1H-indol-5-yl)oxy)pyrimidine-5-carbonitrile (100 mg, 0.330 mmol) and 2-(4-aminophenyl)isothiazolidine 1,1-dioxide (77 mg, 0.36 mmol) in anhydrous isopropanol (5.0 mL) was added TFA (0.05 mL; 0.65 mmol), and the resulting mixture was stirred at 80 °C under argon atmosphere for 22 h. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated, whereupon a precipitate formed. The precipitate was filtered and washed with EtOAc to afford the desired product as an off-white solid (97 mg, 65% yield). ¹H NMR (DMSO-d6, 400 MHz) δ 11.37 (br s, 1H), 10.06 (br s, 1H), 8.26 (s, 1H), 7.52-7.49 (m, 2H), 7.22-7.19 (m, 2H), 7.12 (d, J = 8.4 Hz, 1H), 6.97-6.93 (m, 1H), 3.74 (t, J = 6.4 Hz, 2H), 3.51 (t, J = 7.2 Hz, 2H), 2.44-2.37 (m, 2H), 2.40 (s, 3H); MS (ESI): calcd for C23H19FN6O3S: 478, found: 479 (MH⁺).

### Reference Compound 29

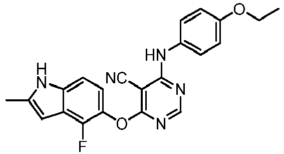

To a mixture of 4-chloro-6-((4-fluoro-2-methyl-1H-indol-5-yl)oxy)pyrimidine-5-carbonitrile (100 mg, 0.330 mmol) and 4-ethoxyaniline (47 µL, 0.36 mmol) in anhydrous DMSO (3.0 mL) was added TEA (0.14 mL; 0.99 mmol), and the resulting biphasic mixture was efficiently stirred at room temperature under argon atmosphere for 23 h. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated. The resulting residue was purified by flash column chromatography on silica gel using 0-100% EtOAc in hexanes (v/v) to afford the desired product as a yellow solid (111 mg, 83% yield). ¹H NMR (DMSO-d6, 400 MHz) δ 11.35 (br s, 1H), 9.90 (br s, 1H), 8.22 (s, 1H), 7.37 (d, J = 9.2 Hz, 1H), 7.12 (d, J = 8.8 Hz, 1H), 6.95-6.90 (m, 3H), 6.23 (s, 1H), 4.02 (q, J = 6.8 Hz, 2H), 2.40 (s, 3H), 1.33 (t, J = 6.8 Hz, 3H); MS (ESI): calcd for C22H18FN5O2: 403, found: 404 (MH⁺).

### Reference Compound 30

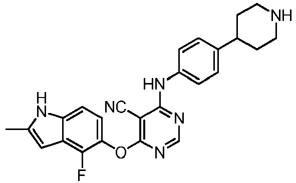

Step 1: To a mixture of 4-chloro-6-((4-fluoro-2-methyl-1H-indol-5-yl)oxy)pyrimidine-5-carbonitrile (200 mg, 0.661 mmol) and tert-butyl 4-(4-aminophenyl)piperidine-1-carboxylate (201 mg, 0.727 mmol) in anhydrous DMSO (3.0 mL) was added TEA (0.28 mL; 1.98 mmol), and the resulting biphasic mixture was efficiently stirred at room temperature under argon atmosphere for 20 h. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated. The resulting residue was purified by flash column chromatography on silica gel using 0-100% EtOAc in hexanes (v/v) to afford the desired product as a yellow solid (322 mg, 90% yield). ¹H NMR (DMSO-d6, 400 MHz) δ 11.36 (br s, 1H), 9.98 (br s, 1H), 8.25 (s, 1H), 7.42 (d, J = 8.4 Hz, 2H), 7.23 (d, J = 8.8 Hz, 2H), 7.12 (d, J = 8.4 Hz, 1H), 6.96-6.92 (m, 1H), 6.24-6.23 (m, 1H), 4.11-4.06 (m, 1H), 2.84-2.78 (m, 2H), 2.70-2.64 (m, 2H), 2.40 (s, 3H), 1.77-1.74 (m, 2H), 1.54-1.41 (m, 2H), 1.42 (s, 9H); MS (ESI): calcd for C30H31FN6O3: 542, found: 442 (M-BOC+H⁺).

Step 2: A mixture of tert-butyl 4-(4-((5-cyano-6-((4-fluoro-2-methyl-1H-indol-5-yl)oxy) pyrimidin-4-yl)amino)phenyl)piperidine-1-carboxylate (200 mg, 0.369 mmol) was stirred into 8% TFA in DCM (10 mL) and the resulting mixture was stirred at room temperature for 17 h. The resulting mixture was diluted with EtOAc (100 mL) and washed with aq. NaHCO₃ (ca. 100 mL each; 50% NaHCO₃ saturation) whereupon a precipitate formed. The precipitate was filtered and washed with EtOAc. The mother liquid was concentrated to afford the desired product as a glassy, white solid (60 mg, 40% yield). ¹H NMR (DMSO-d6, 400 MHz) δ 11.37 (br s, 1H), 9.99 (br s, 1H), 8.26 (s, 1H), 7.44-7.42 (m, 2H), 7.23-7.20 (m, 2H), 7.13 (d, J = 8.8 Hz, 1H), 6.96-6.92 (m, 1H), 6.24-6.23 (m, 1H), 3.13-3.10 (m, 2H), 2.72-2.60 (m, 3H), 2.40 (s, 3H), 1.77-1.72 (m, 2H), 1.62-1.52 (m, 2H); MS (ESI): calcd for C25H23FN6O: 442, found: 443 (MH⁺).

### Reference Compound 31

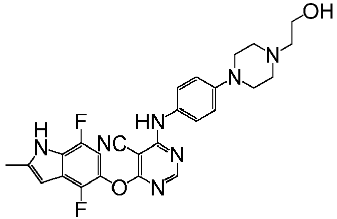

Step 1: To a solution of 4-fluoronitrobenzene (2.0g, 14.16 mmol) in AcN (15 mL), 2-(piperazin-1-yl)ethanol (1.85 g, 14.17mmol) and DIEA (2.97 mL, 17.01 mmol) were added. The mixture was refluxed for 15 h (in a sealed tube). After cooling, the resulting mixture was poured to water (300 ml). The mixture was stirred at room temperature for 30 min. The solids were collected by filtration and washed with water to afford the the desired product as off white solids (2.74g, 72% yield). ESI-MS calcd for (C12H17N3O3) 251, found 252 [M+H]⁺.

Step 2: A solution of the above product (2.74 g) in methanol (50 mL) was hydrogenated in the presence of 10% Pd/C (270 mg) by using an H₂ balloon. After 16 h, the reaction mixture was filtered through a pad of Celite and rinsed with methanol (3 x 15 mL). The filtrate was concentrated to the desired product (2.70 g) as light yellow solids. The product was used directly in the next step without further purification. ESI-MS: calcd for (C13H21N3) 219, found 220 (MH⁺).

Step 3: A flask was charged with intermiediate 2 (50 mg, 0.16 mmol), 2-(4-(4-aminophenyl)piperazin-1-yl)ethanol (30.4 mg, 0.16 mmol), DIPEA (60uL), DMSO (2 mL). The reaction was stirred at room temperature for over. The crude product was purified with flash chromatography (0-10% MeOH-in DCM) to afford the desired product as light yellow solids (42 mg, 54% yield). ¹H NMR (400 MHz, DMSO-d₆) δ 11.83 (br, 1H), 9.86 (br, 1H), 8.23 (s, 1H), 7.30 (d, J=8.4Hz, 2H), 7.00 (dd, J=5.6Hz, J = 10.4 Hz, 1H), 6.90 (d, J=8.8Hz, 2H), 6.34 (s, 1H), 3.51 (m, 2H), 2.84 (m, 2H), 2.41 (s, 3H), 2.11 (m, 2H), 1.03 (s, 3H), 1.01 (s, 3H); ESI-MS: calcd for (C26H25F2N7O) 489, found 490 (MH⁺).

### Reference Compound 32

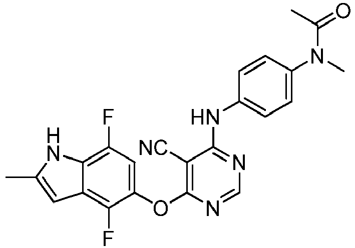

A flask was charged with intermiediate 2 (50 mg, 0.16 mmol), 2-(4-(4-aminophenyl)piperazin-1-yl)ethanol (30.4 mg, 0.16 mmol), DIPEA (60uL), DMSO (2 mL). The reaction was stirred at room temperature for over. The crude product was purified with flash chromatography (0-10% MeOH-in DCM) to afford the desired product as light yellow solids (42 mg, 54% yield). ¹H NMR (400 MHz, DMSO-d₆) δ 11.83 (br, 1H), 9.86 (br, 1H), 8.23 (s, 1H), 7.30 (d, J=8.4Hz, 2H), 7.00 (dd, J=5.6Hz, J = 10.4 Hz, 1H), 6.90 (d, J=8.8Hz, 2H), 6.34 (s, 1H), 3.51 (m, 2H), 2.84 (m, 2H), 2.41 (s, 3H), 2.11 (m, 2H), 1.03 (s, 3H), 1.01 (s, 3H); ESI-MS: calcd for (C26H25F2N7O) 489, found 490 (MH⁺).

### Compound 33 (S) configuration

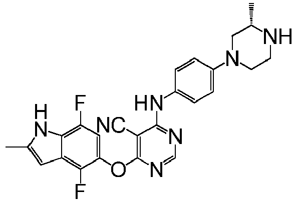

Step 1: To a solution of 4-fluoronitrobenzene (0.5g, 3.54 mmol) in AcN (30 mL), (S)-tert-butyl 2-methylpiperazine-1-carboxylate (0.71 g, 3.54 mmol) and DIEA (0.74 mL, 4.25 mmol) were added. The mixture was refluxed for 15 h (in a sealed tube). After cooling, the resulting mixture was poured to water (300 ml). The mixture was stirred at room temperature for 30 min. The resulting reaction mixture was extracted with EtOAc (3x30 mL) and anhydrous Na₂SO₄ and concentrated *in vacuum.* The resulting crude product was purified by Teledyne-Isco flash system by using EtOAc/Hex, 0 to 30% of ethylacetate in hexane to provide compound tert-butyl (S)-4-(4-nitrophenyl)-2-methylpiperazine-1-carboxylate as light yellow solids (710 mg, 62%) as off white solids. ¹H NMR (400 MHz, DMSO-d₆) δ 8.01 (d, J=9.6Hz, 2H), 6.97 (d, J =9.2Hz, 2H), 3.84 (m, 2H), 2.95-2.50 (m, 4H), 2.40 (m, 1H), 2.30 (br, 1H), 1.00 (d, J=6.0Hz, 3H); ESI-MS: calcd for (C11H15N3O2) 221, found 222 (MH⁺).

Step 2: A solution of tert-butyl (S)-4-(4-nitrophenyl)-2-methylpiperazine-1-carboxylatein methanol (30 mL) was hydrogenated in the presence of 10% Pd/C (70 mg) by using an H₂ balloon. After 16 h, the reaction mixture was filtered through a pad of Celite and rinsed with methanol (3 x 15 mL). The filtrate was concentrated to afford the title compound tert-butyl (S)-4-(4-aminophenyl)-2-methylpiperazine-1-carboxylate (0.62 g, 97%) as light yellow solids. The product was used directly in the next step without further purification. ESI-MS: calcd for (C13H21N3) 219, found 220 (MH⁺).

Step 3: A mixture of Intermediate 2 (150 mg, 0.47 mmol), (S)-tert-butyl 2-methylpiperazine-1-carboxylate (LN927, 0.05g, 0.016 mmol), and DIPEA (60uL, 0.037 mmol) in DMSO (3.0 ml) was stirred at roomtemperaure for overnight. TLC was checked and the reaction was completed. The mixture was added to sat. NH₄Cl/water (25ml/50ml ml) and stirred at room temperature for 30 min. The pH of the mixture was adjusted to about 6 using 2N HCl. After coled with ice for 1h, the solids were collected by filtration, washed by water to give the crude product. The crude product was suspended in DCM (10 ml) and 1 ml of TFA was added (the mixture become clear solution). The mixture was stirred at room temperature for overnight. Potassium phosphate in water was added to the mixture (pH about 8), and extracted with DCM/MeOH. The combined organic was washed by brine, concentrated and purified by column on slica gel (5-15% MeOH in DCM) to give the desired product as yellow solids (97mg, 45% yield).¹H NMR (400 MHz, DMSO-d₆) δ 11.83 (br, 1H), 9.90 (br, 1H), 8.24 (s, 1H), 7.32 (d, J=8.8Hz, 2H), 7.00 (dd, J =5.6Hz, J=10.4Hz,1H), 6.94 (d, J=8.8Hz, 2H), 6.34 (s, 1H), 3.14 (m, 4H), 2.99 (m, 4H), 2.41 (s, 3H) (NH may be berried under solvent peak); ESI-MS: calcd for (C24H21F2N7O) 461, found 462 (MH⁺).

### Compound 34 (R) configuration

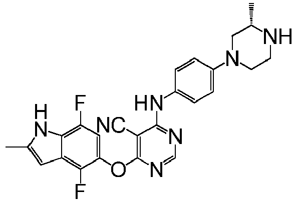

Step 1: To a solution of 4-fluoronitrobenzene (0.5g, 3.54 mmol) in AcN (30 mL), (R)-tert-butyl 2-methylpiperazine-1-carboxylate (0.71 g, 3.54 mmol) and DIEA (0.74 mL, 4.25 mmol) were added. The mixture was refluxed for 15 h (in a sealed tube). After cooling, the resulting mixture was poured to water (300 ml). The mixture was stirred at room temperature for 30 min. The resulting reaction mixture was extracted with EtOAc (3x30 mL) and dried over anhydrous Na₂SO₄ and concentrated *in vacuum.* The resulting crude product was purified by Teledyne-Isco flash system by using EtOAc/Hex, 0 to 30% of ethylacetate in hexane to provide the desired product tert-butyl (S)-2-methyl-4-(4-nitrophenyl)piperazine-1-carboxylate as light yellow solids (720 mg, 63%) as off white solids. ¹H NMR (400 MHz, DMSO-d₆) δ 8.01 (d, J=9.6Hz, 2H), 6.97 (d, J =9.2Hz, 2H), 3.84 (m, 2H), 2.95-2.50 (m, 4H), 2.40 (m, 1H), 2.30 (br, 1H), 1.00 (d, J=6.0Hz, 3H); ESI-MS: calcd for (C11H15N3O2) 221, found 222 (MH⁺).

Step 2: A solution of tert-butyl (S)-2-methyl-4-(4-nitrophenyl)piperazine-1-carboxylate (0.7 g) in methanol (30 mL) was hydrogenated in the presence of 10% Pd/C (70 mg) by using an H₂ balloon. After 16 h, the reaction mixture was filtered through a pad of Celite and rinsed with methanol (3 x 15 mL). The filtrate was concentrated to afford the title compound tert-butyl (S)-4-(4-aminophenyl)-2-methylpiperazine-1-carboxylate (0.61 g, 95%) as light yellow solids. The product was used directly in the next step without further purification. ESI-MS: calcd for (C13H21N3) 219, found 220 (MH⁺).

Step 3: A mixture of intermediate 3 (150 mg, 0.47 mmol), tert-butyl (S)-4-(4-aminophenyl)-2-methylpiperazine-1-carboxylate (0.05g, 0.016 mmol), and DIPEA (60uL, 0.037 mmol) in DMSO (3.0 ml) was stirred at roomtemperaure for overnight. TLC was checked and the reaction was completed. The mixture was added to sat. NH₄Cl/water (25ml/50ml ml) and stirred at room temperature for 30 min. The pH of the mixture was adjusted to about 6 using 2N HCl. After coled with ice for 1h, the solids were collected by filtration, washed by water to give the crude product. The crude product was suspended in DCM (10 ml) and 1 ml of TFA was added (the mixture become clear solution). The mixture was stirred at room temperature for overnight. Potassium phosphate in water was added to the mixture (pH about 8), and extracted with DCM/MeOH. The combined organic was washed by brine, concentrated and purified by column on slica gel (5-15% MeOH in DCM) to give the desired product as yellow solids (97mg, 45% yield). ¹H NMR (400 MHz, DMSO-d₆) δ 11.83 (br, 1H), 9.90 (br, 1H), 8.24 (s, 1H), 7.32 (d, J=8.8Hz, 2H), 7.00 (dd, J =5.6Hz, J=10.4Hz,1H), 6.94 (d, J=8.8Hz, 2H), 6.34 (s, 1H), 3.14 (m, 4H), 2.99 (m, 4H), 2.41 (s, 3H) (NH may be berried under solvent peak); ESI-MS: calcd for (C24H21F2N7O) 461, found 462 (MH⁺).

### Reference Compound 35

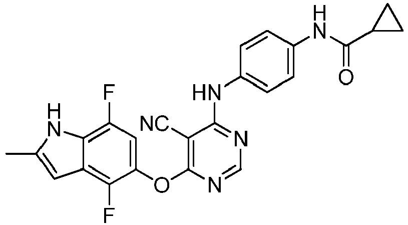

Step 1: To a mixture of 4-Nitroaniline (1.0 g, 7.24 mmol) and cyclopropyl carbonylchloride (0.83g, 7.96 mmol) were dissolved in Dry THF (30 mL). DIPEA (3.16 mL, 18.10 mmol) was added to above reaction mixture and stirred at the room temperature for over night. The mixture was stirred at room temperature for 30 min. The resulting crude mixture was extracted with EtOAc (3x30 mL) and dried over anhydrous Na₂SO₄ and concentrated *in vacuum.* The resulting crude product was purified by Teledyne-Isco flash system by using EtOAc/Hex, 0 to 30% of ethylacetate in hexane to provide the desired product (4-nitrophenyl)cyclopropanecarboxamide (880 mg, 62%) as off white solids. ESI-MS: calcd for (C10H10N2O3) 206, found 207 (MH⁺).

Step 2: A solution (4-nitrophenyl)cyclopropanecarboxamide (0.7 g) in methanol (30 mL) was hydrogenated in the presence of 10% Pd/C (70 mg) by using an H₂ balloon. After 16 h, the reaction mixture was filtered through a pad of Celite and rinsed with methanol (3 x 15 mL). The filtrate was concentrated to afford the title compound (4-aminophenyl)cyclopropanecarboxamide
as light yellow solids. The product was used directly in the next step without further purification.

Step 3: A flask was charged with FA425_1 (100 mg, 0.312 mmol), N-(4-aminophenyl)cyclopropanecarboxamide (LN933_1, 0.055g, 0.312 mmol), DIPEA (60uL), DMSO (2 mL). The reaction was stirred at room temperature for over. The crude product was purified with flash chromatography (0-10% MeOH-in DCM) to afford the desired product as light yellow solids (85 mg, 54% yield). ¹H NMR (400 MHz, DMSO-d₆) δ 11.83 (br, 1H), 9.86 (br, 1H), 8.23 (s, 1H), 7.30 (d, J=8.4Hz, 2H), 7.00 (dd, J=5.6Hz, J = 10.4 Hz, 1H), 6.90 (d, J=8.8Hz, 2H), 6.34 (s, 1H), 3.51 (m, 2H), 2.84 (m, 2H), 2.41 (s, 3H), 2.11 (m, 2H), 1.03 (s, 3H), 1.01 (s, 3H); ESI-MS: calcd for (C26H25F2N7O) 489, found 490 (MH⁺).

### Reference Compound 36

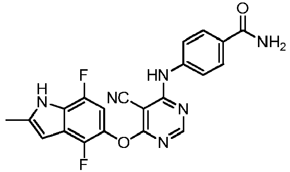

To a mixture of 4-chloro-6-((4-fluoro-2-methyl-1H-indol-5-yl)oxy)pyrimidine-5-carbonitrile (100 mg, 0.330 mmol) and 4-aminobenzamide (47 mg, 0.34 mmol) in anhydrous DMSO (1.5 mL) was added TEA (0.13 mL; 0.93 mmol), and the resulting biphasic mixture was efficiently stirred at room temperature under argon atmosphere for ca. 17 h. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated. The resulting residue was purified by flash column chromatography on silica gel using 0.5-15% MeOH in DCM (v/v) to afford the desired product as a white solid (42 mg, 32% yield). ¹H NMR (DMSO-d6, 400 MHz) δ 11.85 (br s, 1H), 10.22 (brs, 1H), 8.35 (s, 1H), 7.91 (br s, 1H), 7.86 (d, J = 8.8 Hz, 2H), 7.62 (d, J = 8.4 Hz, 2H), 7.29 (br s, 1H), 7.04-7.00 (m, 1H), 6.35 (s, 1H), 2.42 (s, 3H); MS (ESI): calcd for C21H14F2N6O2: 420, found: 421 (MH⁺).

### Reference Compound 37

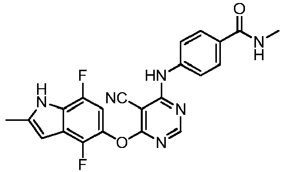

To a mixture of 4-chloro-6-((4,7-difluoro-2-methyl-1H-indol-5-yl)oxy)pyrimidine-5-carbonitrile (80 mg, 0.25 mmol) and 4-amino-N-methylbenzamide (45 mg, 0.30 mmol) in anhydrous DMSO (1.5 mL) was added TEA (0.122 mL; 0.875 mmol), and the resulting biphasic mixture was efficiently stirred at room temperature for ca. 3 days. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated. The resulting residue was purified by flash column chromatography on silica gel using 0.5-15% MeOH in DCM (v/v) to afford the desired product as a white solid (18 mg, 17% yield). ¹H NMR (DMSO-d6, 400 MHz) δ 11.85 (br s, 1H), 10.23 (br s, 1H), 8.37-8.34 (m, 2H), 7.81 (d, J = 8.8 Hz, 2H), 7.64-7.62 (m, 2H), 7.04-7.00 (m, 1H), 6.35 (s, 1H), 2.78 (d, J = 4.0 Hz, 3H), 2.42 (s, 3H); MS (ESI): calcd for C22H16F2N6O2: 434, found: 435 (MH⁺).

### Reference Compound 38

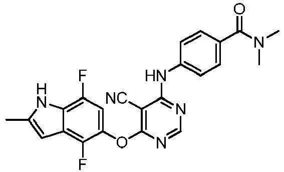

To a mixture of 4-chloro-6-((4,7-difluoro-2-methyl-1H-indol-5-yl)oxy)pyrimidine-5-carbonitrile (80 mg, 0.25 mmol) and 4-amino-N,N-dimethylbenzamide (49 mg, 0.30 mmol) in anhydrous DMSO (1.5 mL) was added TEA (0.122 mL; 0.875 mmol), and the resulting biphasic mixture was efficiently stirred at room temperature for ca. 3 days. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated. The resulting residue was purified by crystallization (DCM) to afford the desired product as a white solid (49 mg, 44% yield). ¹H NMR (DMSO-d6, 400 MHz) δ 11.85 (br s, 1H), 10.20 (br s, 1H), 8.33 (s, 1H), 7.60 (d, J = 8.4 Hz, 2H), 7.41 (d, J = 8.4 Hz, 2H), 7.04-7.00 (m, 1H), 6.35 (s, 1H), 2.97 (s, 6H), 2.42 (s, 3H); MS (ESI): calcd for C23H18F2N6O2: 448, found: 449 (MH⁺).

### Reference Compound 39

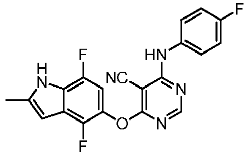

To a mixture of 4-chloro-6-((4,7-difluoro-2-methyl-1H-indol-5-yl)oxy)pyrimidine-5-carbonitrile (80 mg, 0.25 mmol) and 4-fluoroaniline (33 mg, 0.30 mmol) in anhydrous DMSO (1.5 mL) was added TEA (0.122 mL; 0.875 mmol), and the resulting biphasic mixture was efficiently stirred at room temperature for ca. 3 days. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated. The resulting residue was purified by crystallization (DCM) to afford the desired product as a white solid (65 mg, 66% yield). ¹H NMR (DMSO-d6, 400 MHz) δ 11.84 (br s, 1H), 10.10 (br s, 1H), 8.29 (s, 1H), 7.54-7.51 (m, 2H), 7.23-7.29 (m, 2H), 7.03-6.99 (m, 1H), 6.34 (s, 1H), 2.42 (s, 3H); MS (ESI): calcd for C20H12F3N5O: 395, found: 396 (MH⁺).

### Reference Compound 40

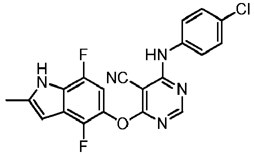

To a mixture of 4-chloro-6-((4,7-difluoro-2-methyl-1H-indol-5-yl)oxy)pyrimidine-5-carbonitrile (80 mg, 0.25 mmol) and 4-chloroaniline (33 mg, 0.30 mmol) in anhydrous DMSO (1.5 mL) was added TEA (0.122 mL; 0.875 mmol), and the resulting biphasic mixture was efficiently stirred at room temperature for ca. 3 days. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated. The resulting residue was purified by crystallization (DCM) to afford the desired product as a white solid (65 mg, 44% yield). ¹H NMR (DMSO-d6, 400 MHz) δ 11.85 (br s, 1H), 10.15 (br s, 1H), 8.34 (d, J = 1.2 Hz, 1H), 7.59-7.57 (m, 2H), 7.43 (d, J = 8.0 Hz, 2H), 7.04-7.00 (m, 1H), 6.35 (s, 1H), 2.42 (s, 3H); MS (ESI): calcd for C20H12ClF2N5O: 411, found: 412 (MH⁺).

### Reference Compound 41

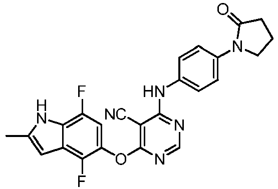

To a mixture of 4-chloro-6-((4,7-difluoro-2-methyl-1H-indol-5-yl)oxy)pyrimidine-5-carbonitrile (80 mg, 0.25 mmol) and 1-(4-aminophenyl)pyrrolidin-2-one (53 mg, 0.30 mmol) in anhydrous DMSO (1.5 mL) was added TEA (0.122 mL; 0.875 mmol), and the resulting biphasic mixture was efficiently stirred at room temperature for ca. 3 days. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated. The resulting residue was purified by crystallization (DCM) to afford the desired product as a white solid (56 mg, 49% yield). ¹H NMR (DMSO-d6, 400 MHz) δ 11.34 (br s, 1H), 10.07 (br s, 1H), 8.26 (s, 1H), 7.64 (d, J = 8.8 Hz, 2H), 7.48 (d, J = 8.8 Hz, 2H), 7.03-6.99 (m, 1H), 6.34 (s, 1H), 3.84 (t, J = 6.8 Hz, 2H), 2.50-2.48 (m, 2H; obscured by DMSO signal), 2.42 (s, 3H), 2.11-2.03 (m, 2H); MS (ESI): calcd for C24H18F2N6O2: 460, found: 461 (MH⁺).

### Reference Compound 42

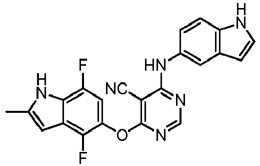

To a mixture of 4-chloro-6-((4,7-difluoro-2-methyl-1H-indol-5-yl)oxy)pyrimidine-5-carbonitrile (80 mg, 0.25 mmol) and 1H-indol-5-amine (40 mg, 0.30 mmol) in anhydrous DMSO (1.5 mL) was added TEA (0.122 mL; 0.875 mmol), and the resulting biphasic mixture was efficiently stirred at room temperature for ca. 3 days. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated. The resulting residue was purified by flash column chromatography on silica gel using 0.5-15% MeOH in DCM (v/v) to afford the desired product as a white solid (32 mg, 33% yield). ¹H NMR (DMSO-d6, 400 MHz) δ 11.83 (br s, 1H), 11.14 (br s, 1H), 10.00 (brs, 1H), 8.21 (s, 1H), 7.58 (s, 1H), 7.39-7.36 (m, 2H), 7.14-7.11 (m, 1H), 7.03-6.99 (m, 1H), 6.44-6.42 (m, 1H), 6.34 (s, 1H), 5.76 (d, J = 0.8 Hz, 1H), 2.41 (s, 3H); MS (ESI): calcd for C22H14F2N6O: 416, found: 417 (MH⁺).

### Reference Compound 43

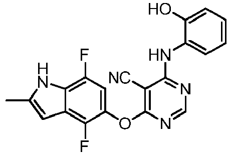

To a mixture of 4-chloro-6-((4,7-difluoro-2-methyl-1H-indol-5-yl)oxy)pyrimidine-5-carbonitrile (80 mg, 0.25 mmol) and 1H-indol-5-amine (40 mg, 0.30 mmol) in anhydrous DMSO (1.5 mL) was added TEA (0.122 mL; 0.875 mmol), and the resulting biphasic mixture was efficiently stirred at room temperature for ca. 3 days. The resulting mixture was diluted with EtOAc (100 mL) and washed with aq. NaHCO₃ (ca. 100 mL each; 50% NaHCO₃ saturation). The organic layer was separated and dried over anhydrous Na₂SO₄ and concentrated. The resulting residue was purified by flash column chromatography on silica gel using 0.5-15% MeOH in DCM (v/v) to afford the desired product as a white solid (32 mg, 33% yield). ¹H NMR (DMSO-d6, 400 MHz) δ 11.84 (br s, 1H), 9.76 (br s, 1H), 9.57 (br s, 1H), 8.24 (s, 1H), 7.40 (d, J = 8.0 Hz, 1H), 7.13-7.09 (m, 1H), 7.02-6.98 (m, 1H), 6.91 (d, J = 8.0 Hz, 1H), 6.84-6.80 (m, 1H), 6.34 (d, J = 3.2 Hz, 1H), 2.41 (s, 3H); MS (ESI): calcd for C20H13F2N5O2: 393, found: 394 (MH⁺).

### Reference Compound 44

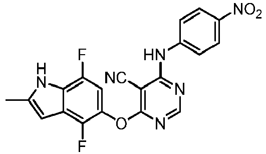

A mixture of 4-chloro-6-((4-fluoro-2-methyl-1H-indol-5-yl)oxy)pyrimidine-5-carbonitrile (100 mg, 0.31 mmol), 4-nitroaniline (47 mg, 0.34 mmol), Pd(OAc)₂ (10 mg, 0.045 mmol), xantphos (45 mg, 0.078 mmol), K₂CO₃ (150 mg, 1.09 mmol) and anhydrous dioxane (12 mL) was sealed in a microwavable tube and degassed with argon for 10 min. The mixture was then heated to 120 °C for 20 min under microwave irradiation. The mixture was cooled to room temperature and partitioned between EtOAc and aq. NaHCO₃ (ca. 100 mL each; 50% saturated NaHCO₃), and the organic layer was separated, dried over anhydrous Na₂SO₄ and concentrated. The resulting residue was taken up into EtOAc, filtered, and washed with additional EtOAc to yield the desired product as a light yellow solid (54 mg, 41% yield). ¹H NMR (DMSO-d6, 400 MHz) δ 11.86 (br s, 1H), 10.59 (br s, 1H), 8.47 (s, 1H), 8.26-8.23 (m, 2H), 7.92-7.88 (m, 2H), 7.06-7.02 (m, 1H), 6.36 (s, 1H), 2.42 (s, 3H); MS (ESI): calcd for C20H12F2N6O3: 422, found: 423 (MH⁺).

### Reference Compound 45

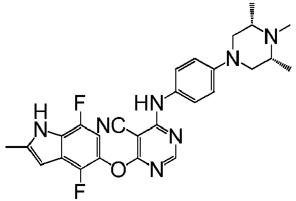

Step 1: To a solution of 4-fluoronitrobenzene (3g, 21.26 mmol) in AcN (60 mL), cis-2,6-dimethylpiperazine (2.55 g, 22.32 mmol) and DIEA (3.90 mL, 22.32 mmol) were added. The mixture was stirred and refluxed for 18 h. The resulting mixture was cooled until room temperature and evaporatedto afford. EtOAc and water was added. The mixture was extracted with EtOAc three times. The organic was washed with brine, dried over sodium sulfate and concentrated. The resisue was crystalized from EtOAc to give the yellow solids of reversed connection of the side product (300mg). The mother liquid was concentrated to minimum amount of solvents and then Hexanes was added to form the yellow precipitate. The solids were collected by filtration and washed with hexanes to afford the desire compound (3R,5S)-3,5-dimethyl-1-(4-nitrophenyl)piperazine as light yellow solids (2.60 g, 52% yield). ¹H NMR (400 MHz, DMSO-d₆) δ 8.02 (d, J=9.6Hz, 2H), 7.02 (d, J =9.2Hz, 2H), 3.90 (m, 2H), 2.78 (m, 2H), 2.40 (m, 2H), 1.03 (d, J=5.6Hz, 6H); ESI-MS: calcd for (C12H17N3O2) 235, found 236 (MH⁺).

Step 2: To a solution of (3R,5S)-3,5-dimethyl-1-(4-nitrophenyl)piperazine (600 mg, 2.55 mmol) in DMF (6 mL) was added sodium hydride (60%, 122 mg g, 3.06 mmol) potion wise and the mixture was stiired at room temperature for 30 min. then idomethan (0.19 ml g, 3.06 mmol) was added. The reaction mixture was stirred at room temperature for overnight. TLC was checked and the starting material was consumed. The mixture was poured to cold water potionwise and the mixture was extracted with EtOAc (3x50 ml). The combined organic phase was washed with water, dried over sodium sulfate and concentrated to give 4-((3R,5S)-3,4,5-trimethylpiperazin-1-yl)nitrobenzine as a yellow solid (100 mg, 15% yield). No further purification was conducted. ESI-MS: calcd for (C13H19N3O2) 249, found 250(MH⁺).

Step 3: A solution of (3R,5S)-3,5-dimethyl-1-(4-nitrophenyl)piperazine (∼100 mg) in methanol (20 mL) was hydrogenated in the presence of 10% Pd/C (10 mg) using an H₂ balloon. After 16 h, the reaction mixture was filtered through a pad of Celite and rinsed with methanol (3 x 15 mL). The filtrate was concentrated to afford the desired product 4-((3R,5S)-3,4,5-trimethylpiperazin-1-yl)aniline (51 mg, 9% yield for 2 steps) as red solids. The product was used directly for the next step reaction without further purification. ESI-MS: calcd for (C13H21N3) 219, found 220 (MH⁺).

Step 4: A flask was charged with Intermediate 2 (50mg, 0.16 mmol), 4-((3R,5S)-3,4,5-trimethylpiperazin-1-yl)aniline (38 mg, 0.17 mmol), TFA (50uL), isopropanol (5mL). The reaction was heated to 80°C for overnight. The reaction mixture was basified with a saturated aqueous potassium phospate solution and then was extracted with DCM/ (10mlx3). The combined organic was dried over sodium sulfate and concentrated. The crude product was purified with flash chromatography (0-10% MeOH-in DCM) to afford the desired product as yellow solids (51 mg, 65% yield). ¹H NMR (400 MHz, DMSO-d₆) δ 11.84 (br, 1H), 9.87 (br, 1H), 8.23 (s, 1H), 7.30 (d, J=8.8Hz, 2H), 7.00 (dd, J=5.6Hz, J = 10.4 Hz, 1H), 6.91 (d, J=9.2Hz, 2H), 6.34 (s, 1H), 3.54 (d, J=11.2Hz, 2H), 2.41 (s, 3H), 2.34 (d, J=11.6Hz, 2H), 2.24 (m, 2H), 2.19 (s, 3H),1.07 (d, J=6.0Hz, 6H); ESI-MS: calcd for (C27H27F2N7O) 503, found 504 (MH⁺).

### Reference Compound 46

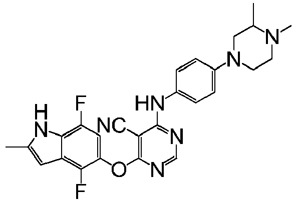

Step 1: To a solution of 4-fluoronitrobenzene (1.80g, 12.76 mmol) in AcN (18 mL), 2-methylpiperazine (3.19 g, 31.89 mmol) and DIEA (3.34 mL, 19.14 mmol) were added. The mixture was stirred at 75 °C for 3 h (in a sealed tube). The resulting mixture was cooled until room temperature and then transferred to water (300 ml). The mixture was stirred at room temperature for 30 min. then cooled with ice. The solids were collected by filtration and washed with water to afford the crude product, which was purified by column chromatography (0-10% MeOH in DCM) to afford the desired compound of 3-methyl-1-(4-nitrophenyl)piperazine as yellow solids (2.03g, 72% yield). ¹H NMR (400 MHz, DMSO-d₆) δ 8.01 (d, J=9.6Hz, 2H), 6.97 (d, J =9.2Hz, 2H), 3.84 (m, 2H), 2.95-2.50 (m, 4H), 2.40 (m, 1H), 2.30 (br, 1H), 1.00 (d, J=6.0Hz, 3H); ESI-MS: calcd for (C11H15N3O2) 221, found 222 (MH⁺).

Step 2: To a solution of 3-methyl-1-(4-nitrophenyl)piperazine (600 mg, 2.71 mmol) in DMF (6 mL) was added sodium hydride (60%, 130 mg g, 3.25 mmol) potion wise and the mixture was stiired at room temperature for 30 min. then idomethan (0.20 ml g, 3.25 mmol) was added. The reaction mixture was stirred at room temperature for overnight. TLC was checked and the starting material was consumed. The mixture was poured to cold water potionwise and the mixture was extracted with EtOAc (3x50 ml). The combined organic phase was washed with water, dried over sodium sulfate and concentrated to give the crude product which was purified by column chromatography (0-10% MeOH in DCM) to give the yellow oil. ESI-MS: calcd for (C12H17N3O2) 235, found 236(MH⁺).

Step 3: A solution of 4-(3,4-dimethylpiperazin-1-yl)nitrobenzine in methanol (∼20 mL) was hydrogenated in the presence of Pd/C (25 mg) using an H₂ balloon. After 16 h, the reaction mixture was filtered through a pad of Celite and rinsed with methanol (3 x 15 mL). The filtrate was concentrated to the desired product 4-(3,4-dimethylpiperazin-1-yl)aniline (398 mg, 72% yield for 2 steps) as brown solids. The product was used directly for the next step reaction without further purification. ¹H NMR (400 MHz, DMSO-d₆) δ 6.65 (d, J=8.4Hz, 2H), 6.45 (d, J =8.0Hz, 2H), 4.51(br, 2H), 3.17 (m, 2H), 2.74 (m, 1H), 2.57 (m,1H), 2.30-2.00 (m, 5H), 0.98 (d, J=6.0Hz, 3H); ESI-MS: calcd for (C12H19N3) 205, found 206 (MH⁺).

Step 4: A flask was charged with intermediate 2 (50mg, 0.16 mmol), 4-(3,4-dimethylpiperazin-1-yl)aniline (35mg, 0.17 mmol), TFA (50uL), isopropanol (5mL). The reaction was heated to 80°C for overnight. The reaction mixture was basified with a saturated aqueous potassium phospate solution and then was extracted with DCM/ (10mlx3). The combined organic was dried over sodium sulfate and concentrated. The crude product was purified with flash chromatography (0-10% MeOH-in DCM) to afford the desired product as yellow solids (60 mg, 79% yield). ¹H NMR (400 MHz, DMSO-d₆) δ 11.83 (br, 1H), 9.88 (br, 1H), 8.23 (s, 1H), 7.30 (d, J=8.8Hz, 2H), 7.00 (dd, J=5.6Hz, J = 10.4 Hz, 1H), 6.90 (d, J=8.8Hz, 2H), 6.34 (s, 1H), 3.51 (m, 2H), 2.83-2.70 (m, 2H), 2.41 (s, 3H), 2.36 (t, J=10.4Hz, 1H), 2.30-2.05 (m, 5H), 1.03 (d, J=6.4Hz, 3H); ESI-MS: calcd for (C26H25F2N7O) 489, found 490 (MH⁺).

### Reference Compound 47

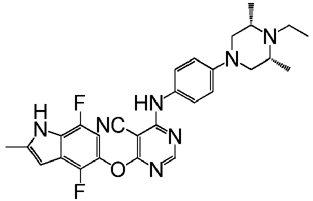

Step 1: To a solution of 4-fluoronitrobenzene (3g, 21.26 mmol) in AcN (60 mL), cis-2,6-dimethylpiperazine (2.55 g, 22.32 mmol) and DIEA (3.90 mL, 22.32 mmol) were added. The mixture was stirred and refluxed for 18 h. The resulting mixture was cooled until room temperature and evaporatedto afford. EtOAc and water was added. The mixture was extracted with EtOAc three times. The organic was washed with brine, dried over sodium sulfate and concentrated. The resisue was crystalized from EtOAc to give the yellow solids of reversed connection of the side product (300mg). The mother liquid was concentrated to minimum amount of solvents and then Hexanes was added to form the yellow precipitate. The solids were collected by filtration and washed with hexanes to afford the desire compound (3R,5S)-3,5-dimethyl-1-(4-nitrophenyl)piperazine as light yellow solids (2.60 g, 52% yield). ¹H NMR (400 MHz, DMSO-d₆) δ 8.02 (d, J=9.6Hz, 2H), 7.02 (d, J =9.2Hz, 2H), 3.90 (m, 2H), 2.78 (m, 2H), 2.40 (m, 2H), 1.03 (d, J=5.6Hz, 6H); ESI-MS: calcd for (C12H17N3O2) 235, found 236 (MH⁺).

Step 2: To a solution of (3R,5S)-3,5-dimethyl-1-(4-nitrophenyl)piperazine (650 mg, 2.76 mmol) and iodoethane (453 mg, 2.90 mmol) in DMF (6 mL) was added potassium carbonate (573 mg g, 4.15 mmol) and the mixture was stiired at room temperature for for overnight. TLC was checked and the starting material was consumed. The mixture was poured to cold water and extracted with DCM (3x15 ml). The combined organic phase was dried over sodium sulfate and concentrated to give the crude product which was purified by column chromatography (0-10% MeOH in DCM) to give the yellow oil. ESI-MS: calcd for (C14H21N3O2) 263, found 264(MH⁺).

Step 3: A solution of above prepared nitro benzine in methanol (∼30 mL) was hydrogenated in the presence of Pd/C (50 mg) using an H₂ balloon. After 16 h, the reaction mixture was filtered through a pad of Celite and rinsed with methanol (3 x 15 mL). The filtrate was concentrated to afford the desired product 4-((3R,5S)-4-ethyl-3,5-dimethylpiperazin-1-yl)aniline (520 mg, 81% yield for 2 steps) as brown solids. The product was used directly for the next step reaction without further purification. ¹H NMR (400 MHz, DMSO-d₆) δ 6.65 (d, J=7.2Hz, 2H), 6.47 (d, J =7.6Hz, 2H), 4.52(br, 2H), 3.20 (d, J=10.8 Hz, 2H), 2.81 (q, J=7.2 Hz, 2H), 2.66 (m, 2H), 2.18(t, J=10.4Hz, 2H), 1.00 (d, J=6.4Hz, 6H), 0.85 (t, J=7.2Hz, 3H); ESI-MS: calcd for (C14H23N3) 233, found 234 (MH⁺).

Step 4: A flask was charged with intermediate 2 (50mg, 0.16 mmol), 4-((3R,5S)-4-ethyl-3,5-dimethylpiperazin-1-yl)aniline (40 mg, 0.17 mmol), TFA (50uL), isopropanol (5mL). The reaction was heated to 80°C for overnight. The reaction mixture was basified with a saturated aqueous potassium phospate solution and then was extracted with DCM/ (10mlx3). The combined organic was dried over sodium sulfate and concentrated. The crude product was purified with flash chromatography (0-10% MeOH-in DCM) to afford the desired product as yellow solids (65 mg, 80% yield). ¹H NMR (400 MHz, DMSO-d₆) δ 11.84 (br, 1H), 9.87 (br, 1H), 8.23 (s, 1H), 7.30 (d, J=8.8Hz, 2H), 7.00 (dd, J=5.6Hz, J = 10.4 Hz, 1H), 6.90 (d, J=8.8Hz, 2H), 6.34 (s, 1H), 3.54 (d, J=11.6Hz, 2H), 2.85 (q, J=7.2Hz, 2H), 2.67 (m, 2H), 2.41 (s, 3H), 2.33 (t, J=10.8Hz, 2H), 1.05 (d, J=6.0Hz, 6H), 0.86 (t, J=7.2Hz, 3H); ESI-MS: calcd for (C28H29F2N7O) 517, found 518 (MH⁺).

### Reference Compound 48

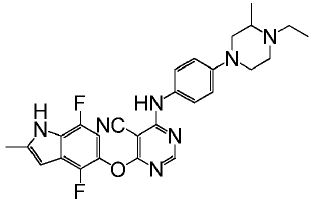

Step 1: To a solution of 4-fluoronitrobenzene (1.80g, 12.76 mmol) in AcN (18 mL), 2-methylpiperazine (3.19 g, 31.89 mmol) and DIEA (3.34 mL, 19.14 mmol) were added. The mixture was stirred at 75 °C for 3 h (in a sealed tube). The resulting mixture was cooled until room temperature and then transferred to water (300 ml). The mixture was stirred at room temperature for 30 min. then cooled with ice. The solids were collected by filtration and washed with water to afford the crude product, which was purified by column chromatography (0-10% MeOH in DCM) to afford the desired compound of 3-methyl-1-(4-nitrophenyl)piperazine as yellow solids (2.03g, 72% yield). ¹H NMR (400 MHz, DMSO-d₆) δ 8.01 (d, J=9.6Hz, 2H), 6.97 (d, J =9.2Hz, 2H), 3.84 (m, 2H), 2.95-2.50 (m, 4H), 2.40 (m, 1H), 2.30 (br, 1H), 1.00 (d, J=6.0Hz, 3H); ESI-MS: calcd for (C11H15N3O2) 221, found 222 (MH⁺).

Step 2: To a solution of 3-methyl-1-(4-nitrophenyl)piperazine (610 mg, 2.76 mmol) and iodoethane (452 mg, 2.89 mmol) in DMF (6 mL) was added potassium carbonate (572 mg g, 4.14 mmol) and the mixture was stiired at room temperature for for overnight. TLC was checked and the starting material was consumed. The mixture was poured to cold water and extracted with DCM (3x15 ml). The combined organic phase was dried over sodium sulfate and concentrated to give the crude product which was purified by column chromatography (0-10% MeOH in DCM) to give 1-ethyl-2-methyl-4-(4-nitrophenyl)piperazine as yellow oil. ESI-MS: calcd for (C13H19N3O2) 249, found 250(MH⁺).

Step 3: A solution of 1-ethyl-2-methyl-4-(4-nitrophenyl)piperazine in methanol (∼30 mL) was hydrogenated in the presence of Pd/C (50 mg) using an H₂ balloon. After 16 h, the reaction mixture was filtered through a pad of Celite and rinsed with methanol (3 x 15 mL). The filtrate was concentrated to afford the the desired product (560 mg, 92% yield for 2 steps) as brown oil. The product was used directly for the next step reaction without further purification. ¹H NMR (400 MHz, DMSO-d₆) δ 6.66 (d, J=8.4Hz, 2H), 6.48 (d, J =8.0Hz, 2H), 4.53(br, 2H), 3.15 (m, 2H), 2.85-2.70 (m, 2H), 2.60 (m,1H), 2.48-2.20 (m, 4H), 1.00 (d, J=6.0Hz, 3H), 0.97 (t, J=7.2Hz, 3H); ESI-MS: calcd for (C13H21N3) 219, found 220(MH⁺).

Step 4: A flask was charged with intermediate 2 (50mg, 0.16 mmol), amine (38 mg, 0.17 mmol), TFA (50uL), isopropanol (5mL). The reaction was heated to 80°C for overnight. The reaction mixture was basified with a saturated aqueous potassium phospate solution and then was extracted with DCM/ (10mlx3). The combined organic was dried over sodium sulfate and concentrated. The crude product was purified with flash chromatography (0-10% MeOH-in DCM) to afford the desired product as yellow solids (63 mg, 80% yield). ¹H NMR (400 MHz, DMSO-d₆) δ 11.83 (br, 1H), 9.88 (br, 1H), 8.23 (s, 1H), 7.30 (d, J=8.8Hz, 2H), 7.00 (dd, J=5.6Hz, J = 10.4 Hz, 1H), 6.91 (d, J=8.8Hz, 2H), 6.34 (s, 1H), 3.44 (m, 2H), 3.00-2.70 (m, 3H), 2.50-2.20 (m, 7H), 1.05 (d, J=5.2Hz, 3H), 0.98 (t, J=7.2Hz, 3H); ESI-MS: calcd for (C27H27F2N7O) 503, found 504 (MH⁺).

### Reference Compound 49

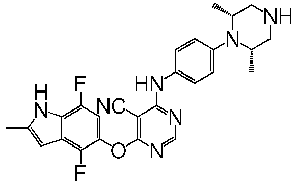

Step 1: A solution of (2S,6R)-2,6-dimethyl-1-(4-nitrophenyl)piperazine (290 mg, 1.23 mmol) in methanol (∼20 mL) was hydrogenated in the presence of Pd/C (28 mg) using an H₂ balloon. After 16 h, the reaction mixture was filtered through a pad of Celite and rinsed with methanol (3 x 15 mL). The filtrate was concentrated to afford the desired product (265 mg, 100% yield) as purple solids. The product was used directly for the next step reaction without further purification. ¹H NMR (400 MHz, DMSO-d₆) δ 6.72 (d, J=7.2Hz, 2H), 6.50 (d, J =7.2Hz, 2H), 4.60(br, 2H), 3.50-3.20 (m, 6H), 2.55 (m,1H), 1.26 (d, J=6.0Hz, 3H); ESI-MS: calcd for (C12H19N3) 205, found 206(MH⁺).

Step 2: A flask was charged with intermediate 2 (50mg, 0.16 mmol), 4-((2S,6R)-2,6-dimethylpiperazin-1-yl)aniline (35 mg, 0.17 mmol), TFA (50uL), isopropanol (5mL). The reaction was heated to 100°C for overnight. The reaction mixture was basified with a saturated aqueous potassium phospate solution and then was extracted with DCM/ (10mlx3). The combined organic was dried over sodium sulfate and concentrated. The crude product was purified with flash chromatography (0-10% MeOH-in DCM) to afford the desired product as yellow solids (50 mg, 65% yield). ¹H NMR (400 MHz, DMSO-d₆) δ 11.83 (br, 1H), 9.87 (br, 1H), 8.23 (s, 1H), 7.30 (d, J=8.8Hz, 2H), 7.00 (dd, J=5.6Hz, J = 10.4 Hz, 1H), 6.91 (d, J=8.8Hz, 2H), 6.34 (s, 1H), 3.51 (d, J=9.6Hz, 2H), 2.84 (m, 2H), 2.41 (s, 3H), 2.11 (t, J=10.8Hz, 2H), 1.02 (d, J=6.0Hz, 6H), (NH, missing); ESI-MS: calcd for (C26H25F2N7O) 489, found 490 (MH⁺).

### Reference Compound 50

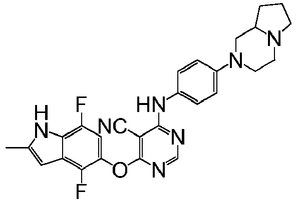

Step 1: To a solution of 4-fluoronitrobenzene (0.61ml, 5.71 mmol) in AcN (6 mL), Octahydropyrrolo[1,2-a]pyrazine (600 mg, 4.75 mmol) and DIEA (1.2 mL, 7.13 mmol) were added. The mixture was stirred at 75 °C for overnight (in a sealed tube). The resulting mixture was cooled to room temperature and then concentrated. The crude product was purified by column chromatography (0-5% MeOH in DCM) to afford the desired compound as yellow oil (1.09g, 92% yield). ESI-MS: calcd for (C13H17N3O2) 247, found 248 (MH⁺).

Step 2: A solution of above prepared nitrobenzine (1.09 g, 4.41 mmol) in methanol (30 mL) was hydrogenated in the presence of 10% Pd/C (0.08 g) using an H₂ balloon. After 16 h, the reaction mixture was filtered through a pad of Celite and rinsed with methanol (3 x 15 mL). The filtrate was concentrated to afford the desired product (0.93 g, 4.28 mmol, 92%) as red solids. The product was used directly for the next step reaction without further purification. ¹H NMR (400 MHz, DMSO-d₆) δ 6.69 (d, J=6.8Hz, 2H), 6.47 (d, J =6.8Hz, 2H), 4.53(br, 2H), 3.41 (d, J=10.8Hz, 1H), 3.27 (d, J=10.8Hz, 1H), 2.98 (m, 2H), 2.58 (t, J=10.8Hz, 1H), 2.30-2.00 (m, 4H), 1.85-1.65 (m, 3H), 1.32 (m, 1H); ESI-MS: calcd for (C13H19N3) 217, found 218 (MH⁺).

Step 3: A flask was charged with Intermedate 2 (50mg, 0.16 mmol), aniline (39 mg, 0.18 mmol), TFA (25uL), isopropanol (3mL). The reaction was heated to 100°C for overnight. The reaction mixture was basified with a saturated aqueous potassium phospate solution and then was extracted with DCM/ (10mlx3). The combined organic was dried over sodium sulfate and concentrated. The crude product was purified with flash chromatography (0-10% MeOH-in DCM) to afford the desired product as yellow solids (56 mg, 72% yield). ¹H NMR (400 MHz, DMSO-d₆) δ 11.83 (br, 1H), 9.88 (br, 1H), 8.23 (s, 1H), 7.30 (d, J=8.4Hz, 2H), 7.00 (dd, J=5.6Hz, J = 10.4 Hz, 1H), 6.93 (d, J=8.8Hz, 2H), 6.34 (s, 1H), 3.76 (d, J=10.8Hz, 1H), 3.62 (d, J=11.6Hz, 1H), 3.03 (m, 2H), 2.73 (t, J=11.6Hz, 1H), 2.41 (s, 3H), 2.40-2.30 (m, 1H), 2.22 (t, J=8.0Hz, 1H), 2.12-2.08 (m, 2H), 1.90-1.60 (m, 3H), 1.42-1.32 (m, 1H); ESI-MS: calcd for (C27H25F2N7O) 501, found 502(MH⁺).

### Reference Compound 51

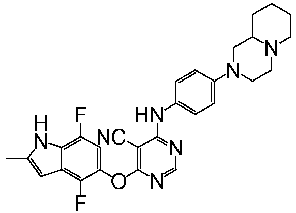

Step 1: To a solution of 4-fluoronitrobenzene (0.55ml, 5.13 mmol) in AcN (6 mL), Octahydro-1H-pyrido[1,2-a]pyrazine (600 mg, 4.28 mmol) and DIEA (1.1 mL, 6.42 mmol) were added. The mixture was stirred at 75 °C for overnight (in a sealed tube). The resulting mixture was cooled to room temperature and then concentrated. The crude product was purified by column chromatography (0-5% MeOH in DCM) to afford the desired product as yellow oil (1.03g, 92% yield). ESI-MS: calcd for (C14H19N3O2) 261, found 262 (MH⁺).

Step 2: A solution of the above nitrobenzine (1.03 g, 3.94 mmol) in methanol (30 mL) was hydrogenated in the presence of 10% Pd/C (0.08 g) using an H₂ balloon. After 16 h, the reaction mixture was filtered through a pad of Celite and rinsed with methanol (3 x 15 mL). The filtrate was concentrated to afford the desired product (0.89 g, 7.85 mmol, 92%) as red solids. The product was used directly for the next step reaction without further purification. ¹H NMR (400 MHz, DMSO-d₆) δ 6.65 (d, J=6.8Hz, 2H), 6.47 (d, J =6.8Hz, 2H), 4.52(br, 2H), 3.23 (d, J=10.8Hz, 1H), 3.16 (d, J=10.8Hz, 1H), 2.72 (m, 2H), 2.56 (t, J=11.2Hz, 1H), 2.19 (m, 2H), 1.93 (t, J=10.8Hz, 2H), 1.75-1.35 (m, 4H), 1.30-1.00 (m, 2H); ESI-MS: calcd for (C14H21N3) 231, found 232 (MH⁺).

Step 3: A flask was charged with intermediate 2 (50mg, 0.16 mmol), the above prepared aniline (42 mg, 0.18 mmol), TFA (25uL), isopropanol (3mL). The reaction was heated to 100°C for overnight. The reaction mixture was basified with a saturated aqueous potassium phospate solution and then was extracted with DCM/ (10mlx3). The combined organic was dried over sodium sulfate and concentrated. The crude product was purified with flash chromatography (0-10% MeOH-in DCM) to afford the desired product as yellow solids (62 mg, 77% yield). ¹H NMR (400 MHz, DMSO-d₆) δ 11.83 (br, 1H), 9.87 (br, 1H), 8.23 (s, 1H), 7.30 (d, J=8.0Hz, 2H), 7.00 (dd, J=5.6Hz, J = 10.4 Hz, 1H), 6.91 (d, J=7.6Hz, 2H), 6.33 (s, 1H), 3.70-3.40 (m, 2H), 2.90-2.60 (m, 3H), 2.41 (s, 3H), 2.40-2.10 (m, 2H), 2.00-1.90 (m, 2H), 1.80-1.10 (m, 5H); ESI-MS: calcd for (C28H27F2N7O) 515, found 516(MH⁺).

### Reference Compound 52

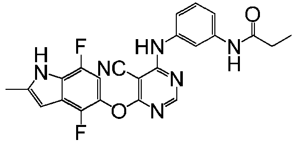

A mixture of QW823 (crude, 70 mg, 0.22 mmol), N-(3-Aminophenyl)propanamide (43mg, 0.26 mmol), and DIPEA (0.08 ml, 0.44 mmol) in DMSO (2 ml) was stiired at room temperature for 2 hours. TLC was checked and the reaction was completed. Ethylacetate (15 ml) was added, followed by NH4Cl (20 mL). After separation, the aquouse was extracted with EtOAc (15 mlx1). The combined organic phase was dried over Na₂SO₄ and concentrated. The crude product was purified by column on slica gel (0-10% MeOH in DCM) to give the desired product as yellow solids (57mg, 58% yield).¹H NMR (400 MHz, DMSO-d₆) δ 11.84 (br, 1H), 10.10 (br, 1H), 9.91 (s, 1H), 8.31 (s, 1H), 7.84 (s, 1H), 7.37 (d, J = 8.4Hz, 1H), 7.27 (t, J=8.0Hz, 1H), 7.18 (d, J=8.0Hz, 1H), 7.00 (dd, J =5.2Hz, J=10.4Hz,1H), 6.34 (s, 1H), 2.42 (s, 3H), 2.32 (q, J=7.6Hz, 2H), 1.08 (t, J=7.6Hz, 3H); ESI-MS: calcd for (C23H18F2N6O2) 448, found 449 (MH⁺).

### Compound 53

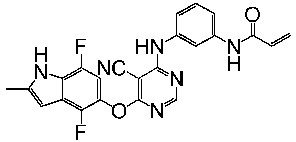

Step 1: To a solution of 3-nitroaniline (5.00 g, 36.2 mmol) in dry THF (50 mL) was added TEA (7.50 mL, 54.3 mmol). The mixture was stirred at r.t. for 10 min followed by dropwise addition of acryloyl chloride (7.38 mL, 90.50 mmol) at 0 °C. The mixture was then stirred at r.t. for 4 hr. The resulting mixture was quenched by sodium bicarbonate and extracted with EtOAc three times. The combined organic was washed with brine, dried over sodium sulfste and concentrated in vacuo. The crude product was crystalized from EtOAc/Hexanes to give the deriseied product as yellow solids (3.37g, 48% yield). ¹H NMR (400 MHz, DMSO-d₆) δ 10.65 (br, 1H), 8.71 (s, 1H), 8.00-7.90 (m, 2H), 7.63 (t, J = 8.0Hz, 1H), 6.50-6.30 (m, 2H), 5.84(d, J=10.0Hz, 1H); ESI-MS: calcd for (C9H8N2O3) 192, found 193(MH⁺).

Step 2: To a solution of N-(3-nitrophenyl)acrylamide (2.06g, 10.72 mmol) in a mixture of MeOH (40 mL) and THF (40 mL) was added Tin(II) chloride dihydrate (12.09g, 53.60 mmol). The mixture was stirred at r.t. overnight and then concentrated. The residue was treated with saturated aq. Na2CO3 to pH=10-11. The mixture was extracted with EtOAc. The combined organic layers were washed with brine, dried over sodium sulfate and concentrated. The residue was used directly used for thennexst step reaction without further purification. (yellow oil, 1.49g, 85% yield). ¹H NMR (400 MHz, DMSO-d₆) δ 9.81 (br, 1H), 6.99 (s, 1H), 6.95 (t, J=9.2Hz, 1H), 6.76 (d, J =7.6Hz, 1H), 6.41 (m, 1H), 6.30-6.10 (m, 2H), 5.6(d, J=10.4Hz, 1H), 5.07 (br, 2H); ESI-MS: calcd for (C9H10N2O) 162, found 163(MH⁺).

Step 3: A mixture of intermediate 2 (crude, 70 mg, 0.22 mmol), aniline (43mg, 0.26 mmol), and DIPEA (0.08 ml, 0.44 mmol) in DMSO (2 ml) was stiired at room temperature for 2 hours. TLC was checked and the reaction was completed. Ethylacetate (15 ml) was added, followed by NH4Cl (20 mL). After separation, the aquouse was extracted with EtOAc (15 mlx1). The combined organic phase was dried over Na₂SO₄ and concentrated. The crude product was purified by column on slica gel (0-10% MeOH in DCM) to give the desired product as yellow solids (50mg, 51% yield).¹H NMR (400 MHz, DMSO-d₆) δ 11.84 (br, 1H), 10.20 (br, 1H), 10.14 (s, 1H), 8.32 (s, 1H), 7.93 (s, 1H), 7.46 (d, J = 8.0Hz, 1H), 7.31 (t, J=8.0Hz, 1H), 7.23 (d, J=8.0Hz, 1H), 7.00 (dd, J =5.2Hz, J=10.4Hz,1H), 6.46 (dd, J=10.0Hz, J=17.2Hz, 1H), 6.34 (s, 1H), 6.26 (d, J=16.8Hz, 1H), 5.76 (d, J=10.0Hz, 1H), 2.42 (s, 3H); ESI-MS: calcd for (C23H16F2N6O2) 446, found 447 (MH⁺).

### Reference Compound 54

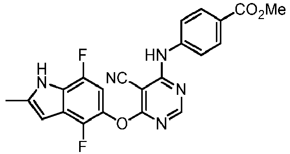

A mixture of 4-chloro-6-((4,7-difluoro-2-methyl-1H-indol-5-yl)oxy)pyrimidine-5-carbonitrile (100 mg, 0.31 mmol), methyl 4-aminobenzoate (47 mg, 0.34 mmol), Pd(OAc)₂ (10 mg, 0.045 mmol), xantphos (45 mg, 0.078 mmol), K₂CO₃ (150 mg, 1.09 mmol) and anhydrous dioxane (12 mL) was sealed in a microwavable tube and degassed with argon for 10 min. The mixture was then heated to 120 °C for 20 min under microwave irradiation. The mixture was cooled to room temperature and partitioned between EtOAc and aq. NaHCO₃ (ca. 100 mL each; 50% NaHCO₃ saturation). The organic layer was separated and dried over anhydrous Na₂SO₄ and concentrated whereupon a precipitate formed. The precipitate was filtered and washed with EtOAc to afford the desired product as a white solid (68 mg, 50% yield). ¹H NMR (DMSO-d6, 400 MHz) δ 11.85 (br s, 1H), 10.39 (br s, 1H), 8.38-8.37 (m, 1H), 7.95-7.93 (m, 2H), 7.75-7.73 (m, 2H), 7.04-7.00 (m, 1H), 6.35 (s, 1H), 3.84 (s, 3H), 2.42 (s, 3H); MS (ESI): calcd for C22H15F2N5O3: 435, found: 436 (MH⁺).

### Reference Compound 55

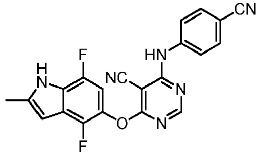

A mixture of 4-chloro-6-((4,7-difluoro-2-methyl-1H-indol-5-yl)oxy)pyrimidine-5-carbonitrile (100 mg, 0.31 mmol), 4-aminobenzonitrile (40 mg, 0.34 mmol), Pd(OAc)₂ (10 mg, 0.045 mmol), xantphos (45 mg, 0.078 mmol), K₂CO₃ (150 mg, 1.09 mmol) and anhydrous dioxane (12 mL) was sealed in a microwavable tube and degassed with argon for 10 min. The mixture was then heated to 120 °C for 20 min under microwave irradiation. The mixture was cooled to room temperature and partitioned between EtOAc and aq. NaHCO₃ (ca. 100 mL each; 50% NaHCO₃ saturation). The organic layer was separated and dried over anhydrous Na₂SO₄ and concentrated whereupon a precipitate formed. The precipitate was filtered and washed with EtOAc to afford the desired product as a white solid (58 mg, 45% yield). ¹H NMR (DMSO-d6, 400 MHz) δ 11.85 (br s, 1H), 10.48 (br s, 1H), 8.38 (s, 1H), 7.81-7.77 (m, 4H), 7.04-7.00 (m, 1H), 6.35 (d, J = 0.8 Hz, 1H), 2.42 (s, 3H); MS (ESI): calcd for C21H12F2N6O: 402, found: 403 (MH⁺).

### Compound 56

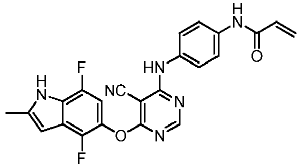

Step 1: To a mixture of 4-nitroaniline (3.0 g, 22 mmol) and TEA (9.2 mL, 66 mmol) in THF (100 mL) was added acryloyl chloride (1.9 mL; 24 mmol), and the mixture was allowed to warm to room temperature and stirred for 17 h. Then additional TEA (9.2 mL, 66 mmol) and acryloyl chloride (1.9 mL; 24 mmol) was added and the mixture was stirred for an additional 2 h. The mixture was partitioned between EtOAc and aq. NaHCO₃ (ca. 100 mL each; 50% NaHCO₃ saturation), and the organic layer was separated and dried over anhydrous Na₂SO₄ and concentrated whereupon a precipitate formed. The precipitate was filtered and washed with EtOAc to afford the desired as a yellow solid (1.9 g, 45% yield). ¹H NMR (DMSO-d6, 400 MHz) δ 10.76 (br s, 1H), 8.25-8.23 (m, 2H), 7.93-7.91 (m, 2H), 6.51-6.44 (m, 1H), 6.37-6.32 (m, 1H), 5.88-5.85 (m, 1H); MS (ESI): calcd for C9H8N2O3: 192, found: 193 (MH⁺).

Step 2: To a suspension of N-(4-nitrophenyl)acrylamide (800 mg, 4.16 mmol) in 5:1 EtOH/water (21 mL) was added iron powder (469 mg, 8.40 mmol) and saturated aq. NH4Cl (2.1 mL), and the mixture was stirred at 80 °C for 3 h. Then additional added iron powder (500 mg, 8.95 mmol) and NH4Cl powder (700 mg, 13.1 mmol) was added and the mixture was stirred for an additional 17 h. The mixture was partitioned between EtOAc and aq. NaHCO3 (100 mL each), and the organic layer was separated and dried over anhydrous Na₂SO₄ and concentrated. The resulting residue was purified by flash column chromatography on silica gel using 0.5-15% MeOH in DCM (v/v) to afford the desired product as a yellow solid (216 mg, 32% yield). ¹H NMR (DMSO-d6, 400 MHz) δ 9.71 (br s, 1H), 7.30 (d, J = 8.4 Hz, 2H), 6.51 (d, J = 8.0 Hz, 2H), 6.40-6.33 (m, 1H), 6.18-6.13 (m, 1H), 5.66-5.63 (m, 1H); MS (ESI): calcd for C9H10N2O: 162, found: 163 (MH⁺).

Step 3: To a mixture of 4-chloro-6-((4,7-difluoro-2-methyl-1H-indol-5-yl)oxy)pyrimidine-5-carbonitrile (230 mg, 0.71 mmol) and N-(4-aminophenyl)acrylamide (120 mg, 1.0 mmol) in anhydrous DMSO (5.0 mL) was added DIPEA (0.43 mL; 2.5 mmol), and the resulting biphasic mixture was efficiently stirred at room temperature for 20 h. The resulting mixture was diluted with 1:9 MeOH/EtOAc (100 mL) and washed with aq. NH₄Cl (ca. 100 mL; 50% NH₄Cl saturation) then brine (100 mL). The organic layer was separated and dried over anhydrous Na₂SO₄ and concentrated whereupon a precipitate formed. The precipitate was filtered and washed with EtOAc to afford the desired product as an off-white solid (219 mg, 69% yield). ¹H NMR (DMSO-d6, 400 MHz) δ 11.84 (br s, 1H), 10.18 (br s, 1H), 10.04 (br s, 1H), 8.29 (d, J = 0.4 Hz, 1H), 7.66 (d, J = 8.8 Hz, 2H), 7.46 (d, J = 8.8 Hz, 2H), 7.03-6.99 (m, 1H), 6.47-6.41 (m, 1H), 6.34-6.24 (m, 2H), 5.77-5.74 (m, 1H), 2.42 (s, 3H); MS (ESI): calcd for C23H16F2N6O2: 446, found: 447 (MH⁺).

### Reference Compound 57

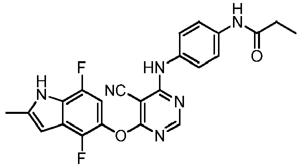

Step 1: To a solution of N-(4-nitrophenyl)propionamide (1.00 g, 5.15 mmol) in MeOH (100 mL) was added 10% Pd/C (100 mg), and the resulting mixture was stirred under H₂ (1 atm) for 21 h. The mixture was then filtered over Celite and concentrated to afford the desired product as an orange oil (858 mg, ca. 100% yield). ¹H NMR (DMSO-d6, 400 MHz) δ 9.39 (br s, 1H), 7.20 (d, J = 8.8 Hz, 2H), 6.48 (d, J = 8.4 Hz, 2H), 4.79 (br s, 2H), 2.22 (q, J = 7.6 Hz, 2H), 1.05 (t, J = 7.6 Hz, 3H).

Step 2: To a mixture of 4-chloro-6-((4,7-difluoro-2-methyl-1H-indol-5-yl)oxy)pyrimidine-5-carbonitrile (80 mg, 0.25 mmol) and N-(4-aminophenyl)propionamide (69 mg, 0.42 mmol) in anhydrous DMSO (1.5 mL) was added TEA (0.10 mL; 0.75 mmol), and the resulting biphasic mixture was efficiently stirred at room temperature for 20 h. The resulting mixture was diluted with 1:9 MeOH/EtOAc (100 mL) and washed with aq. NH₄Cl (ca. 10 mL; 50% NH₄Cl saturation) then brine (10 mL). The organic layer was separated and dried over anhydrous Na₂SO₄ and concentrated whereupon a precipitate formed. The precipitate was filtered and washed with EtOAc to afford the desired product as an off-white solid (69 mg, 62% yield). ¹H NMR (DMSO-d6, 400 MHz) δ 11.83 (br s, 1H), 10.00 (br s, 1H), 9.89 (br s, 1H), 8.27 (d, J = 0.4 Hz, 1H), 7.58 (d, J = 8.8 Hz, 2H), 7.41 (d, J = 8.8 Hz, 2H), 7.03-6.99 (m, 1H), 6.34 (s, 1H), 2.41 (s, 3H), 2.32 (q, J = 7.6 Hz, 2H), 1.09 (t, J = 7.6 Hz, 3H); MS (ESI): calcd for C23H18F2N6O2: 448, found: 449 (MH⁺).

### Reference Compound 58

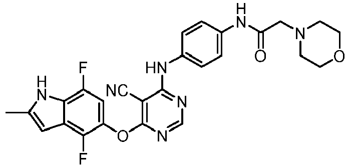

Step 1: To a mixture of 4-nitroaniline (5.0 g, 36 mmol) and TEA (5.0 mL, 36 mmol) in THF (100 mL) stirred at 0 °C was added 2-chloroacetyl chloride (1.9 mL; 24 mmol), and the mixture was allowed to warm to room temperature and stirred for 17 h. The mixture was partitioned between 1:9 MeOH/EtOAc and aq. NaHCO₃ (ca. 100 mL each; 50% NaHCO₃ saturation), and the organic layer was washed with aq. brine (ca. 100 mL; 50% saturation), separated and dried over anhydrous Na₂SO₄ and concentrated whereupon a precipitate formed. The precipitate was filtered and washed with EtOAc to afford the desired product 2-chloro-N-(4-nitrophenyl)acetamide as a yellow solid (6.17 g, 80% yield).¹H NMR (DMSO-d6, 400 MHz) δ 10.90 (br s, 1H), 8.25 (d, J = 8.4 Hz, 2H), 7.84 (d, J = 9.2 Hz, 2H), 4.34 (s, 2H); MS (ESI): calcd for C8H7ClN2O3: 214, found: weak signal.

Step 2: A mixture of 2-chloro-N-(4-nitrophenyl)acetamide (500 mg, 2.33 mmol) and morpholine (2.0 mL, 23 mmol) in isopropanol (ca. 5 mL) was stirred at 80 °C for ca. 17h. The mixture was partitioned between EtOAc and aq. NaHCO₃ (ca. 10 mL each; 50% NaHCO₃ saturation), and the organic layer was separated and dried over anhydrous Na₂SO₄ to afford the desired product as a yellow solid (323 mg, 52% yield). ¹H NMR (DMSO-d6, 400 MHz) δ 10.36 (br s, 1H), 8.24-8.21 (m, 2H), 7.92-7.89 (m, 2H), 3.63 (d, J = 3.2 Hz, 4H), 3.31 (br s, 4H), 3.21 (s, 2H); MS (ESI): calcd for C12H15N3O4: 265, found: 266 (MH⁺).

Step 3: To a solution of 2-morpholino-N-(4-nitrophenyl)acetamide (320 mg, 1.21 mmol) in MeOH (70 mL) was added 10% Pd/C (80 mg), and the resulting mixture was stirred under H₂ (1 atm) for 23 h. The mixture was then filtered over Celite and concentrated to afford the desired product as an orange oil (314 mg, ca. 100% yield). ¹H NMR (DMSO-d6, 400 MHz) δ 9.28 (br s, 1H), 7.22 (d, J = 8.4 Hz, 2H), 6.50 (d, J = 8.8 Hz, 2H), 4.86 (br s, 2H), 3.64-3.62 (m, 4H), 3.04 (s, 2H), 2.51-2.47 (m, 4H); MS (ESI): calcd for C12H17N3O2: 235, found: 236 (MH⁺).

Step 4: To a mixture of 4-chloro-6-((4,7-difluoro-2-methyl-1H-indol-5-yl)oxy)pyrimidine-5-carbonitrile (80 mg, 0.25 mmol) and N-(4-aminophenyl)-2-morpholinoacetamide (68 mg, 0.29 mmol) in anhydrous DMSO (1.5 mL) was added DIPEA (0.15 mL; 0.87 mmol), and the resulting biphasic mixture was efficiently stirred at room temperature for 1.5 days. The resulting mixture was diluted with 1:9 MeOH/EtOAc (10 mL) and washed with aq. NH₄Cl (ca. 10 mL; 50% NH₄Cl saturation) then brine (10 mL). The organic layer was separated and dried over anhydrous Na₂SO₄ and concentrated to afford the desired product as an off-white solid (142 mg, ca. 100% yield). ¹H NMR (DMSO-d6, 400 MHz) δ 11.84 (s, 1H), 10.03 (s, 1H), 9.77 (s, 1H), 8.28 (s, 1H), 7.63-7.61 (m, 2H), 7.44 (d, J = 8.8 Hz, 2H), 7.03-6.99 (m, 1H), 6.34 (br s, 1H), 3.65-3.63 (m, 4H), 2.54-2.52 (m, 4H; obscured by DMSO signal), 2.41 (s, 3H); MS (ESI): calcd for C26H23F2N7O3: 519, found: 520 (MH⁺).

### Reference Compound 59

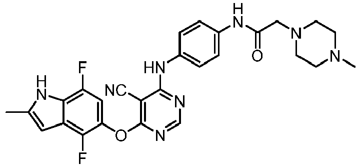

Step 1: A mixture of 2-chloro-N-(4-nitrophenyl)acetamide (500 mg, 2.33 mmol) and N-methylpiperazine (2.6 mL, 23 mmol) in isopropanol (ca. 5 mL) was stirred at 80 °C for ca. 17h. The mixture was partitioned between EtOAc and and aq. NaHCO₃ (ca. 10 mL each; 50% NaHCO₃ saturation), and the organic layer was separated and dried over anhydrous Na₂SO₄ to afford the desired product as a yellow solid (513 mg, 79% yield). ¹H NMR (DMSO-d6, 400 MHz) δ 10.32 (br s, 1H), 8.23-8.21 (m, 2H), 7.91-7.89 (m, 2H), 3.31 (s, 2H), 2.51 (br s, 4H), 2.33 (br s, 4H), 2.17 (s, 3H); MS (ESI): calcd for C13H18N4O3: 278, found: 279 (MH⁺).

Step 2: To a solution of 2-(4-methylpiperazin-1-yl)-N-(4-nitrophenyl)acetamide (500 mg, 1.80 mmol) in MeOH (70 mL) was added 10% Pd/C (75 mg), and the resulting mixture was stirred under H₂ (1 atm) for 19 h. The mixture was then filtered over Celite and concentrated to afford the desired product as an off-white solid (456 mg, ca. 100% yield). ¹H NMR (DMSO-d6, 400 MHz) δ 9.21 (br s, 1H), 7.22 (d, J = 8.8 Hz, 2H), 6.49 (d, J = 8.4 Hz, 2H), 4.86 (br s, 2H), 3.01 (s, 2H), 2.50 (br s, 4H), 2.36 (br s, 4H), 2.16 (s, 3H); MS (ESI): calcd for C13H20N4O: 248, found: 249 (MH⁺).

Step 3: To a mixture of 4-chloro-6-((4,7-difluoro-2-methyl-1H-indol-5-yl)oxy)pyrimidine-5-carbonitrile (80 mg, 0.25 mmol) and N-(4-aminophenyl)-2-(4-methylpiperazin-1-yl)acetamide (71 mg, 0.29 mmol) in anhydrous DMSO (1.5 mL) was added DIPEA (0.15 mL; 0.87 mmol), and the resulting biphasic mixture was efficiently stirred at room temperature for 18 h. The resulting mixture was diluted with 1:9 MeOH/EtOAc (10 mL) and washed with aq. NH₄Cl (ca. 10 mL; 50% NH₄Cl saturation) then brine (10 mL). The organic layer was separated and dried over anhydrous Na₂SO₄ and concentrated, and The resulting residue was purified by flash column chromatography on silica gel using 0-20% MeOH in DCM (v/v) to afford the desired product as an off-white solid (54 mg, 41% yield). ¹H NMR (DMSO-d6, 400 MHz) δ 11.84 (s, 1H), 10.03 (s, 1H), 9.71 (s, 1H), 8.28 (s, 1H), 7.63-7.60 (m, 2H), 7.45-7.42 (m, 2H), 7.03-6.99 (m, 1H), 6.35 (br s, 1H), 2.55-2.52 (m, 4H; obscured by DMSO signal), 2.41 (s, 3H), 2.40-2.37 (m, 4H), 2.17 (s, 3H); MS (ESI): calcd for C27H26F2N8O2: 532, found: 533 (MH⁺).

### Reference Compound 60

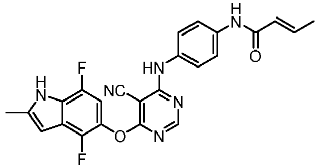

To a mixture of 4-((4-aminophenyl)amino)-6-((4,7-difluoro-2-methyl-1H-indol-5-yl)oxy)pyrimidine-5-carbonitrile (64 mg, 0.16 mmol) and pyridine (39 mg, 0.49 mmol) in DCM (3.0 mL) was added crotonoyl chloride (51 mg, 0.49 mmol). The resulting mixture was efficiently stirred at room temperature for 1 h, whereupon it became homogeneous. The resulting mixture was diluted with EtOAc (10 mL) and washed wit aq. NaHCO₃ (ca. 10 mL; 50% NaHCO₃ saturation). The organic layer was separated and dried over anhydrous Na₂SO₄ and concentrated to afford the desired product as an off-white solid (51 mg, 68% yield). ¹H NMR (DMSO-d6, 400 MHz) δ 11.84 (br s, 1H), 10.03 (s, 1H), 9.82 (s, 1H), 8.29 (s, 1H), 7.68-7.65 (m, 2H), 7.46-7.43 (m, 2H), 7.03-6.99 (m, 1H), 6.34 (d, J = 0.8 Hz, 1H), 5.80 (s, 1H), 5.52 (s, 1H), 2.42 (s, 3H), 1.96 (s, 3H); MS (ESI): calcd for C27H26F2N8O2: 532, found: 533 (MH⁺).

### Compound 61

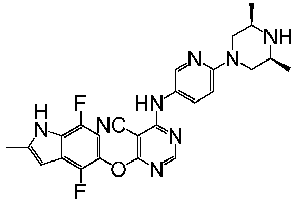

Step 1: To a solution of 2-Chloro-5-nitropyridine (10.00g, 63.07 mmol) and Cis-2,6-dimethylpiperazine (9.00 g, 78.84 mmol) in DMSO (50 ml) was added potassium carbonate (10.90g, 78.84 mmol). The mixture was stirred at 50 °C for 18 h. The resulting mixture was cooled until room temperature and added to a flsk containing water/brine (600 ml). The mixture was stiired at room temperature for 30 min. and then cooled to 0 °C. The solids were collected by filtration, washed with water (100mlx3). The solids were trituated with hexane, collected by filtration and further dried on vac line to give the product as yellow solids (13.82 g, 92% yield). ¹H NMR (400 MHz, DMSO-d₆) δ 8.93 (d, J=2.8Hz, 1H), 8.17 (dd, J =2.8 Hz, J=9.6Hz, 1H), 6.94 (d, J=9.6Hz, 1H), 4.40 (br, 2H), 2.69 (m, 2H), 2.49-2.30 (m, 3H), 1.02 (d, J=6.4Hz, 3H); ESI-MS: calcd for (C11H16N4O2) 236, found 237 (MH⁺).

Step 2: A solution of QW910 (13.72g, 58.07 mmol) in methanol (350 mL) was hydrogenated in the presence of 10% Pd/C (0.60 g) using an H₂ balloon (3x). After 48 h, the reaction mixture was filtered through a pad of celite and rinsed with methanol (250 mL). The filtrate was concentrated to afford the desired product (12.00 g, 100% yield) as purple solids. The product was used directly for the next step reaction without further purification. ¹H NMR (400 MHz, DMSO-d₆) δ 7.58 (d, J=2.8Hz, 1H), 6.88 (dd, J =8.8Hz, J=2.8Hz, 1H), 6.58 (d, J=8.8Hz, 1H), 4.50(br, 2H), 3.80 (m, 2H), 2.75 (m, 2H), 2..02 (m, 3H), 0.98 (d, J=6.0Hz, 3H); ESI-MS: calcd for (C11H18N4) 206, found 207 (MH⁺).

Step 3: To a solution of aniline prepared in step 2 (141 mg, 0.68 mmol) in isopropanol (3mL) was added TFA (50uL) and shaked thoughly. A solution of intermediate 2 (200mg, 0.62 mmolin isopropanol (5ml) was added. The reaction was heated to 85°C for 16h. After cooling to room temperature, hexanes (∼8ml) was added, cooled with ice and filtered to give the purple slods. The solids were supnede in water (50 ml)/MeOH (5ml) and sat. NaHCO₃ (∼20ml) was added (pH >8). The mixture was stiired at room temperature for 45 min., then cooled with ice. The solides were collected by filtration, washed with water (∼5ml) and hexanes (10 ml). The product was obtained as purple solids (263mg, 86%). HPLC was 95% and no further purification was performed. ¹H NMR (400 MHz, DMSO-d₆) δ 11.85 (br, 1H), 9.93 (br, 1H), 8.24 (s, 1H), 8.17 (d, J=2.4Hz, 1H), 7.62 (dd, J=2.4Hz, J =9.2Hz, 1H), 7.00 (dd, J=5.2Hz, J =10.4Hz, 1H), 6.87 (d, J=9.2Hz, 1H), 6.34 (s, 1H), 4.22 (d, J=12Hz, 2H), 2.90 (br, 2H), 2.46-2.00 (m, 6H), 1.11 (d=6.4Hz, 6H); ESI-MS: calcd for (C25H24F2N8O) 490, found 491 (MH⁺).

### EXAMPLES

The following examples are provided to further illustrate the present invention but, of course, should not be construed as in any way limiting its scope.

### Example 1

This example tests exemplary compounds from among Compound 1 to Compound 61 disclosed above for kinase inhibitory activity. A significant number of these compounds have kinase inhibitory activity collectively, over a broad spectrum of kinases.

Kinase assay protocols well known to those of ordinary skill in the art were used. Specifically, the buffer composition was as follows: 20 mM MOPS, 1 mM EDTA, 0.01% Brij-35, 5% Glycerol, 0.1% β-mercaptoethanol, 1 mg/mL BSA. Test compounds were initially dissolved in DMSO at the desired concentration, then serially diluted to the kinase assay buffer. In a final reaction volume of 25 µL FGFR1(h) (5-10 mU) and KDR(h) (5-10 mU) are incubated with 8 mM MOPS pH 7.0, 0.2 mM EDTA, 200 µM LRRASLG (Kemptide), 10 mM magnesium acetate and [γ³³P-ATP]. The reaction was initiated by the addition of the MgATP mix. After incubation for 40 minutes at room temperature, the reaction was stopped by addition of 5 µL of a 3% phosphoric acid solution. Ten µL of the reaction was then spotted onto a P30 filtermat and washed three times for 5 minutes in 50 mM phosphoric acid and once in methanol prior to drying and scintillation counting. Wells containing substrate but no kinase and wells containing a phosphopeptide control were used to set 0% and 100% phosphorylation value, respectively.

Table 1 shows representative data for the inhibition of kinase by compounds of this invention. FGFR1 and KDR kinases would be recognized by those of ordinary skill in the art as associated with cancer.

**Table 1. Inhibition of kinase activity in two kinases associated with cancer.**

| Example No. | % Inhibition at 1 µM | |
|---|---|---|
| | FGFR1(h) | KDR(h) |
| 1 | 99 | 96 |
| 2 | 100 | 96 |
| 3 | 1 | 4 |
| 4 | - | - |
| 5 | 26 | 9 |
| 6 | 85 | 95 |
| 7 | 49 | 94 |
| 8 | 97 | 96 |
| 9 | 100 | 96 |
| 10 | 34 | 23 |
| 11 | 100 | 96 |
| 12 | 56 | 67 |
| 13 | 48 | 85 |
| 14 | 99 | 93 |
| 15 | 94 | 96 |
| 16 | 100 | 96 |
| 17 | 100 | 96 |
| 18 | 76 | 92 |
| 19 | 100 | 86 |
| 20 | 70 | 42 |
| 21 | 100 | 96 |
| 22 | 100 | 96 |
| 23 | 98 | 96 |
| 24 | 86 | 95 |
| 25 | 99 | 96 |
| 26 | 97 | 96 |
| 27 | 99 | 97 |
| 28 | 99 | 96 |
| 29 | 91 | 95 |
| 30 | 91 | 94 |
| 31 | 102 | 95 |
| 32 | 92 | 69 |
| 33 | 101 | 89 |
| 34 | 100 | 89 |
| 35 | 100 | 95 |
| 36 | 99 | 67 |
| 37 | 99 | 70 |
| 38 | 90 | 73 |
| 39 | 91 | 36 |
| 40 | 78 | 20 |
| 41 | 101 | 96 |
| 42 | 101 | 96 |
| 43 | 97 | 76 |
| 44 | 41 | 14 |
| 45 | 101 | 73 |
| 46 | 101 | 90 |
| 47 | 101 | 74 |
| 48 | 100 | 84 |
| 49 | 101 | 82 |
| 50 | 101 | 88 |
| 51 | 101 | 80 |
| 52 | 101 | 91 |
| 53 | 100 | 93 |
| 54 | 89 | 17 |
| 55 | 55 | 6 |
| 56 | 101 | 94 |

### Example 2

A number of studies were performed to analyze the consequences of tyrosine kinase inhibition in cell lines. To do this, 1000 cells are seeded in 27µl/well in 384-well microplates, which are then placed in a humidified CO₂ incubator at 37 C overnight. The next day, 3 µl /well of 10X concentrated drug is added and the plates are returned to the incubator for 72hr. After 72hr incubation, plates are removed and 6 µl /well CellTiterblue (Promega) viability reagent is added. Plates are returned to the incubator for 3 hrs, after which fluorescence measurements are read on the Victor X3 plate reader (Perkin Elmer). Data are analyzed using Excel (Microsoft), and GI₅₀ values are determined using Prism (Graphpad).

For the phospho-FGFR, the following assay Protocol was used. 25,000 cells are seeded in 90**µ**l /well in 96-well microplates, which are then placed in a humidified CO₂ incubator at 37 C overnight. 96-well ELISA plates (Mesoscale Discovery) are coated with capture antibody (R&D Systems Duo-Set) at 4 µg/ml, 30µl/well. The next day, 10µl/well of 10X concentrated drug is added and the plates are returned to the incubator for 20min. ELISA plates are washed using an automated plate washer (BioTek Instruments). After 30 min, cells are inverted and gently tapped to remove excess medium, and immediately placed on ice. 30 1 mPer cell lysis reagent (Thermo Scientific) with protease and phosphatase inhibitors is added/well. After 15min on ice, lysates are mixed and 30µl transferred to the ELISA plate. Plates are incubated for 2hr, washed, and 30µl/well detection antibody is added. After 1 hr, plates are washed and 30µl "SulfoTag" (MesoScale Discovery) detection reagent is added. After 1hr, plates are washed and 150µl/well reading solution is added. Electrochemiluminescence is determined on the Mesoscale Discovery Sector Imager 2000. Data are analyzed using Excel (Microsoft), and EC₅₀ values are determined using Prism (Graphpad).

Table 2 shows representative GI50 data for the inhibition of selected cancer cell lines. Those of ordinary skill in the art appreciate that each cell line is a surrogate for a particular kind of cancer. This example confirms that protein kinase inhibitors can have effects on cell proliferation. Those of ordinary skill would be surprised by the specificity of the kinase inhibition. All of the kinase inhibitors tested herein have been associated with cancer.

**Table 2. Inhibition of Proliferation**

| Compound No. | GI50 (nM) | | | |
|---|---|---|---|---|
| | KG1a | SNU16 | Kato III | RT112 |
| 1 | 49 | - | - | - |
| 2 | 62 | - | - | - |
| 8 | 250 | - | - | - |
| 9 | 314 | - | - | - |
| 11 | 25 | - | | - |
| 14 | 1.6 | 23 | 137 | 76 |
| 16 | 15 | - | - | |
| 17 | 0.74 | 20 | 113 | 83 |
| 19 | 0.2 | 12 | 79 | 66 |
| 22 | 196 | - | - | - |
| 25 | 30 | - | - | - |
| 28 | 226 | - | - | - |
| 31 | 0.35 | 32 | 205 | 171 |
| 33 | 0.28 | 12 | 88 | 126 |
| 34 | 0.28 | 22 | 98 | 52 |
| 35 | 78 | - | - | - |
| 36 | 182 | - | - | - |
| 41 | 180 | - | - | - |
| 42 | 35 | - | - | - |
| 45 | 0.43 | 15 | 79 | 37 |
| 46 | 1.37 | 22 | 111 | 48 |
| 47 | 0.5 | 22 | 123 | 48 |
| 48 | 0.55 | 35 | 152 | 57 |
| 49 | 0.6 | 24 | 86 | 48 |
| 50 | 2.91 | 52 | 174 | 49 |
| 51 | 1.62 | 66 | 208 | 73 |
| 52 | 174 | - | - | |
| 53 | 3.59 | 28 | 152 | 94 |
| 56 | 5.59 | 24 | 106 | 134 |
| 57 | 15 | 6 | - | - |
| 58 | 101 | 122 | - | - |
| 59 | 31 | 79 | - | - |
| 60 | 101 | 92 | - | - |
| 61 | 4 | 75 | - | - |

### Example 3

In this Example the antitumor activity of compounds 14, 17, 19, 45, and 48 of the current invention are tested using an art-recognized xenograft model of AML. ("T/C" refers to the ratio of the tumor's size in treated animals versus the tumor size in control untreated animals. "BWC" refers to "body weight change").

**Table 3. Antitumor Efficacy of Lead Compounds on TG1a Human AML Xenograft**

| **Group** | **No.** | **Compound ID** | **Dose (mg/kg)** | **Dosing Schedule** | **Route** | **T/C (%) (day14)** | **BWC (%)** |
|---|---|---|---|---|---|---|---|
| A | 4 | Vehicle | / | Qdx10 | IP | / | -7.2 |
| B | 4 | Cpd 19 | 5 | Qdx10 | IP | -69.7 | -4.1 |
| C | 4 | Cpd 19 | 10 | Qdx10 | IP | -83.7 | -6.3 |
| D | 4 | Cpd 19 | 20 | Qdx10 | IP | -68.7 (day7) | -26.2(d7) |
| E | 4 | Cpd 14 | 20 | Qdx10 | IP | -60.6 | -4.0 |
| F | 4 | Cpd 17 | 20 | Qdx10 | IP | -79.5 | -5.7 |
| G | 4 | Cpd 45 | 20 | Qdx10 | IP | 4.9 | -11.7 |
| H | 4 | Cpd 48 | 20 | Qdx10 | IP | -72.8 | -3.8 |

| | | | | | | | |
|---|---|---|---|---|---|---|---|
| "Qdx10", once a day for 10 days | | | | | | | |

### Example 4

This Example demonstrates the biologic activity and pharmaceutical suitability of an embodiment of the invention, Compound 19. This Example is offered as exemplary of the characteristics of the inventive compounds disclosed by the current application. However, this Example is in no way meant to be limiting the scope of protection obtained.

Of all of the steps in developing a drug, the hardest is finding promising compounds. (See, e.g., Malo et al., "Statistical practice in high-throughput screening data analysis," Nature Biotechnology, 2006,24, 167-75.) Kinase inhibitors often also have an antiproliferative effect. This characteristic attraction of kinase inhibitors, like the compounds of Example 2, is their ability to inhibit mutant forms of the kinase which have been shown to be important in the transformation of normal cells into cancer cells. The kinase inhibitory activity Compound 19 was tested using the kinase assay described in Example 3. Tables 4 and 5 demonstrate the kinase inhibitory activity of the Compound 19. Unpredictably, Compound 19 is highly active against wild type and mutant forms of fibroblast growth factor receptor kinases. These mutations in the tested kinases are thought to play an important role in transformation of some cancers. In addition, Table 4 also demonstrates that Compound 19 is reasonably selective for fibroblast growth factor receptor kinases.

**Table 4. PHD**

| **Kinase** | **IC50 (nM)** |
|---|---|
| FGFR1 (h) | 0.9 |
| FGFR1(V561M)(h) | 134 |
| FGFR2(h) | 11 |
| FGFR2(N549H)(h) | 7 |
| FGFR3(h) | 12 |
| FGFR4(h) | 12 |
| KDR(h) | 485 |
| Flt4(h) | 116 |
| Lck(h) | >1,000 |
| ABL(h), ALK(h), Aurora-A (h), Axl(h), cSRC(h), Flt3(h), IGF-1R(h), AJK2(h), MEK1(h), Mer(h), Met(h), Ret(h), Tie2(h) | >1,000 |

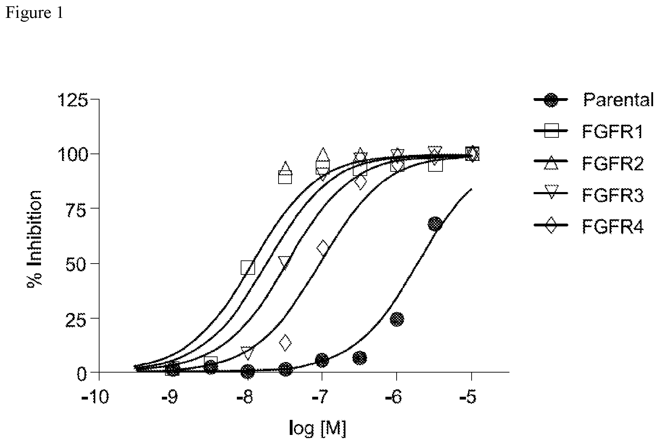

Figure 1 demonstrates the kinase inhibitory dose-response curve for compound 19 against specific kinases in a model system that uses BaF3 cells engineered for the expression of specific kinases. In Figure 1 these kinases are from the fibroblast growth factor receptor class of kinases (FGFR1-FGFR4). Compound 19 kinase inhibitory activity FGFR1-FGFR4 are also depicted in Table 5. Overall, Compound 19 demonstrates significant kinase inhibitory activity.

**Table 5. Compound 19 Activity Against Fibroblast Growth Factor Receptor Kinases**

| Compound | Assay | IC50(nM) |
|---|---|---|
| Cpd 19 | Parental | 1,890 |
| | FGFR1 | 11.4 |
| | FGFR2 | 17.6 |
| | FGFR3 | 32.6 |
| | FGFR4 | 92.1 |

Compound 19 also has significant anti-proliferation properties. Table 6 summarizes results with Compound 19 tested by the proliferation assay described in Example 2.

As shown in Table 6, Compound 19 has significant anti-proliferation activity against some, but not all the cell lines tested. Notably, a common feature of susceptible cells is their expression of one of the fibroblast growth factor receptors. In this assay, Compound 19 is especially effective in KG1a which is a cell line made from a form of acute myeloid leukemia ("AML").

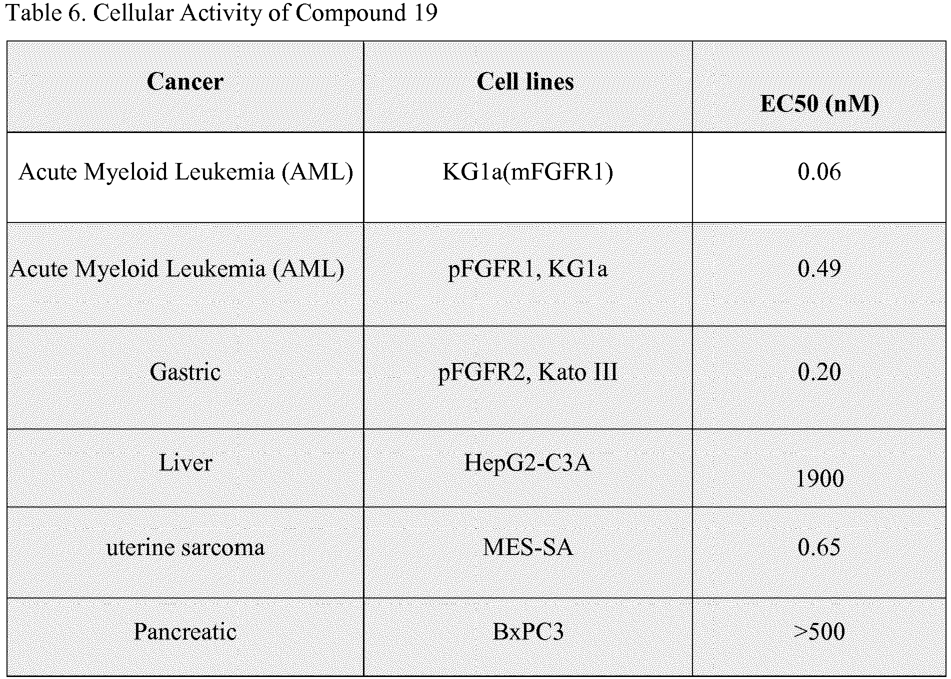

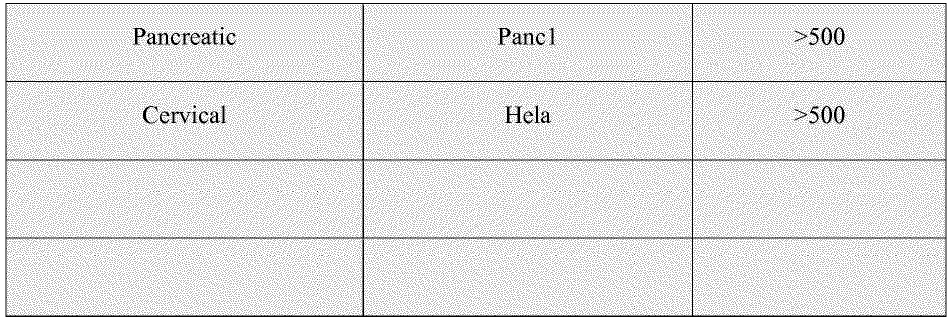

The pharmacokinetic profile of Compound 19 in rats is presented in Table 7. Those of ordinary skill in the art would appreciate that, while not perfectly predictive, these results would be similar in other animals including, e.g., human patients. Compound 19's pharmacokinetic profile is consistent with Compound 19 being used as a therapeutic agent.

**Table 7. Pharmacokinetic Profile of Compound 19 in Rats**

| **Study** | **Results** | | |
|---|---|---|---|
| | | | **Compound 19** |
| PK (Rat) | IV (1 mg/Kg) | Terminal Half Life (hr.) | 6.81 |
| | | Vz(L/kg) | 9.54 |
| | | Cl (mL/min/kg) | 17.57 |
| | PO (5 mg/Kg) | Oral Bioavailability (%F) | 76 |

The metabolic half-life for Compound 19 was determined in human, rat, and mouse model systems his presented in Table 7. Those of ordinary skill in the art will recognize that these results indicate that Compound 19 can be quite stable *in vivo.*

**Table 8. The Metabolic-Stability of Compound 19**

| | **Metabolic Half-Life (min)** | | |
|---|---|---|---|
| **Test Article** | **Human Liver Microsomes** | **Rat Liver Microsomes** | **Mouse Liver Microsomes** |
| DASATINIB | 7.5 | 11.9 | 9.2 |
| COMPOUND 19 | >60 | >60 | >60 |

Table 9 discloses toxicity data for compound 19 from various toxicity tests well known to those of ordinary skill. These results present nothing that would suggest against the institution of the Phase I trial for compound 19.

**Table 9. Compound 19 Toxicity Assessment**

| **Toxicity** | **Test** | **Results** |
|---|---|---|
| Cellular | HepG2 Cell line (CC₅₀) | 1.9 µM |
| | Primary human Hepatocyte (CC₅₀) | 20 µM |
| *In Vivo* Acute | MTD (PO, rat) | 100 mg/kg |
| Cardiovascular | hERG (IC₅₀) | >30 µM |
| CYP450 | 1A2 (IC₅₀) | 72.0 µM |
| | 2C8 (IC₅₀) | 10.6 µM |
| | 2C9 (IC₅₀) | 32.9 µM |
| | 2D6 (IC₅₀) | >100 µM |
| | 3A4 (IC₅₀) | 67.3 µM |

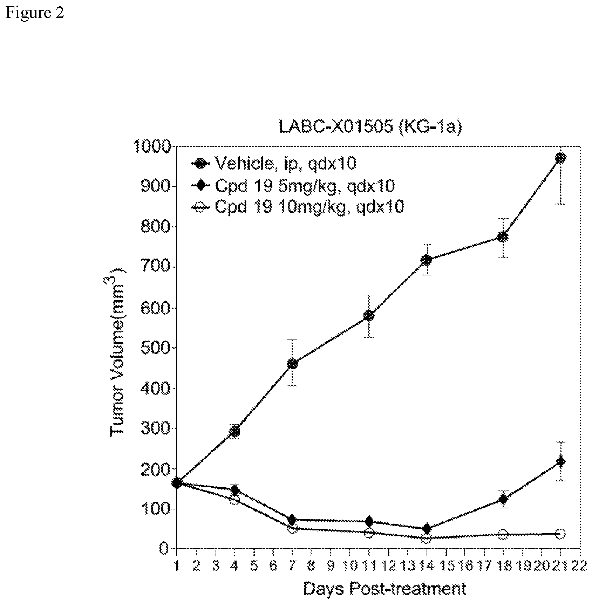

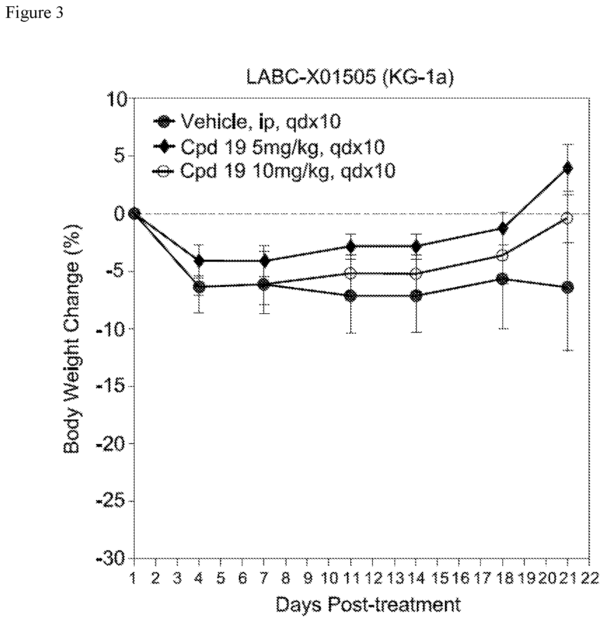

Surprisingly it was found that Compound 19 has significant antitumor activity in a nude mouse model of AML. Figure 2 demonstrates that both of the compound 19 dosages test resulted in near complete suppression of the tumor cells expansion. Figure 3 demonstrates that animal weight, used as a surrogate marker for toxicity, showed no difference between the control and compound 19 treated animals. These results are confirmed in the dose-response study in the same animal model depicted in Figures 4 and 5. (Of note, the highest dose of Compound 19 (20mg/kg) results in significant weight loss suggesting the presence of generalized toxicity, rather than specific effect on cancer cells.)

Overall these data on the behavior of compound 19 are perfectly consistent with this compound being developed into an anti-proliferation/antitumor agent.

## Claims

1. A compound of formula 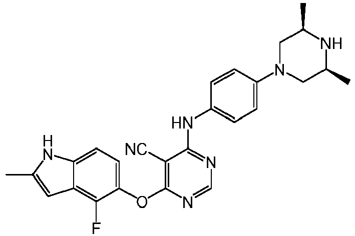 or a pharmaceutically acceptable salt thereof.

2. A compound of formula 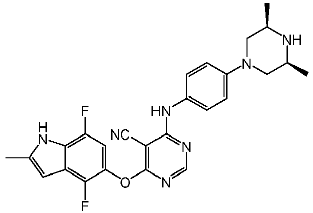 or a pharmaceutically acceptable salt thereof.

3. A compound of formula 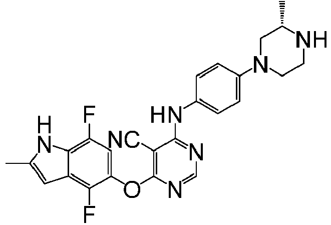 or a pharmaceutically acceptable salt thereof.

4. A compound of formula 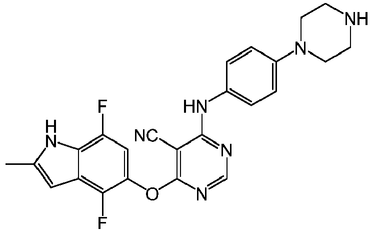 or a pharmaceutically acceptable salt thereof.

5. A compound of formula 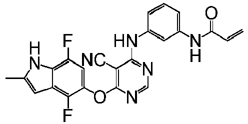 or a pharmaceutically acceptable salt thereof.

6. A compound of formula 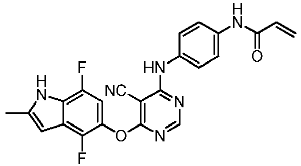 or a pharmaceutically acceptable salt thereof.

7. The compound or pharmaceutically acceptable salt of any one of claims 1-6 for use in treating an animal suffering from a cellular proliferative disorder wherein the use comprises:
a. Preparing, or causing to be prepared, the compound or pharmaceutically acceptable salt of any one of claims 1-6;
b. optionally, formulating, or causing to be formulated, the compound or pharmaceutically acceptable salt of step a with a pharmaceutically acceptable carrier; and
c. that the compound or pharmaceutically acceptable salt of step a or the formulation of step b is to be administered, or caused to be administered to the animal suffering from the proliferative disorder.

8. The compound or pharmaceutically acceptable salt for use according to claim 7, wherein the animal is a mammal.

9. The compound or pharmaceutically acceptable salt for use according to claim 8, wherein the animal is a human.

10. The compound or pharmaceutically acceptable salt for use according to any one claims 7-9, wherein the cellular proliferative disorder is a cancer, a precancerous state, a benign tumor, autoimmune disorder, transplant rejection, graft versus host disease, response to an infection, response to an environmental insult or a genetic disorder.

11. The compound or pharmaceutically acceptable salt for use according to any one of claims 7-9, wherein the cellular proliferative disorder is a cancer.

12. The compound or pharmaceutically acceptable salt for use according to claim 11, wherein the cancer is a skin cancer, breast cancer, uterine cancer, ovarian cancer, testicular cancer, prostate cancer, nasopharyngeal cancer, lung cancer, esophageal cancer, gastric cancer, hepatic cancer, biliary cancer, pancreatic cancer, lymphoma, lung cancer, renal cancer, bladder cancer, esophageal cancers, myeloid leukemia, lymphocytic leukemia, myleoproliferative disorder, myelodysplastic syndrome, lymphoma, neuroendocrine cancer, sarcoma or brain tumor.

## Patentansprüche

1. Eine Verbindung der Formel 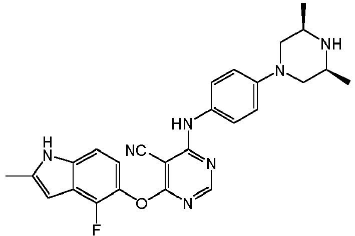 oder ein pharmazeutisch verträgliches Salz davon.

2. Eine Verbindung der Formel 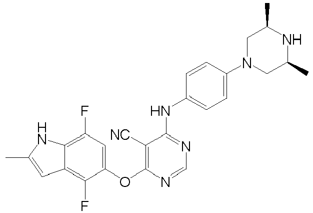 oder ein pharmazeutisch verträgliches Salz davon.

3. Eine Verbindung der Formel 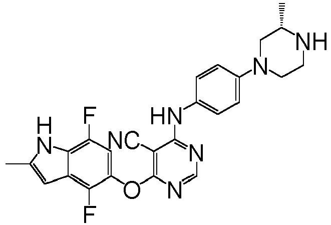 oder ein pharmazeutisch verträgliches Salz davon.

4. Eine Verbindung der Formel 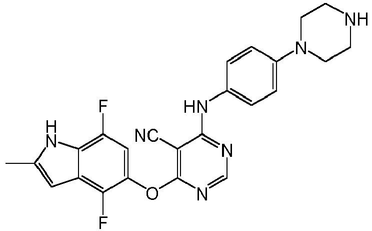 oder ein pharmazeutisch verträgliches Salz davon.

5. Eine Verbindung der Formel 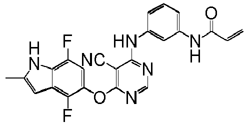 oder ein pharmazeutisch verträgliches Salz davon.

6. Eine Verbindung der Formel 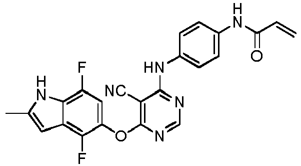 oder ein pharmazeutisch verträgliches Salz davon.

7. Die Verbindung oder das pharmazeutisch verträgliche Salz nach einem der Ansprüche 1-6 zur Verwendung bei der Behandlung eines Individuums, das unter einer Zellproliferationskrankheit leidet, wobei die Verwendung umfasst:
a. die Verbindung oder das pharmazeutisch verträgliche Salz nach einem der Ansprüche 1-6 herzustellen, oder herstellen zu lassen;
b. gegebenenfalls die Verbindung oder das pharmazeutisch verträgliche Salz aus Schritt a. mit einem pharmazeutisch verträglichen Träger zu formulieren, oder formulieren zu lassen; und
c. die Verbindung oder das pharmazeutisch verträgliche Salz aus Schritt a. oder die Formulierung aus Schritt b. dem unter der Zellproliferationskrankheit leidenden Individuum zu verabreichen, oder verabreichen zu lassen.

8. Die Verbindung oder das pharmazeutisch verträgliche Salz zur Verwendung nach Anspruch 7, wobei das Individuum ein Säugetier ist.

9. Die Verbindung oder das pharmazeutisch verträgliche Salz zur Verwendung nach Anspruch 8, wobei das Individuum ein Mensch ist.

10. Die Verbindung oder das pharmazeutisch verträgliche Salz zur Verwendung nach einem der Ansprüche 7-9, wobei die Zellproliferationskrankheit ein Krebs, eine Krebsvorstufe, ein benigner Tumor, eine Autoimmunkrankheit, Transplantatabstoßung, Graft-versus-Host-Krankheit (GvHD), die Reaktion auf eine Infektion, die Reaktion auf einen Umwelt-Insult oder eine genetische Erkrankung ist.

11. Die Verbindung oder das pharmazeutisch verträgliche Salz zur Verwendung nach einem der Ansprüche 7-9, wobei die Zellproliferationskrankheit ein Krebs ist.

12. Die Verbindung oder das pharmazeutisch verträgliche Salz zur Verwendung nach Anspruch 11, wobei der Krebs ein Hautkrebs, Brustkrebs, Gebärmutterkrebs, Ovarialkarzinom, Hodenkrebs, Prostatakrebs, Nasopharynx-Karzinom, Lungenkrebs, Speiseröhrenkrebs, Magenkrebs, Leberkrebs, Gallenblasenkrebs, Pankreaskrebs, Lymphom, Lungenkrebs, Nierenkrebs, Ösophaguskarzinom, myeloische Leukämie, lymphozytische Leukämie, myeloproliferative Erkrankung, myelodysplastisches Syndrom, Lymphom, neuroendokriner Krebs, Sarkom oder Gehirntumor ist.

## Revendications

1. Composé de formule 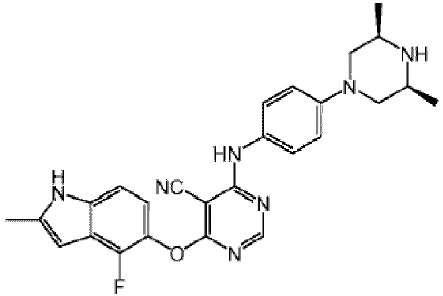 ou son sel pharmaceutiquement acceptable.

2. Composé de formule 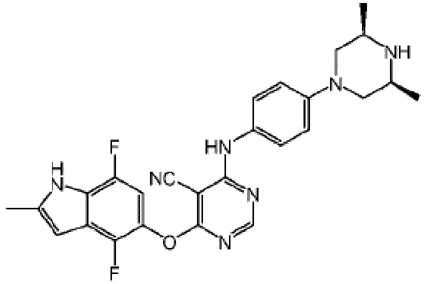 ou son sel pharmaceutiquement acceptable.

3. Composé de formule 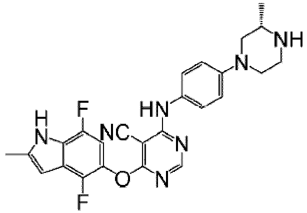 ou son sel pharmaceutiquement acceptable.

4. Composé de formule 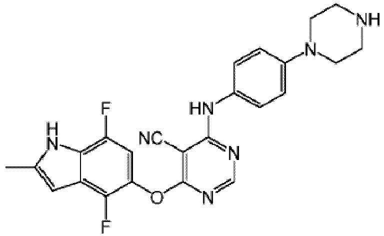 ou son sel pharmaceutiquement acceptable.

5. Composé de formule 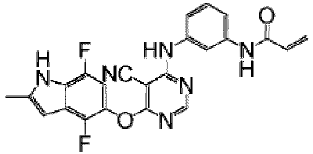 ou son sel pharmaceutiquement acceptable.

6. Composé de formule 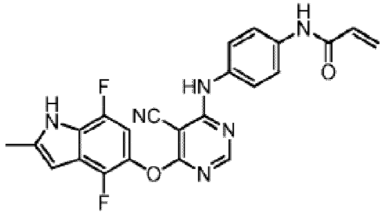 ou son sel pharmaceutiquement acceptable.

7. Composé ou sel pharmaceutiquement acceptable selon l'une quelconque des revendications 1 à 6 pour l'utilisation dans le traitement d'un animal souffrant d'un trouble prolifératif cellulaire où l'utilisation comprend :
a. la préparation, ou le fait d'amener à être préparé, du composé ou son sel pharmaceutiquement acceptable selon l'une quelconque des revendications 1 à 6 ;
b. éventuellement, la formulation, ou le fait d'amener à être formulé, du composé ou du sel pharmaceutiquement acceptable de l'étape a avec un support pharmaceutiquement acceptable ; et
c. le fait que le composé ou le sel pharmaceutiquement acceptable de l'étape a ou la formulation de l'étape b doit être administré-e, ou amenée à être administré-e à l'animal souffrant du trouble prolifératif.

8. Composé ou sel pharmaceutiquement acceptable pour l'utilisation selon la revendication 7, l'animal étant un mammifère.

9. Composé ou sel pharmaceutiquement acceptable pour l'utilisation selon la revendication 8, l'animal étant un être humain.

10. Composé ou sel pharmaceutiquement acceptable pour l'utilisation selon l'une quelconque des revendications 7 à 9, dans lequel le trouble prolifératif cellulaire est un cancer, un état précancéreux, une tumeur bénigne, un trouble auto-immun, un rejet de transplantation, une maladie du greffon *versus* hôte, une réponse à une infection, une réponse à une agression environnementale ou un trouble génétique.

11. Composé ou sel pharmaceutiquement acceptable pour l'utilisation selon l'une quelconque des revendications 7 à 9, dans lequel le trouble prolifératif cellulaire est un cancer.

12. Composé ou sel pharmaceutiquement acceptable pour l'utilisation selon la revendication 11, dans lequel le cancer est un cancer de la peau, un cancer du sein, un cancer de l'utérus, un cancer de l'ovaire, un cancer du testicule, un cancer de la prostate, un cancer nasopharyngé, un cancer du poumon, un cancer de l'oesophage, un cancer de l'estomac, un cancer du foie, un cancer biliaire, un cancer pancréatique, un lymphome, un cancer du poumon, un cancer du rein, un cancer de la vessie, des cancers de l'oesophage, la leucémie myéloïde, la leucémie lymphocytaire, le trouble myéloprolifératif, un syndrome myélodysplasique, un lymphome, un cancer neuroendocrinien, un sarcome ou une tumeur du cerveau.
